# EUROPEAN PATENT APPLICATION

(11) **EP 4 810 544 A1**
(43) Date of publication of application: **23.09.2026**
(21) Application number: 24891434.3
(22) Date of filing: 13.11.2024
(51) Int. Cl.: C08J 3/12, A61F 13/53, B01J 20/26, B01J 20/30

(54) **WATER-ABSORBING AGENT COMPOSITION CONTAINING WATER-ABSORBENT RESIN AS MAIN COMPONENT, AND METHOD FOR PRODUCING SAME**

(30) Priority: 13.11.2023 JP 2023193269
(71) Applicant: Nippon Shokubai Co., Ltd., Osaka-shi, Osaka 541-0043 (JP)
(72) Inventor: TAKEDA, Daisuke, Himeji-shi, Hyogo 671-1282 (JP); OGURA, Tadayuki, Himeji-shi, Hyogo 671-1282 (JP); UEDA, Manabu, Himeji-shi, Hyogo 671-1282 (JP)
(74) Representative: Wallinger Ricker Schlotter PartGmbB
(86) International application number: PCT/JP2024/040354
(87) International publication number: WO 2025/105398

(57) **Abstract**

Provided is a method for producing a water-absorbing agent composition produced using a water-absorbent resin having a large specific surface area, in which particle size segregation due to transportation can be suppressed, the flowability of the particles can be further improved, and the water absorption properties can be satisfactorily maintained. The production method according to the present invention includes a step of mixing a water-soluble flowability improver with a water-absorbent resin under specific conditions in a surface crosslinking step or in a step after the surface crosslinking step,
the method satisfying all of (a) to (d) described below: (a) the water-absorbent resin has a specific surface area of 25 m²/kg or more; (b) when the water-soluble flowability improver is mixed with the water-absorbent resin, the water-soluble flowability improver is in a form of an aqueous solution of 0.01 mass% or more and 20 mass% or less; (c) when the aqueous solution is added to and mixed with the water-absorbent resin, the aqueous solution has an average droplet diameter of 10 µm or more and 1 mm or less; (d) when the aqueous solution is added to and mixed with the water-absorbent resin, a mixing power index is 70000 or more.

## Description

### Technical Field

The present invention relates to a water-absorbing agent composition containing a water-absorbent resin as a main component, and a method for producing the same.

### Background Art

Water-absorbing agent compositions in which water-absorbent resins are used for the purpose of absorbing body fluids are widely used for hygienic materials such as disposable diapers, sanitary napkins, and so-called incontinence pads. As the water-absorbent resin, a crosslinked body of a partially neutralized polyacrylic acid and the like are known. The water-absorbing agent composition is excellent in water absorption characteristics such as the absorption capacity without pressure and the absorption capacity under pressure, and it is also necessary to consider a hygiene problem and a problem of stable operation of the production apparatus in hygienic material applications.

In addition, as the physical properties required for water-absorbent resins, in addition to the absorption capacity without pressure and the absorption capacity under pressure, the water absorption speed is also required to be high. In recent years, a water-absorbent resin having a large specific surface area for increasing the water absorption speed has been disclosed (Patent Documents 1 to 7). A water-absorbent resin having a large specific surface area for blood absorption is also disclosed (Patent Document 8).

### Citation List

### Patent Document

Patent Document 1: WO 97/03114
Patent Document 2: JP 10-057805 A
Patent Document 3: EP 0872491
Patent Document 4: EP 0937739
Patent Document 5: WO 99/03577
Patent Document 6: WO 2013/018571
Patent Document 7: WO 2016/111223
Patent Document 8: WO 02/085959
Patent Document 9: WO 2005/075070
Patent Document 10: WO 2008/120742
Patent Document 11: WO 2019/098244
Patent Document 12: WO 2018/062539

### Summary of Invention

In recent years, a water-absorbent resin having a large specific surface area has been proposed for the purpose of increasing the water absorption speed, and as a method for increasing the specific surface area, a method of increasing the uneven shape of the surface of the water-absorbent resin has exclusively been proposed (Patent Documents 1 to 7). On the other hand, when the uneven shape of the surface is increased, there is a problem that the particles of the water-absorbent resin are caught more at the uneven portion, and as a result, the powder flowability of the water-absorbent resin deteriorates. The inventors of the present invention have examined the use of a surfactant (water-soluble polymer) described in Patent Documents 9 to 12 for a water-absorbent resin having a large specific surface area and an uneven shape increased in size to improve the powder flowability of the resin. However, in the course of the study, a problem has been found that when the water-absorbent resin (water-absorbing agent composition) to which a surfactant (water-soluble flowability improver) has been added is transported with a feeder, particle size segregation occurs in the water-absorbent resin (water-absorbing agent composition) after transportation. In addition, when a surfactant (water-soluble flowability improver) is added, water absorption properties of the water-absorbent resin (water-absorbing agent composition) may deteriorate. Thus, there is a demand for a technique capable of not only solving the problem of flowability during transportation as described above but also satisfactorily maintaining the water absorption properties of the water-absorbent resin (water-absorbing agent composition) while using a surfactant (water-soluble flowability improver).

An object of the present invention is to provide a technique capable of suppressing particle size segregation due to transportation, further improving the flowability of the particles, and satisfactorily maintaining the water absorption properties in a water-absorbing agent composition (water-absorbent resin) produced using a water-absorbent resin having a large specific surface area.

The inventors of the present invention have intensively studied to solve the above-described problems. As a result, the inventors of the present invention have found that the above-described problems can be solved by adding a specific water-soluble flowability improver to a water-absorbent resin under specific conditions in a process for producing a water-absorbing agent composition, specifically, in a surface crosslinking step or a step after the surface crosslinking step, and have completed the present invention.

The present invention that has been able to solve the above-described problems has the following configuration.

That is, one aspect of the present invention is
[1] a method for producing a water-absorbing agent composition including a water-absorbent resin as a main component, the method including a step of preparing an aqueous monomer solution, a polymerization step, a gel-grinding step, a drying step, a pulverizing step, a classification step, and a surface crosslinking step,
   in which during the surface crosslinking step or during a step after the surface crosslinking step, the method includes a step of mixing, with the water-absorbent resin, a water-soluble flowability improver having a mass-average molecular weight of 200 or more and 50000 or less in an amount of more than 0 ppm and less than 200 ppm relative to a mass of the water-absorbent resin,
   the method satisfying all of (a) to (d) described below:
      (a) the water-absorbent resin has a specific surface area of 25 m²/kg or more;
      (b) when the water-soluble flowability improver is mixed with the water-absorbent resin, the water-soluble flowability improver is in a form of an aqueous solution of 0.01 mass% or more and 20 mass% or less;
      (c) when the aqueous solution is added to and mixed with the water-absorbent resin, the aqueous solution has an average droplet diameter of 10 µm or more and 1 mm or less;
      (d) when the aqueous solution is added to and mixed with the water-absorbent resin, a mixing power index defined by (Equation 1) described below is 70000 or more.
         [Math.1] $\begin{matrix}Mixing power index \left(unit: none\right) \\ = peripheral speed \left(m/s\right) \times mixing time \left(s\right) / \left\{average droplet diameter\left(mm\right)/1000\right\}\end{matrix}$
[2] In the production method according to [1], in the water-absorbent resin after addition of the water-soluble flowability improver, particles having a particle diameter of 300 µm or more and less than 600 µm preferably have a kinetic friction coefficient of 0.80 or less;
[3] In the production method according to [1] or [2], the water-soluble flowability improver is preferably one or more selected from a nonionic substance, a
   zwitterionic substance, an anionic substance, and a cationic substance;
[4] In the production method according to [3], the nonionic substance is preferably selected from a polyol, a modified product of a hydroxy group of a polyol, a side-chain and/or terminal polyether-modified polysiloxane, and an alkylene oxide adduct of a higher aliphatic amine; the
   zwitterionic substance is preferably selected from an alkyl betaine and an alkylamine oxide; the anionic substance is preferably selected from an alkyl sulfate ester salt, a sulfate ester salt of a higher alcohol alkylene oxide adduct, a sulfonic acid salt, a dicarboxylic acid salt, an alkylamine diacetic acid salt, a phosphoric acid ester salt of a higher alcohol alkylene oxide adduct, and a carboxylic acid salt of a higher alcohol alkylene oxide adduct; and the cationic substance is preferably selected from an ammonium salt;
[5] In the production method according to any one of [1] to [4], the water-soluble flowability improver preferably includes at least one selected from a nonionic substance;
[6] In the production method according to any one of [1] to [5], the water-soluble flowability improver preferably includes at least one selected from a nonionic substance having a polyalkylene glycol chain in a molecule;
[7] In the production method according to any one of [1] to [6], the water-soluble flowability improver preferably includes at least one selected from a polyol and a modified product of a hydroxy group of a polyol;
[8] In the production method according to any one of [1] to [7], the aqueous solution preferably has a pH of 4.5 or more;
[9] In the production method according to any one of [1] to [8], the water-absorbent resin and the water-absorbing agent composition each preferably include particles having a particle diameter of 300 µm or more and less than 600 µm in an amount of 50 mass% or more, and a kinetic friction coefficient reduction percentage calculated from (Equation 2) described below is preferably 10% or more;
   [Math.2] $Kinetic friction coefficient reduction percentage \left(%\right) = \left(A-B\right) / A \times 100$
   where A is a kinetic friction coefficient of particles of the water-absorbent resin before addition of the water-soluble flowability improver, the particles having a particle diameter of 300 µm or more and less than 600 µm;
   B is a kinetic friction coefficient of particles of the water-absorbing agent composition after addition of the water-soluble flowability improver, the particles having a particle diameter of 300 µm or more and less than 600 µm;
[10] In the production method according to any one of [1] to [9], the method preferably includes further adding a polyalkylene glycol in at least one step selected from the step of preparing an aqueous monomer solution, the polymerization step, and the gel-grinding step, and/or between the steps;
[11] In the production method according to [10], the polyalkylene glycol is preferably a polyethylene glycol having a mass-average molecular weight of 3000 or less;
[12] In the production method according to [10] or [11], an amount of the polyalkylene glycol added is preferably from 0.01 mass% to 0.25 mass% relative to a total mass of monomers included in the aqueous monomer solution.

Another aspect of the present invention is
[13] A water-absorbing agent composition including a water-absorbent resin as a main component, the water-absorbing agent composition including a water-soluble flowability improver and satisfying all of (1) to (5) described below:
   (1) the water-absorbing agent composition has a specific surface area of 25 m²/kg or more;
   (2) the water-absorbing agent composition has a surface tension of 56 mN/m or more;
   (3) the water-absorbing agent composition has a flow rate of 10.0 g/s or more;
   (4) a mass proportion of particles having a particle diameter of 300 µm or more and less than 600 µm in the water-absorbing agent composition is 50 mass% or more;
   (5) the particles having a particle diameter of 300 µm or more and less than 600 µm in the water-absorbing agent composition have a kinetic friction coefficient of 0.80 or less;
[14] In the water-absorbing agent composition according to [13], the water-absorbing agent composition preferably has a Vortex (water absorption speed) of 50 seconds or less;
[15] In the water-absorbing agent composition according to [13] or [14], the water-absorbing agent composition preferably has an SFC (saline flow conductivity) of 1 × 10⁻⁷ cm³·sec/g or more;
[16] In the water-absorbing agent composition according to any one of [13] to [15], the water-absorbing agent composition preferably has a D50 (mass-average particle diameter) of 250 µm or more and less than 550 µm, and a mass proportion of particles preferably has a particle diameter of less than 150 µm is 3 mass% or less;
[17] In the water-absorbing agent composition according to any one of [13] to [16] the water-absorbing agent composition preferably has an AAP (absorption capacity under pressure) of 20 g/g or more.

Another aspect of the present invention is
[18] An absorbent body including the water-absorbing agent composition described in any one of [13] to [17];
[19] In the absorbent body according to [18], a basis weight of pulp is preferably 300 g/m² or less;
[20] In the absorbent body according to [18] or [19], the water-absorbing agent composition preferably has a basis weight of 450 g/m² or less.

Another aspect of the present invention is
[21] An absorbent article including the absorbent body according to any one of [18] to [20];
[22] In the absorbent article according to [21], the absorbent article preferably includes no pulp.

### Brief Description of Drawings

FIG. 1 is a schematic diagram of a measurement portion of a rheometer used for measuring a kinetic friction coefficient. In FIG. 1, reference numeral 1 denotes a dish (container portion), reference numeral 2 denotes a parallel plate, and reference numeral 3 denotes a water-absorbing agent composition.
FIG. 2 is a schematic diagram of an electromagnetic feeder drive unit 4 used in a feeding test. In FIG. 2, reference numeral 4 denotes an electromagnetic feeder drive unit, and reference numeral 5 denotes a trough.

### Description of Embodiments

One aspect of the present invention is a method for producing a water-absorbing agent composition including a water-absorbent resin as a main component, the method including a step of preparing an aqueous monomer solution, a polymerization step, a gel-grinding step, a drying step, a pulverizing step, a classification step, and a surface crosslinking step, in which during the surface crosslinking step or during a step after the surface crosslinking step, the method includes a step of mixing, with the water-absorbent resin, a water-soluble flowability improver having a mass-average molecular weight of 200 or more and 50000 or less in an amount of more than 0 ppm and less than 200 ppm relative to a mass of the water-absorbent resin,
the method satisfying all of (a) to (d) described below:
(a) the water-absorbent resin has a specific surface area of 25 m²/kg or more;
(b) when the water-soluble flowability improver is mixed with the water-absorbent resin, the water-soluble flowability improver is in a form of an aqueous solution of 0.01 mass% or more and 20 mass% or less;
(c) when the aqueous solution is added to and mixed with the water-absorbent resin, the aqueous solution has an average droplet diameter of 10 µm or more and 1 mm or less;
(d) when the aqueous solution is added to and mixed with the water-absorbent resin, a mixing power index defined by (Equation 1) is 70000 or more.
[Math.3]$\begin{matrix}Mixing power index \left(unit: none\right) \\ = peripheral speed \left(m/s\right) \times mixing time \left(s\right) / \left\{average droplet diameter\left(mm\right)/1000\right\}\end{matrix}$

In the present description, the method for producing a water-absorbing agent composition having such a configuration is also simply referred to as "production method according to the present invention" or "method according to the present invention". In the present description, the "water-absorbing agent composition produced by the method according to the present invention" is also simply referred to as "water-absorbing agent composition according to the present invention".

The inventors of the present invention have studied the problem of water-absorbing agent compositions (water-absorbent resins) that occurs when hygienic materials such as disposable diapers are continuously produced, and found that the suppliability (transportability) of the water-absorbing agent compositions tends to deteriorate in comparison to a previous time. In the course of conducting studies to solve this problem, the inventors of the present invention have considered that the decrease in suppliability as described above may be caused by the use of a water-absorbent resin having a large specific surface area.

In recent years, hygienic materials have been reduced in thickness and weight from the viewpoint of sustainability, and water-absorbent resins are required to have a water absorption speed as high as possible. In response to such a demand, a technique has been proposed in which a water-absorbent resin having a high water absorption speed is obtained by increasing the specific surface area. On the other hand, as described above, to improve the suppliability (transportability) of the water-absorbent resin having a large specific surface area, a technique of adding a surfactant (water-soluble polymer) has been proposed (Patent Documents 9 to 12). However, the inventors of the present invention have found a problem that it may be difficult to continuously and stably supply the water-absorbent resin even when a surfactant (water-soluble flowability improver) is added for the purpose of improving the flowability of the powder (water-absorbent resin). More specifically, the inventors of the present invention have found that even when a surfactant (water-soluble flowability improver) is added to a water-absorbent resin having a large specific surface area, particle size segregation occurs in the water-absorbent resin (water-absorbing agent composition) after transportation.

As a result of intensive studies, the inventors of the present invention have found that there is a correlation between continuous and stable supply of a water-absorbent resin having a large specific surface area and the kinetic friction coefficient of the water-absorbing agent composition, and conducted further study on a technique for controlling the kinetic friction coefficient. As a result, the inventors have found that to precisely control the kinetic friction coefficient of a water-absorbing agent composition, it is important to control the type and form of the water-soluble flowability improver to be added and the conditions at the time of addition of the improver, and have reached the present invention.

In the production method according to the present invention, a water-soluble flowability improver (in the present description, it may be simply referred to as "flowability improver") in the form of an aqueous solution is added to a water-absorbent resin. When the aqueous solution of the flowability improver is added, mixing and stirring are performed to obtain the "mixing power index" defined in (Equation 1) having a value equal to or more than a specific value. In the production method according to the present invention, it is possible to uniformly add the flowability improver to the water-absorbent resin having a large specific surface area by comprehensively controlling the mixing power index defined by (Equation 1) and mixing them to obtain the mixing powder index having a value equal to or more than a specific value, rather than individually controlling the stirring speed (peripheral speed), the mixing time, and the average droplet diameter of the aqueous solution of the flowability improver when mixing the aqueous solution of the flowability improver. As a result, the kinetic friction coefficient of the water-absorbing agent composition is uniformly reduced without bias among particle sizes, and thus, the flowability of the water-absorbing agent composition becomes uniform among particle sizes, and the occurrence of the particle size segregation after transportation as described above can be suppressed.

In addition, according to the production method of the present invention, the flowability improver and the water-absorbent resin are sufficiently mixed as described above, and thus, a sufficient flowability improving effect can be obtained even when the amount of the flowability improver added is small. Thus, it is possible to suppress a decrease in water absorption properties due to the addition of the flowability improver and to maintain good water absorption properties.

According to the production method of the present invention, the aqueous solution of the flowability improver can be uniformly added to the water-absorbent resin having a large specific surface area. That is, the flowability improver is not added unevenly to particles having a fine particle diameter and a large specific surface area (particles having a particle diameter of less than 300 µm), and the kinetic friction coefficient of the particles occupying the majority of the water-absorbent resin (particles having a particle diameter of 300 µm or more and less than 600 µm) can be greatly reduced. That is, greatly reducing the kinetic friction coefficient in the particles having a particle diameter of 300 µm or more and less than 600 µm means uniformly improving the flowability of all the particles.

Thus, according to the production method of the present invention, there can be provided a water-absorbing agent composition having a high water absorption speed and a small kinetic friction coefficient in particles having a particle diameter of 300 µm or more and less than 600 µm. That is, there can be provided a water-absorbing agent composition having a high water absorption speed and uniformly improved flowability of all the particles of the water-absorbent resin. By uniformly improving the flowability, the problem that particle size segregation occurs after transportation when the water-absorbing agent composition is transported with a feeder can be solved. It should be noted that the mechanism described above is a surmise and does not limit the technical scope of the present invention.

Hereinafter, the method for producing a water-absorbing agent composition of the present invention will be described in detail, but the scope of the present invention is not limited to these descriptions. Modifications and implementations other than the following examples can be appropriately made without impairing the spirit of the present invention. Specifically, the present invention is not limited to embodiments described below, and may be changed in various ways within the scope of the claims. Thus, an embodiment achieved by appropriately combining technical means disclosed in different embodiments is also included in the technical scope of the present invention.

Throughout the present description, it is to be understood that the expression of a singular form also includes a concept of a plural form thereof, unless otherwise specified. Therefore, it is to be understood that an article for a singular form (for example, "a", "an", and "the" in the case of English) includes a concept of a plural form thereof, unless otherwise stated. Also, it is to be understood that the terms used herein are used in their ordinary meanings in the art, unless otherwise specified. Accordingly, unless defined otherwise, all technical and scientific terms used herein have the same meanings as generally understood by those skilled in the art to which the present invention belongs. In case of conflict, the present description (including definitions) is prior.

### [1] Definition of Terms

### [1-1] Water-Absorbent Resin, Water-Absorbing Agent Composition

The term "water-absorbent resin" in the present description refers to a polymer gelling agent that is water-swellable and water-insoluble, and the water-absorbent resin is generally in a powder form. "Water-swellable" means that CRC (absorption capacity without pressure) defined by NWSP 241.0. R2 (19) is 5 g/g or more, and "water-insoluble" means that Ext (soluble content) defined by NWSP 270.0. R2 (19) is 50 mass% or less.

The "water-absorbent resin" is preferably a hydrophilic crosslinked polymer obtained through crosslinking polymerization of an unsaturated monomer having a carboxyl group, but the whole amount, that is, 100 mass% of the "water-absorbent resin" does not need to be a crosslinked polymer. An additive or the like may be contained within the range that satisfies the performance of CRC, Ext, and the like.

The "water-absorbent resin" may refer to "a polymer in which only the inside is crosslinked, that is, a polymer in which the crosslinking density of the inside and the crosslinking density of the surface are substantially the same" or "a polymer in which the inside and the surface are crosslinked, that is, a polymer in which the crosslinking density of the surface is relatively high relative to the crosslinking density of the inside".

In the present description, the "polymer in which only the inside is crosslinked" and the "polymer in which the inside and the surface are crosslinked" are both described as "water-absorbent resin" without distinction in principle. However, when it is necessary to clearly distinguish the presence or absence of surface crosslinking, the "polymer in which only the inside is crosslinked" is described as "water-absorbent resin before surface crosslinking" because it is a polymer obtained before surface crosslinking is performed, and the "polymer in which the inside and the surface are crosslinked" is described as "water-absorbent resin after surface crosslinking" because it is a polymer obtained after surface crosslinking is performed. The phrase "before surface crosslinking" means "before addition of a surface crosslinking agent" or "before start of a crosslinking reaction through heat treatment even after addition of a surface crosslinking agent".

The "water-absorbent resin" may refer to only a resin component, but it may contain a component other than a resin such as an additive.

The "water-absorbing agent composition" in the present description means a composition containing the "water-absorbent resin" and the "flowability improver". The "water-absorbing agent composition" includes both a case in which a water-absorbent resin composition containing a flowability improver is ready for shipment as a final product as it is and a case in which a water-absorbent resin composition containing a flowability improver is further subjected to any treatment.

The "water-absorbing agent composition" contains a water-absorbent resin as a main component. The "main component" means that the mass proportion of the water-absorbent resin to the whole water-absorbing agent composition is 50 mass% or more where the total mass of the water-absorbing agent composition is 100 mass%. The lower limit value of the mass proportion of the water-absorbent resin to the whole water-absorbing agent composition may be 60 mass% or more, 70 mass% or more, 80 mass% or more, or 90 mass% or more. The upper limit value of the mass proportion is 100 mass% or less, and it may be less than 100 mass% or 99 mass% or less. The "water-absorbing agent composition" may contain water and trace components other than water as components (additional components) other than the water-absorbent resin and the flowability improver. In one embodiment, the mass proportion of the water-absorbent resin contained in the water-absorbing agent composition may be 50 mass% or more and 100 mass% or less, 60 mass% or more and less than 100 mass%, 70 mass% or more and less than 100 mass%, 80 mass% or more and less than 100 mass%, or 90 mass% or more and 99 mass% or less.

### [1-2] Polyacrylic Acid (Salt)-Based Water-Absorbent Resin

The term "polyacrylic acid (salt)-based water-absorbent resin" in the present description means a water-absorbent resin that contains, as a raw material, an acrylic acid and/or a salt thereof (hereinafter, denoted as "acrylic acid (salt)"). That is, the "polyacrylic acid (salt)-based water-absorbent resin" is a polymer having a structural unit derived from an acrylic acid (salt), and is a polymer having a graft component as an optional component. Specifically, the polyacrylic acid (salt)-based water-absorbent resin is a polymer containing an acrylic acid (salt) in an amount of preferably 50 mol% or more, more preferably 70 mol% or more, still more preferably 90 mol% or more, and preferably 100 mol% or less, more preferably substantially 100 mol% relative to the portion excluding an internal crosslinking agent in the monomers involved in the polymerization reaction.

### [1-3] "EDANA" and "NWSP"

"EDANA" is an abbreviation for the European Disposables and Nonwovens Association. "NWSP" is an abbreviation for Non-Woven Standard Procedure, and it indicates an international standard method of measuring a water-absorbing agent composition or a water-absorbent resin provided by EDANA. In the present invention, unless otherwise specified, the physical properties of the water-absorbing agent composition or the water-absorbent resin are measured in accordance with the original NWSP (revised in 2019). In the present invention, the measurements follow the measurement methods in the following Examples unless otherwise stated.

### [1-4] CRC (NWSP 241.0.R2 (19))

"CRC" is an abbreviation for Centrifuge Retention Capacity, and it means an absorption capacity of the water-absorbing agent composition or the water-absorbent resin without pressure. Specific measurement method and measurement conditions are described in Examples.

### [1-5] Ext (NWSP 270.0.R2 (19))

"Ext" is an abbreviation for Extractables, and it means the water-soluble content, that is, the water-soluble component amount of the water-absorbing agent composition or the water-absorbent resin. Specifically, it refers to the amount (unit: mass%) of the dissolved polymer after 1.0 g of the water-absorbing agent composition or the water-absorbent resin is added to 200 ml of a 0.9 mass% aqueous sodium chloride solution and stirred at 250 rpm for 1 hour or 16 hours. The amount of dissolved polymer is determined using pH titration. The stirring time is recorded when the results are reported.

### [1-6] AAP (NWSP 242.0.R2 (19))

"AAP" is an abbreviation for Absorption Against Pressure, and it means an absorption capacity of the water-absorbing agent composition or the water-absorbent resin under pressure. Specific measurement method and measurement conditions are described in Examples.

### [1-7] Specific Surface Area

In the present description, the "specific surface area" means a surface area (unit: m²/kg) per unit mass of the water-absorbing agent composition or the water-absorbent resin, and details of measurement conditions are described in Examples.

### [1-8] Others

In the present description, "A and/or B" and "A and/or B" mean including each of A and B and a combination thereof. "An acid (salt)" means "an acid and/or a salt thereof", and the term "(meth)acryl" means "acryl and/or methacryl". Thus, for example, the term "(meth)acrylic acid" encompasses each of acrylic acid, methacrylic acid, and combinations thereof. The concentration, %, and ppm represent mass concentration, mass%, and mass ppm, respectively, unless otherwise specified, and the ratio is a mass ratio unless otherwise specified. Unless otherwise specified, operations and measurements of physical properties and the like are performed under the conditions of room temperature (23 ± 2°C)/relative humidity 35 ± 5%RH.

### [2] Method for Producing Water-Absorbing Agent Composition

A water-absorbing agent composition obtained by the production method according to the present invention contains a water-absorbent resin as a main component, and further contains a flowability improver. The water-absorbent resin is not particularly limited as long as it has the characteristics described in [1-1], but is preferably a polyacrylic acid (salt)-based water-absorbent resin. That is, one embodiment of the present invention provides a method for producing a water-absorbent resin composition (water-absorbing agent composition) containing a polyacrylic acid (salt)-based water-absorbent resin and a flowability improver. Hereinafter, the method for producing a water-absorbing agent composition will be described in detail.

In one embodiment, the production method according to the present invention includes a step of preparing an aqueous monomer solution, a polymerization step, a gel-grinding step, a drying step, a pulverizing step after drying, a classification step, a surface crosslinking step, and a step of adding an aqueous solution of a flowability improver, in which the step of adding an aqueous solution of a flowability improver is performed in the surface crosslinking step or in a step after the surface crosslinking step. In a preferred embodiment, the production method according to the present invention includes the step of preparing an aqueous monomer solution, the polymerization step, the gel-grinding step, the drying step, the pulverizing step after drying, the classification step, the surface crosslinking step, and the step of adding an aqueous solution of a flowability improver in this order. The production method according to the present invention may further include a step of adding an additive, a cooling step, a rewetting step, a fine powder granulation step, a transportation step, a storage step, a packing step, and a keeping step, as necessary, in addition to each step described above. Hereinafter, each step will be described.

### [2-1] Step of Preparing Aqueous Monomer Solution

This step is a step of preparing an aqueous monomer solution containing a monomer to be a raw material of the water-absorbent resin, preferably an unsaturated monomer, more preferably an unsaturated monomer having a carboxyl group, and still more preferably a monomer containing acrylic acid (salt) as a main component. The aqueous monomer solution preferably contains one or more polymerizable internal crosslinking agents. The "main component" means a component in which the content of acrylic acid (salt) is 50 mol% or more relative to the portion excluding the internal crosslinking agent in the monomer subjected to the polymerization reaction. The lower limit value of the content of acrylic acid (salt) relative to the monomer (excluding the internal crosslinking agent) subjected to the polymerization reaction is preferably 70 mol% or more, more preferably 90 mol% or more. The upper limit value of the content may be 100 mol% or less, 99 mol% or less, or 95 mol% or less. A preferred range of the content of the acrylic acid (salt) can be a range defined by any combinations selected from the upper and lower limit values. A slurry liquid of the monomer may be used within a scope that does not impact the water absorption performance of the water-absorbing agent composition to be obtained as a final product. However, in the present description, an aqueous monomer solution will be described for convenience.

### Acrylic Acid (Salt)

In the present invention, it is preferred to use a known acrylic acid (salt) as the monomer (hereinafter, it may be referred to as "polymerizable monomer") from the viewpoint of the physical properties and productivity of the water-absorbing agent composition or the water-absorbent resin. The known acrylic acid may contain a trace amount of a component such as a polymerization inhibitor or an impurity. As the polymerization inhibitor, methoxyphenols are preferably used, and p-methoxyphenols are more preferably used. The lower limit value of the concentration of the polymerization inhibitor in the acrylic acid is preferably 10 ppm or more, more preferably 20 ppm or more on a mass basis from the viewpoint of the polymerizability of the acrylic acid, the color tone of the water-absorbing agent composition or the water-absorbent resin, and the like. On the other hand, the upper limit value thereof is preferably 200 ppm or less, more preferably 160 ppm or less, still more preferably 100 ppm or less. A preferred range of the concentration of the polymerization inhibitor in the acrylic acid can be a range defined by any combinations selected from the upper and lower limit values.

Examples of the impurity include organic compounds such as acetic acid, propionic acid, and furfural, and the compounds described in US 2008/0161512 A. Examples of the acrylic acid salt include salts obtained by neutralizing the above-described acrylic acids with the following basic compounds. The acrylic acid salt may be a commercially available acrylic acid salt or a salt obtained by neutralizing an acrylic acid.

### Basic Compound

The "basic compound" in the present invention means a compound exhibiting basicity. Specifically, sodium hydroxide or the like corresponds to the above. The commercially available sodium hydroxide contains heavy metals such as zinc, lead, and iron in the order of ppm (on a mass basis), and thus can be strictly expressed as a composition. In the present invention, such a composition is also treated as being included in the category of basic compounds.

The specific examples of the basic compound include a carbonate or a hydrogen carbonate of an alkali metal, a hydroxide of an alkali metal, ammonia, and an organic amine. Among these, a strongly basic compound is selected from the viewpoint of the water absorption performance of the water-absorbing agent composition or the water-absorbent resin. Therefore, hydroxides of alkali metals such as sodium, potassium, and lithium are preferred, and sodium hydroxide is more preferred. Note that the basic compound is preferably an aqueous solution from the viewpoint of ease of handling.

### Neutralization

When a salt obtained by neutralizing an acrylic acid is used as the acrylic acid salt, the neutralization may be performed at any time before polymerization, during polymerization, or after polymerization, or performed at a plurality of times or sites. From the viewpoint of production efficiency of the water-absorbing agent composition or the water-absorbent resin, neutralization is preferred performed continuously.

When an acrylic acid (salt) is used in the present invention, the lower limit value of the neutralization percentage is preferably 10 mol% or more, more preferably 40 mol% or more, still more preferably 50 mol% or more, particularly preferably 60 mol% or more, relative to the acid groups of the monomer. On the other hand, the upper limit value of the neutralization percentage is preferably 90 mol% or less, more preferably 85 mol% or less, still more preferably 80 mol% or less, particularly preferably 75 mol% or less. A preferred range of the neutralization percentage can be a range defined by any combinations selected from the upper and lower limit values. When the neutralization percentage is within the above-described ranges, it is further easy to suppress deterioration in water absorption performance of the water-absorbing agent composition or the water-absorbent resin.

The above-described ranges for the neutralization percentage may be applied to any of the above-described neutralization timings, that is, before polymerization, during polymerization, and after polymerization. The same applies to the acid groups of the water-absorbing agent composition as a final product in addition to the acid groups of the water-absorbent resin.

### Other Monomers

In the present invention, a monomer (hereinafter, referred to as "other monomer") other than the above-described acrylic acid (salt) can be used in combination with the acrylic acid (salt) as necessary. Specific examples of the additional monomer include anionic unsaturated monomers and salts thereof, such as maleic acid, maleic acid anhydride, itaconic acid, cinnamic acid, vinyl sulfonic acid, allyl toluene sulfonic acid, vinyl toluene sulfonic acid, styrene sulfonic acid, 2-(meth)acrylamide-2-methylpropane sulfonic acid, 2-(meth)acryloyl ethane sulfonic acid, 2-(meth)acryloyl propane sulfonic acid, and 2-hydroxyethyl (meth)acryloyl phosphate; mercaptan group-containing unsaturated monomers; phenolic hydroxyl group-containing unsaturated monomers; amide group-containing unsaturated monomers such as (meth)acrylamide, N-ethyl(meth)acrylamide, and N,N-dimethyl(meth)acrylamide; and amino group-containing unsaturated monomers such as N,N-dimethylaminoethyl (meth)acrylate, N,N-dimethylaminopropyl (meth)acrylate, and N,N-dimethylaminopropyl (meth)acrylamide. The additional monomers also include water-soluble or hydrophobic unsaturated monomers. When the additional monomer is used, the amount of the additional monomer used is preferably 30 mol% or less, more preferably 10 mol% or less (lower limit: 0 mol%), still more preferably 5 mol% or less relative to the monomer excluding the internal crosslinking agent.

### Internal Crosslinking Agent

In a preferred production method of the present invention, an internal crosslinking agent is used. Specific examples of the internal crosslinking agent include N,N'-methylenebis(meth)acrylamides, (poly)ethylene glycol di(meth)acrylates, (poly)propylene glycol di(meth)acrylates, trimethylolpropane di(meth)acrylates, trimethylolpropane tri(meth)acrylates, glycerin tri(meth)acrylates, ethylene oxide-modified glycerin tri(meth)acrylates, glycerin acrylate methacrylates, ethylene oxide-modified trimethylolpropane tri(meth)acrylates, pentaerythritol tetra(meth)acrylates, dipentaerythritol hexa(meth)acrylates, triallyl cyanurate, triallyl isocyanurate, triallyl phosphate, triallylamine, poly(meth)allyloxyalkanes, (poly)ethylene glycol diglycidyl ethers, glycerol diglycidyl ether, ethylene glycol, polyethylene glycols, propylene glycols, glycerin, pentaerythritol, ethylenediamine, polyethyleneimines, and glycidyl (meth)acrylates. One of these internal crosslinking agents may be used alone, or two or more thereof may be used in combination. Among these internal crosslinking agents, one or more internal crosslinking agents are selected in consideration of reactivity and the like. From the viewpoint of water absorption performance or the like of the water-absorbing agent composition or the water-absorbent resin, an internal crosslinking agent having two or more polymerizable unsaturated groups is preferably selected, an internal crosslinking agent that is thermally decomposable at the drying temperature described later is more preferably selected, and an internal crosslinking agent having a (poly)alkylene glycol structure and having two or more polymerizable unsaturated groups is still more preferably selected.

Specific examples of the polymerizable unsaturated group include an allyl group and a (meth)acrylate group. Among these, a (meth)acrylate group is preferred. Specific examples of the internal crosslinking agent having a (poly)alkylene glycol structure include polyethylene glycol. The number of alkylene glycol units (hereinafter, it may be denoted to as "n") is preferably 1 or more, more preferably 6 or more, and preferably 100 or less, more preferably 50 or less, still more preferably 20 or less, particularly preferably 10 or less.

Examples of the internal crosslinking agent having a polymerizable unsaturated group and a (poly)alkylene glycol structure include (poly)ethylene glycol di(meth)acrylate and (poly)propylene glycol di(meth)acrylate.

The lower limit value of the amount of the internal crosslinking agent used is preferably 0.0001 mol% or more, more preferably 0.001 mol% or more, still more preferably 0.01 mol% or more, relative to the monomer excluding the internal crosslinking agent. The upper limit value thereof is preferably 10 mol% or less, more preferably 5 mol% or less, still more preferably 1 mol% or less. A preferred range of the amount of the internal crosslinking agent used can be a range defined by any combination selected from the upper and lower limit values described above. When the amount used is within these ranges, the water-absorbing agent composition or the water-absorbent resin having desired water absorption performance (for example, suppression of increase in water-soluble content and decrease in absorption capacity) is further easily obtained.

The internal crosslinking agent is preferably added in advance when preparing the aqueous monomer solution, and in this case, the crosslinking reaction is performed simultaneously with the polymerization reaction. On the other hand, the polymerization reaction may be initiated without adding the internal crosslinking agent, and the internal crosslinking agent may then be added during or after the polymerization reaction to cause a crosslinking reaction. These techniques may also be used in combination. In addition, self-crosslinking can be performed without using an internal crosslinking agent.

### Substances to Be Added to Aqueous Monomer Solution

From the viewpoint of improving the physical properties of the water-absorbing agent composition or the water-absorbent resin, the following substances may be added to the aqueous monomer solution during the polymerization reaction and the crosslinking reaction or at any one or more points after the polymerization reaction and the crosslinking reaction at the time of preparing the aqueous monomer solution. Specific examples of the substances include hydrophilic polymers such as a crosslinked body of starch, starch derivatives, cellulose, cellulose derivatives, polyvinyl alcohol (hereinafter, it may be referred to as "PVA"), polyacrylic acid (salt), and crosslinked products of polyacrylic acid (salt); and compounds such as carbonates, azo compounds, foaming agents that generate various bubbles, surfactants, chelating agents, and chain transfer agents. The substance such as a surfactant to be added to the aqueous monomer solution is not added for the purpose of improving the flowability of the water-absorbing agent composition.

The upper limit value of the amount of the hydrophilic polymer added is preferably 50 mass% or less, more preferably 20 mass% or less, still more preferably 10 mass% or less, particularly preferably 5 mass% or less, relative to the aqueous monomer solution. On the other hand, the lower limit value thereof is preferably 0 mass%, more preferably more than 0 mass%. The upper limit value of the amount of the compound added is preferably 5 mass% or less, more preferably 1 mass% or less, still more preferably 0.5 mass% or less, relative to the aqueous monomer solution. On the other hand, the lower limit value thereof is preferably 0 mass% or more, more preferably more than 0 mass%. Preferred ranges of the amounts of the hydrophilic polymer and the compound added can each be a range defined by any combination selected from the upper and lower limit values described above.

When a water-soluble resin or a water-absorbent resin is used as the hydrophilic polymer, a graft polymer such as a starch-acrylic acid (salt) copolymer or a PVA-acrylic acid (salt) copolymer, or a water-absorbent resin composition is obtained. These graft polymers or water-absorbent resin compositions are also within the scope of the polyacrylic acid (salt)-based water-absorbent resin according to the present invention.

### Concentration of Monomer Component

Various substances and components (hereinafter, referred to as "monomer component") described above are selected according to the purpose, and the amounts thereof are defined so as to satisfy the above-described ranges, and mixed with each other, whereby an aqueous monomer solution is produced. In the present invention, the monomer may be a mixed solution of water and a hydrophilic solvent in addition to an aqueous solution, and such a form is also referred to as an aqueous monomer solution.

The lower limit value of the total concentration of the monomer components is preferably 10 mass% or more, more preferably 20 mass% or more, still more preferably 30 mass% or more, from the viewpoint of the physical properties of the water-absorbing agent composition or the water-absorbent resin. On the other hand, the upper limit value thereof is preferably 80 mass% or less, more preferably 75 mass% or less, still more preferably 70 mass% or less. A preferred range of the total concentration of the monomer components can be a range defined by any combinations selected from the upper and lower limit values. The concentration of the monomer component is calculated from the following (Equation I). 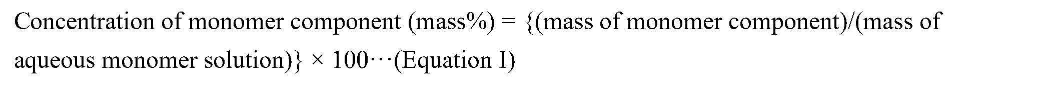 Concentration of monomer component (mass%) = {(mass of monomer component)/(mass of aqueous monomer solution)} × 100···(Equation I)

In (Equation I), the "mass of aqueous monomer solution" does not include the mass of the graft component, the water-absorbent resin, or the hydrophobic organic solvent in the reverse phase suspension polymerization.

### Polyalkylene Glycol

In the step of preparing an aqueous monomer solution, a polyalkylene glycol may be added. In the step of preparing an aqueous monomer solution, the polyalkylene glycol can also be added as an internal crosslinking agent. Further, the polyalkylene glycol may be added in the polymerization step and/or the gel-grinding step described in detail below. That is, in one embodiment, the method for producing a water-absorbing agent composition according to the present invention preferably includes further adding a polyalkylene glycol in at least one step selected from the step of preparing an aqueous monomer solution, the polymerization step, and the gel-grinding step, and/or between the steps (step of preparing an aqueous monomer solution, polymerization step, and gel-grinding step).

The polyalkylene glycol added here is not intended to improve the flowability of the water-absorbent resin. That is, the polyalkylene glycol added in at least one step selected from the step of preparing an aqueous monomer solution, the polymerization step, and the gel-grinding step, and/or each step described above is not included in the flowability improver in the present invention. In general, to improve the flowability of the water-absorbent resin, it is necessary to form a coating film on the particle surface of the water-absorbent resin from the viewpoint of surface modification. However, when the polyalkylene glycol is added in the above-described step, that is, a step in which the polyalkylene glycol can be uniformly distributed inside the water-absorbent resin, the polyalkylene glycol is hardly present on the particle surface of the water-absorbent resin, and a desired flowability improving effect cannot be obtained.

Here, the polyalkylene glycol may be added in any one of the step of preparing an aqueous monomer solution, the polymerization step, and the gel-grinding step, may be added in any two steps, or may be added in all the steps. In addition, the polyalkylene glycol may be added between the step of preparing an aqueous monomer solution and the polymerization step, between the polymerization step and the gel-grinding step, or between all the steps, or may be added not only between these steps but also in each step described above. When the polyalkylene glycol is added in a plurality of steps or between the steps, the polyalkylene glycols to be added in each step or between the steps may be the same or different. In particular, the polyalkylene glycol is preferably added in the step of preparing an aqueous monomer solution. That is, in one embodiment, the method for producing a water-absorbing agent composition according to the present invention preferably further includes adding a polyalkylene glycol in the step of preparing an aqueous monomer solution.

Examples of the polyalkylene glycol include a polyalkylene glycol having a structure represented by the following general formula (1).

H-(OR)ₙ-OH (1)

In the general formula (1), R is an alkylene group having from 2 to 4 carbon atoms, and it may be linear or branched. n is the average addition mole number of the oxyalkylene group (-OR-), and it is preferably from 4 to 70, more preferably from 4 to 50, still more preferably from 6 to 15.

In the general formula (1), oxyalkylene groups (-OR-) in one molecule may be the same or different. More specifically, examples of the polyalkylene glycol include polyethylene glycol, polypropylene glycol, a polyethylene glycol-polypropylene glycol copolymer, and a polyethylene glycol-polypropylene glycol-polybutylene glycol copolymer. One of these polyalkylene glycols may be used alone, or two or more thereof may be used in combination.

The polyalkylene glycol preferably has an upper limit value of its mass-average molecular weight of 3000 or less. The lower limit value of the mass-average molecular weight of the polyalkylene glycol is preferably 200 or more, more preferably 300 or more, still more preferably 400 or more. The mass-average molecular weight is more preferably 2500 or less, still more preferably 2400 or less, particularly preferably 2300 or less. The water absorption performance in the water-absorbent resin after surface crosslinking is improved by setting the mass-average molecular weight of the polyalkylene glycol within the above ranges. Here, the mass-average molecular weight of the polyalkylene glycol is a value measured by gel permeation chromatography.

The polyalkylene glycol having the mass-average molecular weight may be water-soluble. The definition of the term "water-soluble" is as described later. Since the polyalkylene glycol is water-soluble, the polyalkylene glycol can be more uniformly added at the time of addition in at least one step selected from the step of preparing an aqueous monomer solution, the polymerization step, and the gel-grinding step, or between these three steps. As a result, a water-absorbent resin in which the polyalkylene glycol is uniformly present can be obtained. Further, as a result, the water absorption performance of the water-absorbent resin after surface crosslinking improves. The polyalkylene glycol to be added in at least one step selected from the step of preparing an aqueous monomer solution, the polymerization step, and the gel-grinding step, or between these three steps does not correspond to the flowability improver.

In one preferred embodiment, the polyalkylene glycol may be polyethylene glycol having a mass-average molecular weight of 3000 or less. In another embodiment, the polyalkylene glycol may be polyethylene glycol having a mass-average molecular weight of 200 or more and 3000 or less. The mass-average molecular weight of the polyethylene glycol is more preferably 300 or more and 2500 or less, still more preferably 400 or more and 2400 or less, particularly preferably 400 or more and 2300 or less.

The amount of the polyalkylene glycol added is preferably 0.01 mass% or more and 0.25 mass% or less relative to the total mass of the monomers contained in the aqueous monomer solution. Here, the total mass of monomers contained in the aqueous monomer solution means the total mass of monomers excluding the internal crosslinking agent. The lower limit value of the amount of the polyalkylene glycol added is more preferably 0.02 mass% or more, still more preferably 0.03 mass% or more. On the other hand, the upper limit value thereof is more preferably 0.23 mass% or less, still more preferably 0.20 mass% or less, particularly preferably 0.18 mass% or less. When two or more polyalkylene glycols are added, the amount added refers to the total amount thereof. A preferred range of the amount of the polyalkylene glycol added can be a range defined by any combinations selected from the upper and lower limit values. Thus, the amount of the polyalkylene glycol added may be, for example, 0.02 mass% or more and 0.23 mass% or less, 0.02 mass% or more and 0.20 mass% or less, or 0.03 mass% or more and 0.18 mass% or less. When the polyalkylene glycol is added in a plurality of steps, that is, when the polyalkylene glycol is added in at least one step selected from the step of preparing an aqueous monomer solution, the polymerization step, and the gel-grinding step, and/or between the steps (step of preparing an aqueous monomer solution, polymerization step, and gel-grinding step), the polyalkylene glycol is preferably added so that the total amount of the polyalkylene glycol added in each step falls within the above-described ranges. The water absorption performance in the water-absorbent resin after surface crosslinking is improved by setting the amount of the polyalkylene glycol added within the above-described ranges.

As the polyalkylene glycol, two or more polyalkylene glycols may be used. When one or two or more polyalkylene glycols are used, polyalkylene glycols having a plurality of mass-average molecular weights may be used in combination.

### [2-2] Polymerization Step

This step is a step of polymerizing the aqueous monomer solution containing a monomer containing acrylic acid (salt) as a main component and one or more polymerizable internal crosslinking agents obtained in the step of preparing an aqueous monomer solution to obtain a hydrogel.

### Polymerization Initiator

In this step, a polymerization initiator is preferably used. Examples of the polymerization initiator include thermally decomposable polymerization initiators, photo-decomposable polymerization initiators, or redox-based polymerization initiators that use a reducing agent in combination to accelerate the decomposition of these polymerization initiators. Specific examples of the polymerization initiator include radical polymerization initiators such as sodium persulfate, potassium persulfate, ammonium persulfate, t-butyl hydroperoxide, hydrogen peroxide, and 2,2'-azobis(2-amidinopropane)dihydrochloride. Among these polymerization initiators, one or more polymerization initiators are selected in consideration of the type of polymerization and the like. From the viewpoint of the handleability of the polymerization initiator and the physical properties of the water-absorbing agent composition or the water-absorbent resin, a peroxide or an azo compound is preferably selected as the polymerization initiator, a peroxide is more preferably selected as the polymerization initiator, and a persulfate is still more preferably selected as the polymerization initiator. When an oxidative radical polymerization initiator is used, redox polymerization may be performed using a reducing agent such as sodium sulfite, sodium hydrogen sulfite, ferrous sulfate, or L-ascorbic acid in combination.

The lower limit value of the amount of the polymerization initiator used is preferably 0.001 mol% or more, more preferably 0.01 mol% or more, relative to the monomer excluding the internal crosslinking agent. On the other hand, the upper limit value thereof is preferably 1 mol% or less, more preferably 0.5 mol% or less, still more preferably 0.1 mol% or less. The lower limit value of the amount of the reducing agent used is preferably 0.0001 mol% or more, more preferably 0.0005 mol% or more, relative to the monomer excluding the internal crosslinking agent. On the other hand, the upper limit value thereof is preferably 0.02 mol% or less, more preferably 0.015 mol% or less. Preferred ranges of the amounts of the polymerization initiator and the reducing agent used can each be a range defined by any combination selected from the upper and lower limit values described above. By setting the amount used within these ranges, the water-absorbing agent composition or the water-absorbent resin having desired water absorption performance is more easily obtained.

In the present invention, the polymerization reaction may be started through irradiation with an active energy ray such as a radiation, an electron beam, or an ultraviolet ray. Irradiation with an active energy ray and the polymerization initiator may be used in combination.

### Type of Polymerization

Examples of the type of polymerization applicable to the present invention include aqueous solution polymerization, reverse phase suspension polymerization, spray polymerization, droplet polymerization, bulk polymerization, and precipitation polymerization. Among these, from the viewpoint of ease of polymerization control and water absorption performance of the water-absorbing agent composition or the water-absorbent resin, aqueous solution polymerization or reverse phase suspension polymerization is preferably selected, aqueous solution polymerization is more preferably selected, and continuous aqueous solution polymerization is still more preferably selected. The reverse phase suspension polymerization is described in WO 2007/004529, WO 2012/023433, and the like. Examples of the continuous aqueous solution polymerization described above include continuous belt polymerization described in US 4893999, US 6906159, US 7091253, US 7741400, US 8519212, JP 2005-36100 A, and the like, and continuous kneader polymerization described in US 6987151, and the like.

Preferred forms of the continuous aqueous solution polymerization include high-temperature initiation polymerization, high-concentration polymerization, and foaming polymerization. The "high-temperature initiation polymerization" is a type of polymerization in which the temperature of the aqueous monomer solution when polymerization is initiated is preferably set to 30°C or higher, more preferably 35°C or higher, still more preferably 40°C or higher, particularly preferably 50°C or higher, with the upper limit being the boiling point of the aqueous monomer solution. The "high-concentration polymerization" is a type of polymerization in which the monomer concentration when polymerization is initiated is preferably 30 mass% or more, more preferably 35 mass% or more, still more preferably 40 mass% or more, and particularly preferably 42 mass% or more, with the upper limit being the saturation concentration of the aqueous monomer solution. The "foaming polymerization" refers to a type of polymerization in which the aqueous monomer solution containing a foaming agent or bubbles is polymerized. Each of these types of polymerization may be performed alone, or two or more thereof may be used in combination.

The foaming polymerization is one of methods for improving the specific surface area of the water-absorbing agent composition or the water-absorbent resin, and is one of preferred aspects. Examples of the method for dispersing bubbles in the foaming polymerization include:
(I) a method in which a gas dissolved in the aqueous monomer solution is dispersed as bubbles by decreasing the solubility;
(II) a method in which a gas is introduced from the outside and dispersed as bubbles; and
(III) a method in which a foaming agent is added to the aqueous monomer solution and foaming is performed. The dispersion methods may be used in combination depending on the water absorption performance of the water-absorbing agent composition or the water-absorbent resin.

Examples of the gas dissolved in the aqueous monomer solution in (I) include oxygen used for stabilizing the monomer, nitrogen as an inert gas, carbon dioxide, and ozone, and mixed gases thereof.

In the case of (II) a method in which a gas is introduced from the outside and dispersed as bubbles, specific examples of the gas include oxygen, air, nitrogen, carbon dioxide, and ozone, and mixed gases thereof. Among these, from the viewpoint of polymerizability and cost, an inert gas such as nitrogen or carbon dioxide is preferably used, and nitrogen is more preferably used.

In the case of (III) a method in which a foaming agent is added to the aqueous monomer solution and foaming is performed, specific examples of the foaming agent include azo compounds, organic or inorganic carbonate solutions, dispersions, and powders having a particle size of 0.1 µm or more and 1000 µm or less. Carbonates such as sodium carbonate, ammonium carbonate, and magnesium carbonate, and hydrogencarbonate are preferably used. In the aqueous monomer solution containing the foaming agent or bubbles, a surfactant may be used for stably holding the bubbles.

In the methods (I) to (III) listed as the method for dispersing the bubbles in the foaming polymerization, a surfactant may be used in combination. Examples of the surfactant include an anionic surfactant, a nonionic surfactant, a cationic surfactant, an amphoteric surfactant, a fluorine-based surfactant, and an organometallic surfactant. Specific examples thereof include surfactants described in WO 97/017397 and US 6107358.

In the methods (I) to (III), the surfactant that can be used does not provide the effect of improving the flowability of the water-absorbent resin desired in the present description. To improve the flowability of the water-absorbent resin, it is necessary to form a coating film on the particle surface of the water-absorbent resin from the viewpoint of surface modification. However, when a surfactant is added in the polymerization step, that is, a step in which the surfactant can be uniformly distributed inside the water-absorbent resin, the surfactant is hardly present on the particle surface of the water-absorbent resin, and a desired flowability improving effect cannot be obtained.

Each of the types of polymerization described above can be performed in an air atmosphere, but from the viewpoint of the color tone of the water-absorbing agent composition or the water-absorbent resin, the polymerization is preferably performed in an inert gas atmosphere such as nitrogen or argon, and more preferably in an atmosphere having an oxygen concentration of 1 vol% or less. It is also preferred to sufficiently substitute dissolved oxygen in the aqueous monomer solution using an inert gas, and it is more preferred to set the amount of dissolved oxygen to less than 1 mg/L.

By forming a hydrogel, a water-absorbent resin, or a water-absorbing agent composition in a foamed shape through foaming polymerization, the water absorption speed of the water-absorbing agent composition or the water-absorbent resin is increased, and the water-absorbing agent composition is easily immobilized on an absorbent article, which is preferred. The foamed shape can be confirmed by observing pores on the particle surface with an electron microscope. As the size of the hole, a hole having a diameter of 1 µm or more and 100 µm or less is exemplified. The lower limit value of the number of pores is preferably 1 or more, more preferably 10 or more per particle of the water-absorbing agent composition or the water-absorbent resin. On the other hand, the upper limit value thereof is preferably 10000 or less, more preferably 1000 or less. A preferred range of the number of holes can be a range defined by any combinations selected from the upper and lower limit values. The pores can be controlled through the foaming polymerization. The foaming polymerization is a preferred technique for increasing the specific surface area of the water-absorbing agent composition and the water-absorbent resin.

### [2-3] Gel-Grinding Step

The gel-grinding step is a step of performing gel-grinding on the hydrogel obtained in the polymerization step to obtain a hydrogel having a particulate shape (hereinafter, also referred to as "particulate hydrogel"). To distinguish from "pulverizing" in the pulverizing step described later, the grinding in the present step is referred to as "gel-grinding". The "gel-grinding" means that the hydrogel is adjusted to a predetermined size using a gel-grinding apparatus such as a kneader, a meat chopper, or a cutter mill.

As to embodiments, operating conditions and the like of the gel-grinding, the contents described in JP 5989913 B or JP 6067126 B are also preferably applied to the present invention. When the type of polymerization is kneader polymerization, the polymerization step and the gel-grinding step are performed simultaneously. When the particulate hydrogel is obtained in the polymerization step through polymerization such as reverse phase suspension polymerization, spray polymerization, or droplet polymerization, the gel-grinding step is regarded to be performed simultaneously with the polymerization step. By undergoing the gel-grinding step, a water-absorbing agent composition or a water-absorbent resin in an irregularly crushed shape can be obtained.

The particle diameter of the particulate hydrogel that has been finely granulated by the gel-grinding step is preferably 0.05 mm or more and 10 mm or less. When the particle diameter of the particulate hydrogel is 0.05 mm or more, the physical properties of the resulting water-absorbing agent composition or water-absorbent resin improve. When the particle diameter of the particulate hydrogel is 10 mm or less, the particulate hydrogel can be efficiently dried.

The lower limit value of D50 (mass-average particle diameter) of the particulate hydrogel is preferably 50 µm or more, more preferably 100 µm or more, still more preferably 140 µm or more. On the other hand, the upper limit value thereof is preferably 2000 µm or less, more preferably 1500 µm or less, still more preferably 1000 µm or less. A preferred range of D50 of the particulate hydrogel can be a range defined by any combinations selected from the upper and lower limit values. For example, controlling D50 (mass-average particle diameter) of the particulate hydrogel to fall within the above preferred ranges by the following method (B) is one of the methods for improving the specific surface area of the water-absorbing agent composition or the water-absorbent resin, and is one of preferred aspects.

As the PSD (particle size) of the particulate hydrogel, σζ (logarithmic standard deviation) indicating the narrowness of the particle size distribution is preferably 0.2 or more. On the other hand, the upper limit value thereof is preferably 1.5 or less, more preferably 1.3 or less, still more preferably 1.2 or less. The preferred range of σζ (logarithmic standard deviation of particle size distribution) can be a range defined by any combinations selected from the upper and lower limit values. The smaller the value of σζ (logarithmic standard deviation of particle size distribution) is, the more uniform the particle size is, and there is an advantage that more uniform drying can be performed. However, to set σζ (logarithmic standard deviation of particle size distribution) to less than 0.2, it is necessary to perform special operations such as particle size control at the time of polymerization before gel-grinding and classification of a particulate hydrogel after gel-grinding, and thus, it is substantially difficult to perform it from the viewpoint of productivity and cost.

In the present invention, it is desirable to control any one or more methods of (A) foaming polymerization of the aqueous monomer solution, (B) pulverization and granulation of a particulate hydrogel or a dried polymer thereof, and (C) fine powder recycling so that the specific surface area of the water-absorbent resin is 25 m²/kg or more.

The specific surface area of the water-absorbent resin can be increased to 25 m²/kg or more by adopting, as (A) foaming polymerization of the aqueous monomer solution, for example, a foaming polymerization method in which a dissolved gas dissolved in the aqueous monomer solution is encapsulated and foamed in a system when being gelled by high-temperature and short-time polymerization, or a surfactant is made to coexist in the aqueous monomer solution, that is, a foaming polymerization method described in JP 5647625 B (specifically, for example, a method of generating bubbles in the aqueous monomer solution by reducing the solubility of a dissolved gas in the aqueous monomer solution in the presence of a surfactant), a foaming polymerization method in which a gas is externally introduced into the aqueous monomer solution and dispersed as bubbles to perform polymerization, a foaming polymerization method in which a foaming agent is added to the aqueous monomer solution and foaming is performed, or the like. Thus, it is also preferred that the water-absorbent resin is obtained by foaming polymerization of an aqueous unsaturated monomer solution.

The specific surface area of the water-absorbent resin can be increased to 25 m²/kg or more by adopting, as (B) pulverization and granulation of the particulate hydrogel or the dried polymer thereof, for example, a gel-grinding method described in JP 5989913 B, JP 6067126 B, or WO 2016/204302 in the gel-grinding step, and by further performing drying. A water-absorbent resin having a desired specific surface area can also be obtained by appropriately controlling the die hole diameter, the number of holes, the die thickness, the amount of hot water added, the rotational speed of the screw shaft, and the like of a gel-grinding apparatus such as a meat chopper. The granulation may be performed on the hydrogel at the time of polymerization, may be performed simultaneously with drying of the finely pulverized product of the hydrogel after polymerization, or may be performed using water and/or an organic or inorganic binder for the finely pulverized product after drying. Thus, it is also preferred to contain a granulated product of a hydrogel of the water-absorbent resin or a dried product thereof.

The specific surface area of the water-absorbent resin can be increased to 25 m²/kg or more by adopting, as (C) fine powder recycling, for example, recovering fine powder of the water-absorbent resin that has passed through a sieve with a mesh size of 150 µm in the polymerization step, the gel-grinding step, or the drying step, or granulating and recovering the fine powder. Thus, it is also preferred that the water-absorbent resin contains a fine recycled product of the water-absorbent resin. The methods (A) to (C) may be performed alone or in combination.

As the method for increasing the specific surface area of the water-absorbent resin to 25 m²/kg or more, there is also a method of containing a large amount of particles having a small particle diameter. However, in this method, a large amount of particles having a small particle diameter, in particular, fine powder passing through a sieve with a mesh diameter of 150 µm is contained. As a result, gel blocking of the resulting water-absorbing agent composition is likely to occur, and liquid absorbing performance and liquid permeability under pressure deteriorate, which is not preferred. Thus, when the specific surface area is adjusted using the fine powder, it is preferred to adopt the method of (B) and/or (C) described above. In the present invention, it is preferred to perform the adjustment method described later with sufficient attention to the adjustment of the particle size distribution.

D50 (mass-average particle diameter) and σζ (logarithmic standard deviation of particle size distribution) of the particulate hydrogel are measured by the methods described in paragraphs [0257] to [0270] of WO 2016/111223, which is incorporated herein by reference.

### [2-4] Drying Step

The drying step is a step of producing a dried polymer by drying the hydrogel and/or the particulate hydrogel obtained through the polymerization step and/or the gel-grinding step to a desired solid content. The solid content of the dried polymer is determined from a mass change when 1 g of the water-absorbent resin is heated at 180°C for 3 hours. The lower limit value of the solid content of the dried polymer is preferably 80 mass% or more, more preferably 85 mass% or more, still more preferably 90 mass% or more, particularly preferably 92 mass% or more. On the other hand, the upper limit value thereof is preferably 99 mass% or less, more preferably 98 mass% or less, still more preferably 97 mass% or less. A preferred range of the solid content of the dried polymer can be a range defined by any combinations selected from the upper and lower limit values.

Specific examples of the method for drying the hydrogel and/or the particulate hydrogel include thermal drying, hot air drying, drying under reduced pressure, fluidized bed drying, infrared drying, microwave drying, drum dryer drying, drying through azeotropic dehydration with a hydrophobic organic solvent, and high humidity drying with the use of high-temperature water vapor. Among these, from the viewpoint of drying efficiency, hot air drying is preferred, and band drying in which hot air drying is performed on a ventilation belt is more preferred.

The lower limit value of the drying temperature in the hot air drying is preferably 100°C or higher, more preferably 150°C or higher, from the viewpoint of the color tone and drying efficiency of the water-absorbing agent composition or the water-absorbent resin. On the other hand, the upper limit value thereof is preferably 300°C or lower, more preferably 200°C or lower. A preferred range of the drying temperature can be a range defined by any combinations selected from the upper and lower limit values. The drying temperature in hot air drying is defined by the temperature of hot air. Drying conditions other than the drying temperature, such as hot air speed and drying time, may be appropriately set according to the water content and total mass of the particulate hydrogel to be dried and the target solid content. When band drying is performed, various conditions described in WO 2006/100300, WO 2011/025012, WO 2011/025013, WO 2011/111657, and the like, are appropriately applied.

The lower limit value of the drying time in the present invention is preferably 1 minute or more, more preferably 5 minutes or more, still more preferably 10 minutes or more. On the other hand, the upper limit value thereof is preferably 10 hours or less, more preferably 3 hours or less, still more preferably 1 hour or less. A preferred range of the drying time can be a range defined by any combinations selected from the upper and lower limit values. By setting the drying temperature and the drying time within these ranges, the physical properties of the resulting water-absorbing agent composition can be set within desired ranges. In addition, the physical properties of the water-absorbent resin as an intermediate product can be set within a desired range. When drying is performed by hot air drying, the lower limit value of the wind speed of hot air is preferably 0.5 m/s or more. On the other hand, the upper limit value thereof is preferably 3.0 m/s or less, more preferably 2.0 m/s or less. A preferred range of the wind speed of the hot air can be a range defined by any combinations selected from the upper and lower limit values. Other drying conditions may be appropriately set according to the water content and the total mass of the particulate hydrogel to be dried, the intended solid content, and the like.

### [2-5] Pulverizing Step and Classification Step

The pulverizing step is a step of pulverizing the dried polymer obtained through the drying step. Through the pulverizing step after drying, a water-absorbent resin in an irregularly crushed shape is obtained. The classification step is a step of classifying the dried polymer pulverized in the pulverizing step to obtain a particle size within a desired range. Through such steps, a water-absorbent resin before surface crosslinking is obtained.

Specific examples of the pulverizing apparatus used in the pulverizing step include high-speed rotary pulverizing apparatuses such as a roll mill, a hammer mill, a screw mill, and a pin mill; vibration mills, knuckle type pulverizing apparatuses, and cylindrical mixers. Among these, a roll mill is preferably selected from the viewpoint of pulverizing efficiency. A plurality of these pulverizing apparatuses can be used in combination.

Examples of the method for adjusting the particle size in the classification step include air flow classification and sieve classification using JIS standard sieves (JIS Z 8801-1 (2000)). Among these, the sieve classification is preferably selected from the viewpoint of classification efficiency. The particle size adjustment of the water-absorbing agent composition or the water-absorbent resin is not limited to in the pulverizing step and/or the classification step, and it may be performed in the polymerization step, particularly, reverse phase suspension polymerization, droplet polymerization, or the like, or in other steps, for example, a granulation step or a fine powder recovery step.

The proportion of (i) particles having a particle diameter of less than 150 µm (hereinafter, referred to as "particles of less than 150 µm") contained in the water-absorbent resin before surface crosslinking after classification is preferably 3 mass% or less, more preferably 2.5 mass% or less, still more preferably 2 mass% or less. In continuous commercial production, it may be very difficult to set the proportion of particles of less than 150 µm to 0 mass% from the viewpoint of production efficiency. Thus, the proportion of particles of less than 150 µm is preferably 0.1 mass% or more, more preferably 0.2 mass% or more, still more preferably 0.3 mass% or more. In the present description, the term "particles having a particle diameter of less than 150 µm" refers to particles that pass through a sieve with a mesh size of 150 µm after being classified by the same method as the classification method in the method for evaluating a kinetic friction coefficient of Examples.

The lower limit value of (ii) D50 (mass-average particle diameter) is preferably 250 µm or more, more preferably 300 µm or more, still more preferably 330 µm or more. On the other hand, the upper limit value thereof is preferably less than 550 µm, more preferably less than 500 µm, still more preferably less than 450 µm. A preferred range of the D50 (mass-average particle diameter) can be a range defined by any combinations selected from the upper and lower limit values.

Further, in (iii) the particle size distribution of the water-absorbent resin before surface crosslinking, it is more preferred that D50 (mass-average particle diameter) falls within the range of (ii), and the proportion of particles having a particle diameter of less than 150 µm falls within the range of (i).

Further, the lower limit value of (iv) σζ (logarithmic standard deviation of particle size distribution) is preferably 0.20 or more, more preferably 0.25 or more, still more preferably 0.27 or more. On the other hand, the upper limit value thereof is preferably 0.50 or less, more preferably 0.40 or less, still more preferably 0.35 or less. The preferred range of σζ (logarithmic standard deviation of particle size distribution) can be a range defined by any combinations selected from the upper and lower limit values. σζ (Logarithmic standard deviation of particle size distribution) has an advantage that the smaller the value is, the more uniform the particle size is and the less segregation of particles is. However, to make the σζ (logarithmic standard deviation of particle size distribution) excessively small, it is necessary to remove coarse particles and fine particles by repeating pulverization and classification, which may bring a disadvantage from the viewpoint of productivity and cost.

The above-described particle sizes and the like, that is, the above-described (i) to (iv) are applied not only to the water-absorbent resin before surface crosslinking, but also to the water-absorbent resin after surface crosslinking and the water-absorbing agent composition. Thus, it is preferred to perform the surface crosslinking treatment, that is, the surface crosslinking step so as to maintain the particle size within the above-described range adjusted with the water-absorbent resin before surface crosslinking, and it is more preferred to perform the particle size adjustment by providing the particle size adjustment step after the surface crosslinking step. In addition, the above-described (i) and (iv), (ii) and (iv), (iii) and (iv), and the like can be freely selected and combined, and in such cases, the respective preferred ranges can be freely combined.

### [2-6] Surface Crosslinking Step

This step is a step of further providing a portion having a high crosslinking density on the surface layer of the water-absorbent resin before surface crosslinking obtained through the above-described steps, and includes a mixing step and a heat treatment step. In the surface crosslinking step, radical crosslinking, surface polymerization, a crosslinking reaction with a surface crosslinking agent, and the like occur on the surface of the water-absorbent resin before the surface crosslinking step, and thus a surface crosslinked water-absorbent resin is obtained. In the production method according to the present invention, an aqueous solution of a flowability improver may be added in the surface crosslinking step. When the flowability improver is added in the surface crosslinking step like this, the water-absorbent resin obtained after the surface crosslinking step contains the flowability improver. Thus, in this case, the "water-absorbing agent composition" may be a water-absorbent resin after the surface crosslinking step.

### [2-6-1] Mixing Step

This step is a step of obtaining a humidified mixture by mixing a solution containing a surface crosslinking agent (hereinafter, referred to as "surface crosslinking agent solution") with the water-absorbent resin before surface crosslinking in a mixing apparatus. When an aqueous solution of the flowability improver is added in the surface crosslinking step, it is preferred to add the aqueous solution in this step. Preferred conditions and the like at this time are as described in [2-9] described later.

### Surface Crosslinking Agent

In the present invention, a surface crosslinking agent is preferably used at the time of surface crosslinking. Specific examples of the surface crosslinking agent include a polyhydric alcohol compound, an amino alcohol compound, an alkylene carbonate compound, an oxazolidinone compound, an oxetane compound, and an epoxy compound. It is preferred to use at least one or more surface crosslinking agents selected from these surface crosslinking agents. As the surface crosslinking agent, an organic surface crosslinking agent capable of forming an ester bond with a carboxyl group is preferred. Examples of the surface crosslinking agent that forms an ester bond (preferably, a dehydrated ester bond) with a functional group of the polyacrylic acid (salt)-based water-absorbent resin, for example, a carboxyl group, include surface crosslinking agents having a hydroxyl group in the molecule, such as a polyhydric alcohol compound or an amino alcohol compound, and surface crosslinking agents that generate a hydroxyl group via ring-opening, such as an alkylene carbonate compound, an oxazolidinone compound, an oxetane compound, and an epoxy compound.

More specific examples of the surface crosslinking agent include polyhydric alcohol compounds such as ethylene glycol, diethylene glycol, triethylene glycol, tetraethylene glycol, polyethylene glycol, propylene glycol, 1,3-propanediol, 1-methyl-1,3-propanediol, 2-methyl-1,3-propanediol, dipropylene glycol, 2,2,4-trimethyl-1,3-pentanediol, 2,3,4-trimethyl-1,3-pentanediol, polypropylene glycol, glycerin, polyglycerin, 2-butene-1,4-diol, 1,4-butanediol, 1,3-butanediol, 1,5-pentanediol, 1,6-hexanediol, 1,2-cyclohexanemethanol, 1,2-cyclohexanedimethanol, 1,2-cyclohexanediol, trimethylolpropane, diethanolamine, triethanolamine, polyoxypropylene, oxyethylene-oxypropylene block copolymers, pentaerythritol, meso erythritol, D-sorbitol, and sorbitol; epoxy compounds such as ethylene glycol diglycidyl ether, polyethylene glycol diglycidyl ether, glycerol polyglycidyl ether, diglycerol polyglycidyl ether, polyglycerol polyglycidyl ether, propylene glycol diglycidyl ether, polypropylene glycol diglycidyl ether, and glycidol; polyvalent amine compounds such as ethylenediamine, diethylenetriamine, triethylenetetramine, tetraethylenepentamine, pentaethylenehexamine, polyethyleneimine, and polyamidepolyamine, and inorganic salts or organic salts thereof, for example, aziridinium salts, and the like; polyvalent isocyanate compounds such as 2,4-tolylene diisocyanate and hexamethylene diisocyanate; haloepoxy compounds such as epichlorohydrin, epibromhydrin, and α-methylepichlorohydrin; polyvalent oxazoline compounds such as 1,2-ethylene bisoxazoline; oxazolidinone compounds such as N-acyl oxazolidinone and 2-oxazolidinone; alkylene carbonate compounds such as 1,3-dioxolan-2-one, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, 1,3-dioxopan-2-one; cyclic urea compounds; oxetane compounds such as oxetane, 2-methyloxetane, 3-methyl-3-hydroxymethyloxetane, and 3-ethyl-3-hydroxymethyloxetane; amino alcohol compounds such as ethanolamine; and polyvalent metal compounds, such as hydroxides or chlorides of zinc, calcium, magnesium, aluminum, iron, zirconium, and the like.

Among these surface crosslinking agents, at least one surface crosslinking agent selected from the group consisting of a polyhydric alcohol compound, an epoxy compound, a polyvalent amine compound and salts thereof, an oxetane compound, and an alkylene carbonate compound is suitable. More preferably, the surface crosslinking agent is one or more selected from the group consisting of a polyhydric alcohol compound having from 3 to 6 carbon atoms and from 2 to 3 hydroxyl groups contained in the molecule, an epoxy compound having from 6 to 12 carbon atoms, an alkylene carbonate compound having from 3 to 5 carbon atoms, and an oxetane compound having from 3 to 10 carbon atoms. As the surface crosslinking agent described above, one or two or more surface crosslinking agents are used in consideration of the reactivity thereof and the heating temperature in the heat treatment step. The surface crosslinking step may be performed twice or more in consideration of the effect, and in this case, the second and subsequent steps may be performed using the same surface crosslinking agent as in the first step, or may be performed using different surface crosslinking agents.

The lower limit value of the amount of the surface crosslinking agent used is preferably 0.01 parts by mass or more relative to 100 parts by mass of the water-absorbent resin before surface crosslinking. On the other hand, the upper limit value thereof is preferably 10 parts by mass or less, more preferably 5 parts by mass or less, still more preferably 2 parts by mass or less, relative to 100 parts by mass of the water-absorbent resin before surface crosslinking. A preferred range of the amount of the surface crosslinking agent used can be a range defined by any combination selected from the upper and lower limit values described above. By setting the amount of the surface crosslinking agent used within the ranges, an optimum crosslinked structure can be formed on the surface layer of the water-absorbent resin before surface crosslinking, and a water-absorbent resin and a water-absorbing agent composition having higher physical properties are more easily obtained. The amount used when a plurality of surface crosslinking agents are used is the total amount thereof.

The surface crosslinking agent is preferably added to the water-absorbent resin in a solution state, more preferably added to the water-absorbent resin before surface crosslinking as an aqueous solution. Thus, when a flowability improver is added in the surface crosslinking step, it is preferred to further contain a flowability improver in the aqueous solution of the surface crosslinking agent. When the surface crosslinking agent is added as an aqueous solution, the lower limit value of the amount of water used is preferably 0.1 parts by mass or more, more preferably 0.3 parts by mass or more, still more preferably 0.5 parts by mass or more, relative to 100 parts by mass of the water-absorbent resin before surface crosslinking. On the other hand, the upper limit value thereof is preferably 20 parts by mass or less, more preferably 15 parts by mass or less, still more preferably 10 parts by mass or less, relative to 100 parts by mass of the water-absorbent resin before surface crosslinking. A preferred range of the amount of water used can be a range defined by any combination selected from the upper and lower limit values described above. By setting the amount of water used within the above range, the handleability of the surface crosslinking agent solution is further improved, and the surface crosslinking agent is easily mixed uniformly with the water-absorbent resin before surface crosslinking.

The lower limit value of the concentration of the surface crosslinking agent in the surface crosslinking agent solution is preferably 0.1 mass% or more, more preferably 10 mass% or more, still more preferably 15 mass% or more, particularly preferably 20 mass% or more. On the other hand, the upper limit value thereof is preferably 60 mass% or less, more preferably 50 mass% or less, still more preferably 45 mass% or less. A preferred range of the concentration of the surface crosslinking agent to be used can be a range defined by any combinations selected from the upper and lower limit values. By setting the concentration of the surface crosslinking agent within the above-described ranges, an optimum crosslinked structure can be formed on the surface layer of the water-absorbent resin before surface crosslinking, the water-absorbent resin having a high specific surface area, and physical properties such as water absorption performance can be improved.

The surface crosslinking agent solution can also be obtained by using a hydrophilic organic solvent in combination with the water as necessary. In this case, the amount of the hydrophilic organic solvent used is preferably 5 parts by mass or less, more preferably 3 parts by mass or less, still more preferably 1 part by mass or less, relative to 100 parts by mass of the water-absorbent resin before surface crosslinking. Specific examples of the hydrophilic organic solvent include lower alcohols such as methyl alcohol; ketones such as acetone; ethers such as dioxane; amides such as N,N-dimethylformamide; sulfoxides such as dimethyl sulfoxide; and polyhydric alcohols such as ethylene glycol. These hydrophilic organic solvents contribute as a mixing aid for uniformly dispersing the surface crosslinking agent on the surface of the water-absorbent resin, but this leads to an increase in cost from a commercial viewpoint, and thus, it is preferred that the amount of the hydrophilic organic solvent used is limited to as small as possible when used.

### Mixing Method and Mixing Conditions

Examples of the method for mixing the water-absorbent resin before surface crosslinking with the surface crosslinking agent solution include a method in which a surface crosslinking agent solution is prepared in advance, and the solution is preferably sprayed or dropped onto the water-absorbent resin before surface crosslinking, and more preferably sprayed and mixed. When a flowability improver is added in the surface crosslinking step, the surface crosslinking agent solution preferably further contains a flowability improver. Preferred conditions and the like at this time are as described in [2-9] described later.

As the mixing apparatus that performs the mixing, a mixing apparatus having a torque necessary for uniformly and reliably mixing the water-absorbent resin before surface crosslinking and the surface crosslinking agent is preferred. The mixing apparatus is preferably a high-speed stirring type mixer, and more preferably a high-speed stirring type continuous mixer. The lower limit value of the rotational speed of the high-speed stirring type mixer is preferably 100 rpm or more, more preferably 300 rpm or more. On the other hand, the upper limit value thereof is preferably 10000 rpm or less, and more preferably 2000 rpm or less. A preferred range of the rotational speed of the high-speed stirring type mixer can be a range defined by any combinations selected from the upper and lower limit values.

The lower limit value of the temperature of the water-absorbent resin before surface crosslinking to be supplied to this step is preferably 25°C or higher, more preferably 35°C or higher, from the viewpoint of the mixing property with the surface crosslinking agent solution and the cohesiveness of the humidified mixture. On the other hand, the upper limit value thereof is preferably 80°C or lower, more preferably 70°C or lower, still more preferably 60°C or lower. The lower limit value of the mixing time is preferably 1 second or more, more preferably 5 seconds or more. On the other hand, the upper limit value thereof is preferably 1 hour or less, more preferably 10 minutes or less. A preferred range of the temperature and mixing time of the water-absorbent resin before surface crosslinking can be a range defined by any combinations selected from the upper and lower limit values.

### [2-6-2] Heat Treatment Step

This step is a step of applying heat to the humidified mixture obtained in the mixing step to cause a crosslinking reaction on the surface of the water-absorbent resin before surface crosslinking. In the heat treatment on the humidified mixture, the humidified mixture may be heated in a stationary state or may be heated in a flowing state using power such as stirring, but it is preferred to heat the humidified mixture under stirring in that the entire humidified mixture can be uniformly heated. Specific examples of the heat treatment apparatus that performs the heat treatment include a paddle dryer, a multi-fin processor, and a towered dryer.

The lower limit value of the heating temperature in this step is preferably 80°C or higher, more preferably 90°C or higher. On the other hand, the upper limit value thereof is preferably 250°C or lower, more preferably 230°C or lower. A preferred range of the heating temperature can be a range defined by any combinations selected from the upper and lower limit values.

The heating time in this step is preferably 5 minutes or more, more preferably 7 minutes or more. On the other hand, the upper limit value thereof is preferably 1.5 hours or less, more preferably 1 hour or less. A preferred range of the heating time can be a range defined by any combinations selected from the upper and lower limit values.

By controlling the heating temperature and the heating time to be within the above-described ranges, the resulting water-absorbing agent composition or the water-absorbent resin after surface crosslinking is preferably improved in water absorption performance.

### [2-7] Cooling Step

After the heat treatment (heat treatment step) is performed in the surface crosslinking step, a cooling step may be performed as necessary. That is, the cooling step is an optional step provided as necessary after the heat treatment step in the surface crosslinking step. This step is a step of forcibly cooling the water-absorbent resin after surface crosslinking after the heat treatment step to a predetermined temperature to quickly terminate the surface crosslinking reaction.

The water-absorbent resin after surface crosslinking may be cooled in a stationary state or may be cooled in a flowing state using power such as stirring, but it is preferred to cool the water-absorbent resin under stirring from the viewpoint of uniformly cooling the entire water-absorbent resin. From the above viewpoint, examples of the cooling apparatus that performs the cooling include a paddle dryer, a multi-fin processor, and a towered dryer. These cooling apparatuses may have the same specifications as those of the heat treatment apparatus used in the heat treatment step. This is because the heat treatment apparatus can be used as a cooling apparatus by changing the heat medium of the heat treatment apparatus to a refrigerant.

The cooling temperature in this step may be appropriately set according to the heating temperature in the heat treatment step, the water absorption performance of the water-absorbing agent composition or the water-absorbent resin after surface crosslinking, and the like. Specifically, the temperature of the water-absorbent resin after surface crosslinking is preferably 150°C or lower, more preferably 100°C or lower, still more preferably 90°C or lower, particularly preferably 80°C or lower. On the other hand, the lower limit value thereof is preferably 20°C or higher, more preferably 30°C or higher.

In the production method according to the present invention, an aqueous solution of a flowability improver is added to the water-absorbent resin in the surface crosslinking step or in a step after the surface crosslinking step. At this time, the water-absorbent resin having a high specific surface area has a higher water absorption speed than known products, and thus, it is difficult to uniformly apply the aqueous solution of the flowability improver between particles. In addition, the water absorption speed of the water-absorbent resin is also affected by temperature. Thus, when an aqueous solution of the flowability improver is added to and mixed with the water-absorbent resin having a too high temperature, a part of the water-absorbent resin may absorb the aqueous solution of the flowability improver. Then, the surface of the water-absorbent resin becomes tacky, aggregated coarse particles are easily generated, and the uniformity after mixing may deteriorate. Thus, particularly in consideration of the mixing property between the water-absorbent resin having a high specific surface area and the aqueous solution of the flowability improver, the temperature of the water-absorbent resin after surface crosslinking when the aqueous solution of the flowability improver is added is preferably controlled within the above-described ranges. By controlling the temperature within the above-described ranges, the aqueous solution of the flowability improver can be uniformly added to and mixed with the water-absorbent resin, and the kinetic friction coefficient of particles of the water-absorbent resin having a particle diameter of 300 µm or more and less than 600 µm is easily reduced.

The shape of the water-absorbent resin after surface crosslinking may be any of a spherical shape, a granulated product, an aggregate, an irregularly crushed shape, and the like, but in consideration of the water absorption speed of the water-absorbent resin, an irregularly crushed shape is preferred. When the water-absorbent resin is crushed or the like after surface crosslinking, the surface crosslinking effect is reduced. Thus, the shape of the water-absorbent resin before and after the surface crosslinking is preferably an irregularly crushed shape. Specifically, in consideration of the fact that the shape of the water-absorbent resin in the surface crosslinking step of the water-absorbent resin is in an irregularly crushed shape, and the effect of adding the aqueous solution of the flowability improver, the shape of the water-absorbent resin when the aqueous solution of the flowability improver is added in the step of mixing the aqueous solution of the flowability improver is also preferably an irregularly crushed shape. The water-absorbent resin in an irregularly crushed shape can be obtained by pulverizing a hydrogel or a dried polymer.

The proportion of particles of the water-absorbent resin having a particle diameter of less than 150 µm (hereinafter, referred to as "particles of less than 150 µm") contained in the water-absorbent resin after surface crosslinking is preferably less than 3 mass%. Particles having a particle diameter of less than 150 µm have a remarkably increased specific surface area as compared with particles having a particle diameter of 150 µm or more, and thus have a high aqueous liquid absorption speed. Thus, when there are many particles of less than 150 µm (for example, when the mass proportion is 3 mass% or more), these particles preferentially absorb the aqueous solution of the flowability improver. Then, when the aqueous solution of the flowability improver is added, the aqueous solution may be difficult to uniformly mix with the entire water-absorbent resin after surface crosslinking, or coarse particles in which particles of less than 150 µm are aggregated may be generated. Thus, in the water-absorbent resin before the aqueous solution of the flowability improver is added, the proportion of particles of the water-absorbent resin having a particle diameter of less than 150 µm is preferably less than 3 mass%.

Particles having a particle diameter of less than 150 µm can be appropriately adjusted by employing the same method for adjusting the particle diameter of the water-absorbent resin as in the classification step. As a preferred form of the water-absorbent resin after surface crosslinking, the descriptions of (i) to (iv) described in the section "[2-5] pulverizing step and Classification Step" described above are cited. Suitable ranges for "(i) the proportion of particles less than 150 µm", "(ii) D50 (mass-average particle diameter)", "(iii) D50 (mass-average particle diameter) and the proportion of particles less than 150 µm", "(iv) σζ (logarithmic standard deviation of particle size distribution)", and combinations thereof are as described above. In particular, when (iv) σζ (logarithmic standard deviation of particle size distribution) is within the above-described desired ranges, the variation in the specific surface area between particles is small, the absorption speed of the aqueous liquid is also small, and the particles are easily mixed uniformly when the aqueous solution of the flowability improver is added, which is preferred.

### [2-8] Additive and Step of Adding Additive

In the present invention, an additive other than the flowability improver (hereinafter, it is also simply referred to as "additive") may be added to any one or more of the water-absorbent resin before surface crosslinking and the water-absorbent resin after surface crosslinking. In other words, the water-absorbing agent composition may contain an additive in addition to the water-absorbent resin and the flowability improver.

### [2-8-1] Additive

Examples of the additive other than the flowability improver used in the present invention include a liquid permeability improver or an agent having a similar component, and other additives. One of these may be used, or two or more thereof may be used in combination.

### Liquid Permeability Improver or Agent Having Similar Component

Examples of the liquid permeability improver used in the present invention include an additive having a function of improving the saline flow conductivity (hereinafter, referred to as "SFC") and the gel bed permeability (hereinafter, referred to as "GBP") under a load or no load of the water-absorbing agent composition or the water-absorbent resin. For example, at least one compound selected from a polyvalent metal salt, a cationic polymer (excluding the flowability improver described in detail below), and inorganic fine particles can be used, and two or more compounds can be used in combination as necessary.

These additives may be used to exhibit other functions, such as functions of an anti-Caking agent under moisture absorption and a binder of a water-absorbent resin, without intending to improve liquid permeability. When an additive is added for the purpose of other functions, it is referred to as an agent having a similar component. The amount of the liquid permeability improver or the agent having a similar component is appropriately set according to the selected compound. In addition to the case of using one of these additives alone, the range of suitable amounts added in the case of using two or more additives in combination can be appropriately selected within the range described below.

"SFC" is an abbreviation for Saline Flow Conductivity, and is liquid permeability of a 0.69 mass% aqueous sodium chloride solution to the water-absorbing agent composition or the water-absorbent resin under a load of 2.07 kPa.

"GBP" is an abbreviation for Gel Bed Permeability, which is the liquid permeability of a 0.9 mass% aqueous sodium chloride solution to the water-absorbing agent composition or the water-absorbent resin under a load or under free swelling, and is a value measured in accordance with the GBP test method described in WO 2005/016393.

### (Polyvalent Metal Salt)

When a polyvalent metal salt is used, the polyvalent metal cation of the polyvalent metal salt preferably has divalent or higher valence, more preferably has trivalent or higher valence, and preferably has tetravalent or lower valence. Examples of the polyvalent metal that can be used include aluminum and zirconium. Thus, examples of the polyvalent metal salt that can be used in this step include aluminum lactate, zirconium lactate, aluminum sulfate, and zirconium sulfate. Among these, from the viewpoint of the effect of improving SFC, aluminum lactate or aluminum sulfate is more preferred, and aluminum sulfate is still more preferred. The amount of the polyvalent metal salt added is preferably 0 mol or more and less than 3.6 × 10⁻⁵ mol, more preferably 0 mol or more and less than 1.4 × 10⁻⁵ mol, still more preferably 0 mol or more and less than 1.0 × 10⁻⁵ mol, relative to 1 g of the water-absorbent resin.

### Cationic Polymer

When a cationic polymer is used, examples of the cationic polymer include the substances described in US 7098284. In particular, a vinylamine polymer is more preferred from the viewpoint of the effect of improving SFC and GBP. The mass-average molecular weight of the cationic polymer is preferably 5000 or more and 1000000 or less.

The lower limit value of the amount of the cationic polymer added is preferably 0 parts by mass or more, more preferably more than 0 parts by mass, relative to 100 parts by mass of the water-absorbent resin. On the other hand, the upper limit value thereof is preferably less than 2.5 parts by mass, more preferably less than 2.0 parts by mass, still more preferably less than 1.0 parts by mass, relative to 100 parts by mass of the water-absorbent resin. A preferred range of the amount of the cationic polymer added can be a range defined by any combinations selected from the upper and lower limit values.

### Inorganic Fine Particles

When an inorganic fine particle is used, examples of the inorganic fine particle include substances described in US 7638570. In particular, silicon dioxide is preferred from the viewpoint of the effect of improving SFC and GBP.

When the primary particle diameter of the inorganic fine particles is less than 20 nm, the lower limit value of the amount of the inorganic fine particles added is preferably 0 parts by mass or more, more preferably more than 0 parts by mass, relative to 100 parts by mass of the water-absorbent resin. On the other hand, the upper limit value thereof may be preferably less than 1.2 parts by mass, more preferably less than 1.0 parts by mass, still more preferably less than 0.5 parts by mass, relative to 100 parts by mass of the water-absorbent resin. When the primary particle diameter of the inorganic fine particles is 20 nm or more, the lower limit value of the amount of the inorganic fine particles added is preferably 0 parts by mass or more, more preferably more than 0 parts by mass, relative to 100 parts by mass of the water-absorbent resin. On the other hand, the upper limit value thereof may be preferably less than 2.0 parts by mass, more preferably less than 1.5 parts by mass, still more preferably less than 1.0 parts by mass, relative to 100 parts by mass of the water-absorbent resin. A preferred range of the amount of the inorganic fine particles added can be a range defined by any combinations selected from the upper and lower limit values.

### Other additives

Specific examples of the other additives include chelating agents, inorganic reducing agents, aromatic substances, organic reducing agents, hydroxycarboxylic acid compounds, compounds having a phosphorus atom, oxidizing agents, organic powders such as metal soaps, deodorants, antibacterial agents, pulps, and thermoplastic fibers. One or more of these other additives can be used. Among these, a chelating agent is preferred, and an amino polycarboxylic acid or an amino polyphosphoric acid is more preferred. Specific examples of the chelating agent include chelating agents described in JP 11-060975 A, WO 2007/004529, WO 2011/126079, WO 2012/023433, JP 2009-509722 T, JP 2005-097519 A, JP 2011-074401 A, JP 2013-076073 A, JP 2013-213083 A, JP 59-105448 A, JP 60-158861 A, JP 11-241030 A, JP 2-41155 A, and the like.

Another additives, in particular, a chelating agent, is added or contained in an amount of preferably 0.001 mass% or more and 1 mass% or less relative to the monomer or the water-absorbent resin.

### [2-8-2] Step of Adding Additive

The additive can be added before or after or in the middle of at least one step selected from the step of preparing an aqueous monomer solution, the polymerization step, the gel-grinding step, the drying step, the pulverizing step, the classification step, and the surface crosslinking step. That is, in one embodiment, the production method according to the present invention may further include a step of adding an additive in addition to the step of preparing an aqueous monomer solution, the polymerization step, the gel-grinding step, the drying step, the pulverizing step, the classification step, the surface crosslinking step, and the step of adding an aqueous solution of a flowability improver flowability improver. Preferably, the additive is added before, after, or in the middle of any step after the polymerization step.

When the additive is added to the water-absorbent resin, and the additive is a liquid or a solution of an aqueous medium such as water, it is preferred to spray the liquid or solution onto the water-absorbent resin and apply sufficient torque to uniformly and reliably mix the water-absorbent resin and the additive. On the other hand, when the additive is in a solid form such as a powder form, the additive may be dry-blended with the water-absorbent resin, or an aqueous liquid such as water may be used as a binder.

Specific examples of the apparatus used for the mixing include a stirring type mixer, a cylindrical mixer, a double-wall conical mixer, a V-shaped mixer, a ribbon-shaped mixer, a screw type mixer, a flow type rotary disk-type mixer, a flow-type mixer, a double-arm type kneader, an internal mixer, a pulverizing type kneader, a rotary mixer, and a screw type extruder. In the case of using a stirring type mixer, the rotational speed thereof is preferably 5 rpm or more, more preferably 10 rpm or more, and preferably 10000 rpm or less, more preferably 2000 rpm or less.

### [2-9] Aqueous Solution of Flowability Improver and Step of Adding Aqueous Solution of Flowability Improver

This step is a step of adding a flowability improver in an aqueous solution state to the water-absorbent resin having a high specific surface area obtained through the above-described steps, and is performed in the surface crosslinking step or in a step after the surface crosslinking step. Here, "performing in a step after the surface crosslinking step" includes not only providing a step of adding the aqueous solution after the surface crosslinking step but also performing addition of the aqueous solution of the flowability improver in an optional step (for example, any step such as a cooling step, a rewetting step, or a fine powder granulation step) performed after the surface crosslinking step. According to one embodiment of the present invention, by performing this step under the following specific conditions, in the water-absorbing agent composition having a high specific surface area, the particle size segregation due to transportation can be suppressed, and the flowability of the powder can be further improved. In addition, the water absorption properties of the resulting water-absorbing agent composition are also maintained well. According to another embodiment of the present invention, there is provided a water-absorbing agent composition having a high specific surface area and having a kinetic friction coefficient reduced by 10% or more in particles having a particle diameter of 300 µm or more and less than 600 µm.

One aspect of the present invention is a method for producing a water-absorbing agent composition including a water-absorbent resin as a main component, the method including a step of preparing an aqueous monomer solution, a polymerization step, a gel-grinding step, a drying step, a pulverizing step, a classification step, and a surface crosslinking step,
in which during the surface crosslinking step or during a step after the surface crosslinking step, the method includes a step of mixing, with the water-absorbent resin, a water-soluble flowability improver having a mass-average molecular weight of 200 or more and 50000 or less in an amount of more than 0 ppm and less than 200 ppm relative to a mass of the water-absorbent resin,
the method satisfying all of (a) to (d) described below:
   (a) the water-absorbent resin has a specific surface area of 25 m²/kg or more;
   (b) when the water-soluble flowability improver is mixed with the water-absorbent resin, the water-soluble flowability improver is in a form of an aqueous solution of 0.01 mass% or more and 20 mass% or less;
   (c) when the aqueous solution is added to and mixed with the water-absorbent resin, the aqueous solution has an average droplet diameter of 10 µm or more and 1 mm or less;
   (d) when the aqueous solution is added to and mixed with the water-absorbent resin, a mixing power index defined by (Formula 1) described below is 70000 or more.
$\begin{matrix}Mixing power index \left(unit: none\right) \\ = peripheral speed \left(m/s\right) \times mixing time \left(s\right) / \left\{average droplet diameter\left(mm\right)/1000\right\}\end{matrix}$

### [2-9-1] Water-Absorbent Resin

In one embodiment of the present invention, an aqueous solution of a flowability improver is added to and mixed with the water-absorbent resin. In this case, the water-absorbent resin may be a water-absorbent resin before surface crosslinking or a water-absorbent resin after surface crosslinking, but is preferably a water-absorbent resin after surface crosslinking. That is, the water-absorbent resin to which a flowability improver is added may be a water-absorbent resin (surface crosslinked water-absorbent resin) obtained through the surface crosslinking step. The lower limit value of the specific surface area of the water-absorbent resin is 25 m²/kg or more. When the specific surface area of the water-absorbent resin is less than 25 m²/kg, a water-absorbing agent composition having a sufficient water absorption speed (Vortex) cannot be obtained. The specific surface area of the water-absorbent resin to be used for the production of the water-absorbing agent composition may be set according to a desired specific surface area of the water-absorbing agent composition. A higher specific surface area of the water-absorbent resin is more preferred. The specific surface area is preferably 26 m²/kg or more, more preferably 27 m²/kg or more, still more preferably 28 m²/kg or more, yet still more preferably 29 m²/kg or more, yet still more preferably 30 m²/kg or more, particularly preferably 35 m²/kg or more, most preferably 36 m²/kg or more. On the other hand, the upper limit value thereof is preferably 60 m²/kg or less, more preferably 55 m²/kg or less. A preferred range of the specific surface area can be a range defined by any combinations selected from the upper and lower limit values. Thus, the specific surface area of the water-absorbent resin may be, for example, 25 m²/kg or more and 60 m²/kg or less, 26 m²/kg or more and 60 m²/kg or less, 27 m²/kg or more and 60 m²/kg or less, 28 m²/kg or more and 60 m²/kg or less, 29 m²/kg or more and 60 m²/kg or less, 30 m²/kg or more and 55 m²/kg or less, 35 m²/kg or more and 55 m²/kg or less, or 36 m²/kg or more and 55 m²/kg or less.

The physical properties of the water-absorbent resin when the aqueous solution of the flowability improver is added preferably satisfy the physical properties disclosed in "[2-5] pulverizing step and Classification Step" described, and for example, (i) to (iv) described above can be applied. In particular, it is more preferred that, as the particle size distribution of the water-absorbent resin, D50 (mass-average particle diameter) falls within the range of (ii), and the proportion of particles having a particle diameter of less than 150 µm falls within the range of (i).

The production method according to the present invention preferably further satisfies the following (e) in addition to the above (a) to (d): (e) the particles having a particle diameter of 300 µm or more and less than 600 µm in the water-absorbent resin after addition of the water-soluble flowability improver has a kinetic friction coefficient of 0.80 or less. The kinetic friction coefficient can be measured as the kinetic friction coefficient of particles having a particle diameter of 300 µm or more and less than 600 µm in the water-absorbing agent composition, and is, for example, 0.10 or more and 0.80 or less, preferably 0.30 or more and 0.79 or less, more preferably 0.50 or more and 0.78 or less, still more preferably 0.60 or more and 0.77 or less, particularly preferably 0.60 or more and 0.76 or less, most preferably 0.60 or more and 0.73 or less. Detailed measurement conditions of the kinetic friction coefficient of the particles having a particle diameter of 300 µm or more and less than 600 µm are described in Examples.

### [2-9-2] Flowability Improver

In the production method according to the present invention, (an aqueous solution of) a flowability improver is added to the water-absorbent resin under specific conditions. In the present description, the "flowability improver" means a component that is water-soluble and can improve the flowability (reduce the kinetic friction coefficient) of the water-absorbing agent composition (water-absorbent resin) through the addition of the agent. The term "water-soluble" means that 0.1 g or more of the compound is dissolved in 100 g of water at 25°C. The amount of the flowability improver dissolved in 100 g of water at 25°C is preferably 1 g or more, more preferably 5 g or more. The flowability improver is a paraphrase of and is synonymous with "water-soluble polymer" in the basic application of the present application. In the present description, the "water-soluble polymer" in the basic application of the present application is referred to as "water-soluble flowability improver" or "flowability improver".

The lower limit value of the mass-average molecular weight of the flowability improver is 200 or more, preferably 220 or more, more preferably 250 or more, still more preferably 300 or more. When the mass-average molecular weight of the flowability improver is less than 200, sufficient slidability cannot be imparted to the water-absorbent resin, and it becomes difficult to obtain the effect of improving the flowability during transportation of the water-absorbing agent composition. The upper limit value of the mass-average molecular weight of the flowability improver is 50000 or less, preferably 40000 or less, more preferably 30000 or less, still more preferably 20000 or less, particularly preferably 10000 or less, most preferably 5000 or less. When the mass-average molecular weight of the flowability improver exceeds 50000, the viscosity of the flowability improver itself increases, and it becomes difficult to uniformly add the flowability improver to the water-absorbent resin. A preferred range of the mass-average molecular weight of the flowability improver can be a range defined by any combinations selected from the upper and lower limit values. Thus, the mass-average molecular weight of the flowability improver may be, for example, 200 or more and 50000 or less, 220 or more and 40000 or less, 220 or more and 30000 or less, 250 or more and 20000 or less, 300 or more and 10000 or less, or 300 or more and 5000 or less.

In addition, by using a flowability improver having a mass-average molecular weight within the above-described ranges, the kinetic friction coefficient in particles having a particle diameter of 300 µm or more and less than 600 µm can be significantly reduced. The mass-average molecular weight of the flowability improver is measured by, for example, gel permeation chromatography (hereinafter referred to as GPC) using polyethylene glycol as a standard substance. In the present description, also in the case of referring to the molecular weight of the flowability improver having a single structure (for example, those having a relatively low molecular weight), it is collectively referred to as "mass-average molecular weight". Regarding the molecular weight of such a flowability improver, the molecular weight may be calculated from a chemical formula instead of the measurement by GPC as described above.

According to a preferred embodiment of the present invention, the water-soluble flowability improver may be one or more selected from a nonionic substance, a
zwitterionic substance, an anionic substance, and a cationic substance. In one embodiment, the nonionic substance is selected from (a) polyols, (b) modified products of hydroxy groups of polyols, (c) side-chain and/or terminal polyether-modified polysiloxanes, and (d) alkylene oxide adducts of a higher aliphatic amine. The
zwitterionic substance is selected from (e) alkyl betaines, and (f) alkylamine oxides, the anionic substance is selected from (g) alkyl sulfate ester salts, (h) sulfate ester salts of higher alcohol alkylene oxide adducts, (i) sulfonic acid salts, (j) dicarboxylic acid salts, (k) alkylamine diacetic acid salts, (l) phosphoric acid ester salts of higher alcohol alkylene oxide adducts, and (m) carboxylic acid salts of higher alcohol alkylene oxide adducts. The cationic substance is selected from (n) ammonium salts. With such a configuration, the desired effect of the present invention can be efficiently exhibited.

### (a) Polyols

According to a preferred embodiment of the invention, "polyols" means compounds having a plurality of hydroxy groups. Specific examples thereof include polyalkylene glycols such as polyethylene glycol and polypropylene glycol, and polyalkylene glycols such as block copolymers or random copolymers of polyethylene glycol and polypropylene glycol. Here, the number of carbon atoms of the alkylene unit of the repeating unit in the polyalkylene glycol is preferably from C1 to C6, more preferably from C2 to C4, and particularly preferably from C2 to C3 (In the present description, the number of carbon atoms may be represented by adding a number after "C". For example, 1 carbon atom may be denoted as C1, and 10 carbon atoms may be denoted as C10). With such a configuration, the kinetic friction coefficient in particles having a particle diameter of 300 µm or more and less than 600 µm can be significantly reduced.

Polyalkylene glycols such as block copolymers or random copolymers of polyethylene glycol and polypropylene glycol can be readily available from the market, and for example, the following products are preferably exemplified.

Products available from ADEKA Corporation
   - Pluronic (trade name, the same applies hereinafter) series
      Pluronic L-34, Pluronic L-44, Pluronic L-64, Pluronic P-84, Pluronic P-85, Pluronic P-103, Pluronic F-68, Pluronic F-88, Pluronic F-108, Pluronic 17R-3, Pluronic 17R-4, Pluronic TR-704, Pluronic TR-913R
Products available from NOF Corporation
   PRONON (trade name, the same applies hereinafter) #104, PRONON #204, PRONON #208, UNILUBE 70DP-600B, UNILUBE 70DP-950B
Products available from DKS Co., Ltd.
   EPAN (trade name, the same applies hereinafter) 450, EPAN 485, EPAN 680, EPAN 740, EPAN 750, EPAN 785, EPAN U-103, EPAN U-105, EPAN U-108

### (b) Modified product of hydroxy group of polyol

According to a preferred embodiment of the present invention, the "modified product of a hydroxy group of a polyol" means a compound in which one or more hydroxy groups contained in a polyol are ester-modified and/or ether-modified. The modification of the ester and/or ether is preferably performed by a hydrocarbon group. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms is C30 or less, the hydrophobicity is not too strong, and the surface tension can be favorably maintained.

The hydrocarbon group is not limited to a linear hydrocarbon group, but it may be a branched or cyclic saturated hydrocarbon group and/or unsaturated hydrocarbon group, or an aromatic hydrocarbon group such as a phenyl group or an alkylphenyl group. Further, the hydrocarbon group may have a reactive functional group such as a hydroxy group, an amino group, or a glycidyl group.

According to a preferred embodiment of the present invention, examples of (b) modified products of hydroxy groups of polyols include (b-1) glycidyl-modified polyols, (b-2) alkylene oxide adducts of higher alcohols, and (b-3) alkylene oxide adducts of polyhydric alcohol fatty acid esters. (b-1) may be a compound in which at least one terminal of the (poly)alkylene glycol is modified with a glycidyl group. (b-2) may be a compound in which one terminal of the (poly)alkylene glycol is modified with a substituent having a C1-C30 hydrocarbon group. (b-3) may be a compound in which an alkylene oxide is added to at least one hydroxy group of the polyhydric alcohol and which is modified with a substituent having a C1-C30 hydrocarbon group via an ester bond to at least one hydroxy group of the polyhydric alcohol. The polyhydric alcohol in (b-3) may be glycerin, pentaerythritol, sorbitol, sorbitan, or a saccharide. With such a configuration, the kinetic friction coefficient in particles having a particle diameter of 300 µm or more and less than 600 µm can be significantly reduced.

### (b-1) Glycidyl-Modified Polyols

The glycidyl-modified polyols are compounds in which at least one terminal of a (poly)alkylene glycol is modified with a glycidyl group. Specific examples thereof include water-soluble polyglycidyl ethers of polyols such as water-soluble (poly)alkylene glycol diglycidyl ethers, for example, diethylene glycol diglycidyl ether and polyethylene glycol diglycidyl ether, polypropylene glycol diglycidyl ether, hexanediol diglycidyl ether, glycerol polyglycidyl ether, trimethylolpropane polyglycidyl ether, pentaerythritol polyglycidyl ether, diglycerol polyglycidyl ether, polyglycerol polyglycidyl ether, and sorbitol polyglycidyl ether.

Glycidyl-modified polyols can be readily available from the market, and for example, the following products are preferably exemplified.

### · Products available from Nagase ChemteX Corporation

DENACOL (trade name, the same applies hereinafter) EX-145, DENACOL EX-171, DENACOL EX-211, DENACOL EX-212, DENACOL EX-252, DENACOL EX-810, DENACOL EX-811, DENACOL EX-850, DENACOL EX-851, DENACOL EX-821, DENACOL EX-830, DENACOL EX-832, DENACOL EX-841, DENACOL EX-861, DENACOL EX-911, DENACOL EX-941, DENACOL EX-920, DENACOL EX-931, DENACOL EX-313, DENACOL EX-314, DENACOL EX-321, DENACOL EX-411, DENACOL EX-421, DENACOL EX-512, DENACOL EX-521, DENACOL EX-612, DENACOL EX-614, DENACOL EX-614B.

### (b-2) Alkylene Oxide Adduct of Higher Alcohol

The alkylene oxide adduct of a higher alcohol may be a compound modified with a substituent having a C1-C30 (preferably C6-C30) hydrocarbon group at one terminal of a (poly)alkylene glycol. In one embodiment, the alkylene oxide adduct of the higher alcohol is preferably a compound represented by the following general formula (Chem. 1).

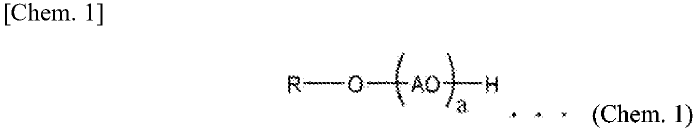

In the above formula (Chem. 1), R is a C1-C30 (preferably C6-C30) hydrocarbon group. The hydrocarbon group may be a linear, branched or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained. The hydrocarbon group is preferably a linear or branched saturated hydrocarbon group.

In the above formula, (AO) is a repeating unit that can also be represented by CₙH₂ₙO (n is a natural number). The number of carbon atoms constituting AO (n described above) is preferably from C1 to C6, more preferably from C1 to C3, still more preferably from C2 to C3, particularly preferably C2. That is, the repeating unit (AO) in the above general formula (Chem. 1) is particularly preferably [CH₂CH₂O], and the repeating unit may have a structure derived from ethylene oxide addition or condensation of ethylene glycol.

In the above formula, a represents the number of the repeating units (AO), and when a is 2 or more, the number of carbon atoms constituting each repeating unit may be the same or different. That is, the alkylene oxide adduct of a higher alcohol represented by the above general formula (Chem. 1) may be a polymer having the same repeating unit (AO), or may be a block polymer or a random polymer having different repeating units (AO).

In the above formula, a is preferably from 1 to 1000, more preferably from 2 to 500, still more preferably from 2 to 300. When the repeating unit is 1000 or less, the viscosity does not become too high, and it becomes easy to uniformly add the polymer to the water-absorbent resin.

The alkylene oxide adduct of a higher alcohol can be readily available from the market, and for example, the following products are preferred.
Products available from Kao Corporation
   · Polyoxyethylene lauryl ether
      EMULGEN (trade name, the same applies hereinafter) 106 (HLB = 10.5), EMULGEN 108 (HLB = 12.1), EMULGEN 109P (HLB = 13.6), EMULGEN 120 (HLB = 15.3), EMULGEN 123P (HLB = 16.9), EMULGEN 130K (HLB = 18.1), EMULGEN 147 (HLB = 16.3), EMULGEN 150 (HLB = 18.4)
   · Polyoxyethylene polyoxypropylene alkyl ether
      EMULGEN MS-110 (HLB = 12.7)
   · Polyoxyethylene cetyl ether
      EMULGEN 210P (HLB = 10.7), EMULGEN 220 (HLB = 14.2)
   · Polyoxyethylene stearyl ether
      EMULGEN 320P (HLB = 13.9), EMULGEN 350 (HLB = 17.8)
   · Polyoxyethylene oleyl ether
      EMULGEN 408 (HLB = 10.0), EMULGEN 409PV (HLB = 12.0), EMULGEN 420 (HLB = 13.6), EMULGEN 430 (HLB = 16.2)
   · Polyoxyethylene myristyl ether
      EMULGEN 4085 (HLB = 18.9)
   · Polyoxyethylene octyldodecyl ether
      EMULGEN 2020G-HA (HLB = 13.0), EMULGEN 2025G (HLB = 15.7)
      Products available from NOF Corporation
   · Polyoxyethylene isodecyl ether
      Nonionic ID-203 (HLB = 12.5), Nonionic ID-209 (HLB = 14.3)
   · Polyoxyethylene-2-ethylhexyl ether
      Nonionic EH-204 (HLB = 11.5), Nonionic EH-208 (HLB = 14.6)
Products available from Nippon Nyukazai Co., Ltd.
   · Polyoxyethylene nonylphenyl ether
      NEWCOL (trade name, the same applies hereinafter) 560 (HLB = 10.9), NEWCOL 564 (HLB = 12.3), NEWCOL 565 (HLB = 13.3), NEWCOL 566 (HLB = 14.1), NEWCOL 568 (HLB = 15.2), NEWCOL 504 (HLB = 16.0), NEWCOL 506 (HLB = 17.2), NEWCOL 509 (HLB = 18.0), NEWCOL 516 (HLB = 18.8)

### (b-3) Alkylene Oxide Adduct of Polyhydric Alcohol Fatty Acid Ester

The ethylene oxide adduct of polyhydric alcohol fatty acid ester may be a compound in which an alkylene oxide is added to at least one hydroxy group of the polyhydric alcohol and which is modified with a substituent having a C1-C30 hydrocarbon group via an ester bond to at least one hydroxy group of the polyhydric alcohol. Examples of the polyhydric alcohol include glycerin, pentaerythritol, sorbitol, sorbitan, and sugars.

Preferred examples include alkylene oxide adducts of glycerin fatty acid monoesters and alkylene oxide adducts of sorbitan fatty acid monoesters. In one embodiment, the alkylene oxide adduct of the glycerin fatty acid monoester is preferably a compound represented by the following general formula (Chem. 2). The alkylene oxide adduct of a sorbitan fatty acid monoester contains a structural isomer. Thus, in one embodiment, the alkylene oxide adduct of a sorbitan fatty acid monoester may be a compound represented by the following general formula (Chem. 3) or (Chem. 4).

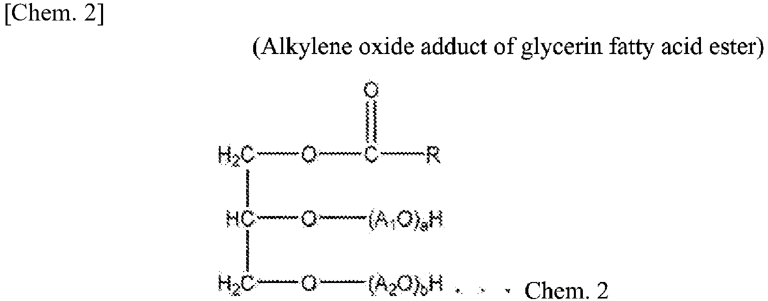

In the above formula (Chem. 2), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, (A₁O) and (A₂O) are repeating units that can also be represented by CₙH₂ₙO (n is a natural number). The number of carbon atoms (n described above) constituting each of A₁O and A₂O is preferably from C1 to C6, more preferably from C1 to C3, still more preferably from C2 to C3, particularly preferably C2. That is, the repeating unit (A₁O or A₂O) in the above general formula (Chem. 2) is particularly preferably [CH₂CH₂O], and the repeating unit may have a structure derived from ethylene oxide addition or condensation of ethylene glycol.

In the above formula, a and b each represent the number of the repeating units (A₁O and A₂O), and when a or b is 2 or more, the number of carbon atoms constituting each repeating unit may be the same or different. In A₁O and A₂O, the number of carbon atoms constituting A₁ and the number of carbon atoms constituting A₂ may be the same or different from each other. That is, the alkylene oxide moiety contained in the above general formula (Chem. 2) may be a polymer having the same repeating unit (A₁O or A₂O), or may be a block polymer or a random polymer having different repeating units (A₁O or A₂O).

In the above formula, the sum (a + b) of a and b is preferably from 1 to 1000, more preferably from 2 to 500, still more preferably from 2 to 300. a and b may be different from or the same as each other. When the total of a + b is 1000 or less, the viscosity does not become too high, and it becomes easy to uniformly add the compound to the water-absorbent resin.

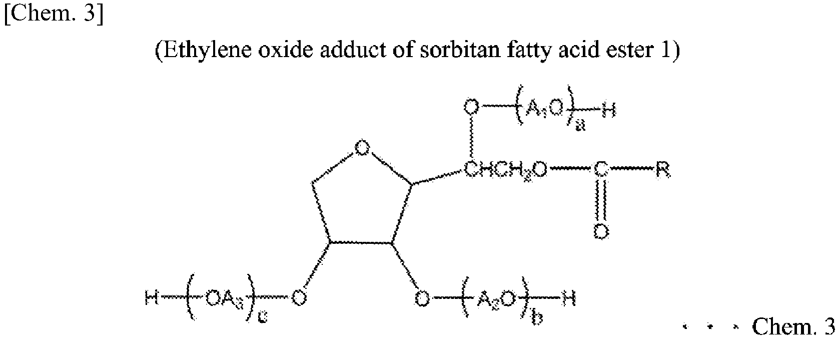

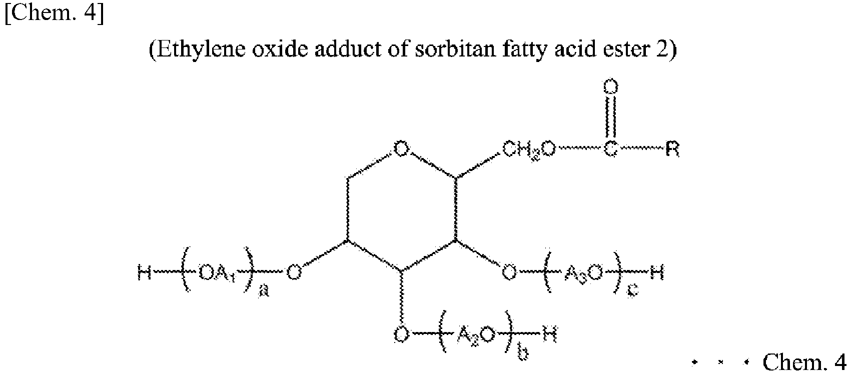

In the above formulas (Chem. 3 and Chem. 4), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained. The hydrocarbon group is preferably a linear or branched saturated hydrocarbon group.

In the above formula, each of (A₁O), (A₂O), and (A₃O) is a repeating unit that can also be represented by CₙH₂ₙO (n is a natural number). The number of carbon atoms (n described above) constituting each of A₁O, A₂O, and A₃O is preferably from C1 to C6, more preferably from C1 to C3, still more preferably from C2 to C3, particularly preferably C2. That is, the repeating unit (A₁O, A₂O, or A₃O) in the above general formulas (Chem. 3) and (Chem. 4) is particularly preferably [CH₂CH₂O], and the repeating unit may have a structure derived from ethylene oxide addition or condensation of ethylene glycol.

In the above formula, a to c each represent the number of the repeating unit (A₁O, A₂O, and A₃O), and when a, b, or c is 2 or more, the number of carbon atoms constituting each repeating unit may be the same or different. In A₁O, A₂O, and A₃O, the number of carbon atoms constituting A₁, the number of carbon atoms constituting A₂, and the number of carbon atoms constituting A₃ may be the same or different from each other. That is, the alkylene oxide moiety contained in the above general formulas (Chem. 3) and (Chem. 4) may be a polymer having the same repeating unit (A₁O, A₂O, or A₃O), or may be a block polymer or a random polymer having different repeating units (A₁O, A₂O, or A₃O).

In the above formula, the sum (a + b + c) of a to c is preferably from 1 to 1000, more preferably from 2 to 500, still more preferably from 2 to 300. a, b, and c may be different from or the same as each other. When the total of a + b + c is 1000 or less, the viscosity does not become too high, and it becomes easy to uniformly add the compound to the water-absorbent resin.

The alkylene oxide adduct of the polyhydric alcohol fatty acid ester can be readily available from the market, and for example, the following products are preferably exemplified.
Products available from Kao Corporation
   · Polyoxyethylene sorbitan monolaurate
      RHEODOL (trade name, the same applies hereinafter) TW-L120 (HLB = 16.7), RHEODOL TW-L106 (HLB = 13.3), RHEODOL Super TW-L120
   · Polyoxyethylene sorbitan monopalmitate
      RHEODOL TW-P120 (HLB = 15.6)
   · Polyoxyethylene sorbitan monostearate
      RHEODOL TW-S120V (HLB = 14.9)
   · Polyoxyethylene sorbitan tristearate
      RHEODOL TW-S320V (HLB = 10.5)
   · Polyoxyethylene sorbitan monooleate
      RHEODOL TW-O120V (HLB = 15.0), RHEODOL TW-O106V (HLB = 10.0)
   · Polyoxyethylene sorbitan triolate
      RHEODOL TW-O320V (HLB = 11.0)
Products available from NOF Corporation
   · Polyoxyethylene coconut fatty acid glyceryl
      UNIGLY (trade name, the same applies hereinafter) MK-207 (HLB = 13.0), UNIGLY MK-230 (HLB = 17.4)

### (c) Side Chain and/or Terminal Polyether-Modified Polysiloxane

According to a preferred embodiment of the present invention, the "side chain and/or terminal polyether-modified polysiloxane" means a compound in which a side chain and/or a terminal of the polysiloxane is polyether-modified. The polyether-modified site of the polysiloxane is not particularly limited, and may be any of a side chain of the polysiloxane, two terminals of the polysiloxane, one terminal of the polysiloxane, and both a side chain and two terminals of the polysiloxane. The polyether-modified group includes a polyoxyethylene group, a polyoxypropylene group, and a group having both a polyoxyethylene group and a polyoxypropylene group. With such a configuration, the kinetic friction coefficient in particles having a particle diameter of 300 µm or more and less than 600 µm can be significantly reduced.

The polyether-modified polysiloxane can be readily available from the market, and for example, the following products are preferably exemplified.
Products available from Shin-Etsu Chemical Co., Ltd.
   KF-351A (HLB = 12), KF-353 (HLB = 10), KF-354L (HLB = 16), KF-355A (HLB = 12), KF-615A (HLB = 10), KF-640 (HLB = 14), KF-642 (HLB = 12), KF-643 (HLB = 14), KF-6011 (HLB = 12)
Products available from Dow Corning Toray Co., Ltd.
   FZ-77 (HLB = 11), L-7604 (HLB = 11)

### (d) Alkylene Oxide Adduct of Higher Aliphatic Amine

According to a preferred embodiment of the present invention, the "alkylene oxide adduct of a higher aliphatic amine" means a compound in which an alkylene oxide is added to two hydrogens on the nitrogen atom of a primary amine having a C1-C30 hydrocarbon group. With such a configuration, the kinetic friction coefficient in particles having a particle diameter of 300 µm or more and less than 600 µm can be significantly reduced.

In one embodiment, the alkylene oxide adduct of the higher aliphatic amine is preferably a compound represented by the following general formula (Chem. 5).

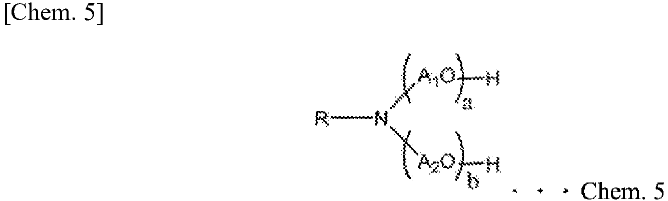

In the above formula (Chem. 5), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, (A₁O) and (A₂O) are repeating units that can also be represented by CₙH₂ₙO (n is a natural number). The number of carbon atoms (n described above) constituting each of A₁O and A₂O is preferably from C1 to C6, more preferably from C1 to C3, still more preferably from C2 to C3, particularly preferably C2. That is, the repeating unit (A₁O or A₂O) in the above general formula (Chem. 5) is particularly preferably [CH₂CH₂O], and the repeating unit may have a structure derived from ethylene oxide addition or condensation of ethylene glycol.

In the above formula, a and b each represent the number of the repeating units (A₁O and A₂O), and when a or b is 2 or more, the number of carbon atoms constituting each repeating unit may be the same or different. In A₁O and A₂O, the number of carbon atoms constituting A₁ and the number of carbon atoms constituting A₂ may be the same or different from each other. That is, the alkylene oxide moiety contained in the above general formula (Chem. 5) may be a polymer having the same repeating unit (A₁O or A₂O), or may be a block polymer or a random polymer having different repeating units (A₁O or A₂O).

In the above formula, the sum (a + b) of a and b is preferably from 1 to 1000, more preferably from 2 to 500, still more preferably from 2 to 300. a and b may be different from or the same as each other. When the total of a + b is 1000 or less, the viscosity does not become too high, and it becomes easy to uniformly add the compound to the water-absorbent resin.

The alkylene oxide adduct of the higher aliphatic amine can be readily available from the market, and for example, the following products are preferably exemplified.
Products available from NOF Corporation
   · Polyoxyethylene lauryl amine
      Nymeen (trade name, the same applies hereinafter) L-207 (HLB = 12.5)
   · Polyoxyethylene alkyl coconut oil alkyl amine
      Nymeen F-215 (HLB = 15.4)
   · Polyoxyethylene stearylamine
      Nymeen S-210 (HLB = 12.5), Nymeen S-215 (HLB = 14.5), Nymeen S-220 (HLB = 15.4)
   · Polyoxyethylene tallowalkylamine
      Nymeen T2-210 (HLB = 12.5), Nymeen T2-230 (HLB = 16.7)
   · Polyoxyethylene alkylpropylenediamine
      Nymeen DT-208 (HLB = 10.7)
Products available from Kao Corporation
   AMITE (trade name, the same applies hereinafter) 105A (HLB = 10.8), AMITE 320 (HLB = 15.4)

### (e) Alkyl Betaine

According to a preferred embodiment of the present invention, the "alkyl betaine" means a compound having a cationic group and an anionic group at non-adjacent positions in the same molecule, the cationic group being a secondary to quaternary ammonium cation, and at least one of substituents on the secondary to quaternary ammonium cation being a substituent having a C1-C30 hydrocarbon group. With such a configuration, the kinetic friction coefficient in particles having a particle diameter of 300 µm or more and less than 600 µm can be significantly reduced. In one embodiment, the alkyl betaine is preferably a compound represented by the following general formula (Chem. 6).

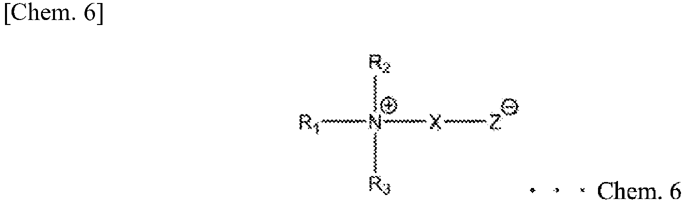

In the above formula (Chem. 6), R₁ is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, R₂ and R₃ are each independently hydrogen or a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C1 to C25, still more preferably from C1 to C20. When the number of carbon atoms in the hydrocarbon group is C30 or less, the hydrophobicity is not too strong, and the surface tension of the water-absorbing agent composition can be favorably maintained. R₁, R₂, and R₃ may be different from or the same as each other.

In the above formula, the structure of X is not particularly limited except that X contains C1 or more carbon atoms.

In the above formula, the anion unit (Z) may be a carboxylic acid salt (carboxylate anion), a sulfonic acid salt (sulfonate anion), or a phosphoric acid salt (phosphate anion).

In one embodiment, in addition to the compound represented by the above general formula (Chem. 6), the alkyl betaine may be a compound (having a cationic group on an imidazolium ring), the compound being represented by the following general formula (Chem. 7). Examples of such a compound include AMPHITOL (trade name, the same applies hereinafter) 20YB (available from Kao Corporation) as a commercially available product.

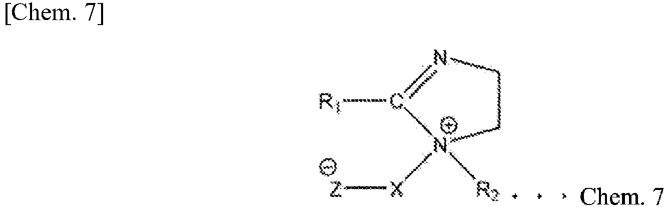

In the above formula (Chem. 7), R₁ is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, R₂ is hydrogen or a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C1 to C25, still more preferably from C1 to C20. When the number of carbon atoms in the hydrocarbon group is C30 or less, the hydrophobicity is not too strong, and the surface tension of the water-absorbing agent composition can be favorably maintained. R₁ and R₂ may be different from or the same as each other.

In the above formula, the structure of X is not particularly limited except that X contains C1 or more carbon atoms.

In the above formula, the anion unit (Z) may be a carboxylic acid salt (carboxylate anion), a sulfonic acid salt (sulfonate anion), or a phosphoric acid salt (phosphate anion).

### (f) Alkylamine Oxide

According to a preferred embodiment of the present invention, the "alkylamine oxide" means a compound having a cationic group and an anionic group at adjacent positions in the same molecule, the cationic group being a secondary to quaternary ammonium cation, and at least one of substituents on the secondary to quaternary ammonium cation being a substituent having a C1-C30 hydrocarbon group. With such a configuration, the kinetic friction coefficient in particles having a particle diameter of 300 µm or more and less than 600 µm can be significantly reduced. In one embodiment, the alkylamine oxide is preferably a compound represented by the following general formula (Chem. 8).

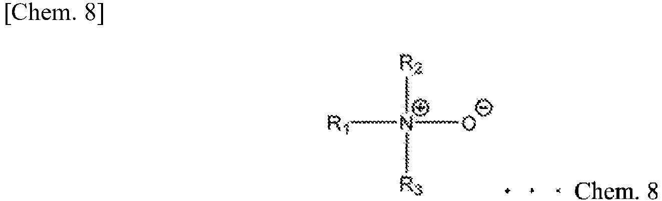

In the above formula (Chem. 8), R₁ is a hydrocarbon group having from C1 to C30 carbon atoms. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, R₂ and R₃ are each independently hydrogen or a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C1 to C25, still more preferably from C1 to C20. When the number of carbon atoms in the hydrocarbon group is C30 or less, the hydrophobicity is not too strong, and the surface tension of the water-absorbing agent composition can be favorably maintained. R₁, R₂, and R₃ may be different from or the same as each other.

In one embodiment, the alkylamine oxide as a flowability improver is preferably a compound represented by the above general formula (Chem. 8) in which R₁ is a C4-C24 linear saturated hydrocarbon group (alkyl group), and R₂ and R₃ are each independently a C1-C25 linear saturated hydrocarbon group (alkyl group). In one embodiment, the alkylamine oxide as a flowability improver is preferably a compound represented by the above general formula (Chem. 8) in which R₁ is a C6-C22 linear saturated hydrocarbon group (alkyl group), and R₂ and R₃ are each independently a C1-C20 linear saturated hydrocarbon group (alkyl group).

The alkyl betaine and the alkylamine oxide can be readily available from the market, and for example, the following products are preferably exemplified.
Products available from Kao Corporation:
   AMPHITOL 20BS, AMPHITOL 24B (desalted product of 20BS), AMPHITOL 86B, AMPHITOL 20N, AMPHITOL 20YB, AMPHITOL 20AB, AMPHITOL 55AB, AMPHITOL 20HD
Products available from DKS Co., Ltd.:
   Amogen (trade name, the same applies hereinafter) S-H, Amogen K, Amogen LB-C, Amogen CB-H, Amogen HB-C, Amogen AOL
Products available from ADEKA Corporation:
   ADEKA ANHORT (trade name, the same applies hereinafter) PB-30L, ADEKA ANHORT AB-35L
Products available from NOF Corporation:
   NISSAN ANON (trade name, the same applies hereinafter) BF, NISSAN ANON BL, NISSAN ANON BL-SF, NISSAN ANON BDF-R, NISSAN ANON BDF-SF, NISSAN ANON BDC-SF, NISSAN ANON BDL-SF, NISSAN ANON GLM-R, UNISAFE (trade name, the same applies hereinafter) A-LM, UNISAFE A-SM, UNISAFE A-LE
Products available from Nippon Nyukazai Co., Ltd.:
   TEXNOL (trade name, the same applies hereinafter) R2
Products available from TOHO Chemical Industry Co., Ltd.:
   Obazoline (trade name, the same applies hereinafter) LB-SF
Products available from New Japan Chemical Co., Ltd.:
   WANDAMINE (trade name, the same applies hereinafter) OX-300

### (g) Alkyl Sulfate Ester Salt

According to a preferred embodiment of the present invention, the "alkyl sulfate ester salt" means a compound having a sulfate group (-SO₄-) in the same molecule. With such a configuration, the kinetic friction coefficient in particles having a particle diameter of 300 µm or more and less than 600 µm can be significantly reduced. In one embodiment, the alkyl sulfate ester salt is preferably a compound represented by the following general formula (Chem. 9).

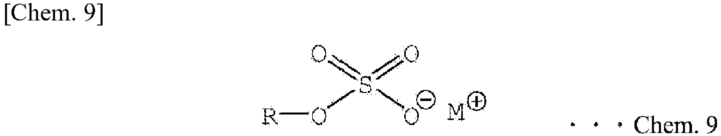

In the above formula (Chem. 9), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, M⁺ may be an alkali metal ion (Li⁺, Na⁺, K⁺, etc.) or an ammonium ion.

The alkyl sulfate ester salt can be readily available from the market, and for example, the following products are preferably exemplified.
Products available from Kao Corporation:
   EMAL (trade name, the same applies hereinafter) 2F, LATEMUL (trade name, the same applies hereinafter) AD-25
Products available from TAKEMOTO OIL & FAT Co., Ltd.:
   TAKESURF (trade name, the same applies hereinafter) A-24
Products available from NOF Corporation:
   SINTREX (trade name, the same applies hereinafter) EH-R, Persoft (trade name, the same applies hereinafter) SK
Products available from Lion Specialty Chemicals Co., Ltd.:
   SANNOL (trade name, the same applies hereinafter) LM-1130

### (h) Sulfate Ester Salt of Higher Alcohol Alkylene Oxide Adduct

According to a preferred embodiment of the present invention, "sulfate ester salt of higher alcohol alkylene oxide adduct" means a compound in which one terminal of a (poly)alkylene glycol is modified with a substituent having a C1-C30 hydrocarbon group, and the other terminal is a sulfate ester salt. With such a configuration, the kinetic friction coefficient in particles having a particle diameter of 300 µm or more and less than 600 µm can be significantly reduced. In one embodiment, the sulfate ester salt of the higher alcohol alkylene oxide adduct is preferably a compound represented by the following general formula (Chem. 10).

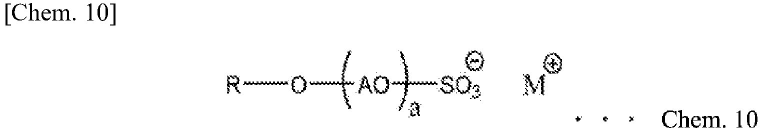

In the above formula (Chem. 10), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, (AO) is a repeating unit that can also be represented by CₙH₂ₙO (n is a natural number). The number of carbon atoms constituting AO (n described above) is preferably from C1 to C6, more preferably from C1 to C3, still more preferably from C2 to C3, particularly preferably C2. That is, the repeating unit (AO) in the above general formula (Chem. 10) is particularly preferably [CH₂CH₂O], and the repeating unit may have a structure derived from ethylene oxide addition or condensation of ethylene glycol.

In the above formula, a represents the number of the repeating units (AO), and when a is 2 or more, the number of carbon atoms constituting each repeating unit may be the same or different. That is, the alkylene oxide moiety contained in the above general formula (Chem. 10) may be a polymer having the same repeating unit (AO), or may be a block polymer or a random polymer having different repeating units (AO).

In the above formula, a is preferably from 1 to 1000, more preferably from 2 to 500, still more preferably from 2 to 300. When the repeating unit is 1000 or less, the viscosity does not become too high, and it becomes easy to uniformly add the polymer to the water-absorbent resin.

In the above formula, M⁺ may be an alkali metal ion (Li⁺, Na⁺, K⁺⁾ or an ammonium ion.

The sulfate ester salt of a higher alcohol alkylene oxide adduct can be readily available from the market, and for example, the following products are preferably exemplified.
Products available from Kao Corporation
   · Sodium polyoxyethylene lauryl ether sulfate
      EMAL 20C, EMAL E-27C, EMAL 270J, EMAL 20CM
Products available from Nippon Nyukazai Co., Ltd.
   · Polyoxyethylene alkyl ether sulfate ester salt
      NEWCOL 1020-SN, NEWCOL 2308-SF, NEWCOL 2320-SN, NEWCOL 2360-SN, NEWCOL 1305-SN, NEWCOL 1330-SF, NEWCOL 1703-SFD, NEWCOL 1525-SFC Products available from NOF Corporation
   · Sodium polyoxyethylene alkyl ether sulfate
      Persoft EP, NISSAN TRAX (trade name, the same applies hereinafter) K-40, NISSAN TRAX K-300, Persoft EF, Persoft EDO, Persoft EL, Persoft EK

### (i) Sulfonic Acid Salt

According to a preferred embodiment of the present invention, the "sulfonic acid salt" means a compound having a sulfonic acid group (-SO₃-) in the same molecule. With such a configuration, the kinetic friction coefficient in particles having a particle diameter of 300 µm or more and less than 600 µm can be significantly reduced. In one embodiment, the sulfonic acid salt is preferably a compound represented by the following general formula (Chem. 11).

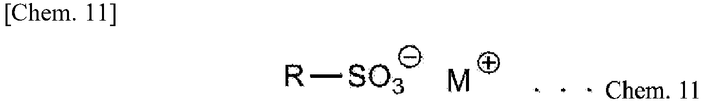

In the above formula (Chem. 11), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, M⁺ may be an alkali metal ion (Li⁺, Na⁺, K⁺⁾ or an ammonium ion.

The sulfonic acid salt is readily available from the market, and for example, the following products are preferably exemplified.
Products available from Kao Corporation
   · Alkylbenzenesulfonic acid
      NEOPELEX (trade name, the same applies hereinafter) GS
   · Sodium dodecylbenzenesulfonate
      NEOPELEX G-15, NEOPELEX G-25, NEOPELEX G-65
   · Sodium alkylnaphthalenesulfonate
      PELEX (trade name, the same applies hereinafter) NB-L
   · Sodium dialkyl sulfosuccinate
      PELEX OT-P, PELEX TR
   · Alkyl monoamide disodium sulfosuccinate
      PELEX TA
   · Sodium alkyl diphenyl ether disulfonate
      PELEX SS-L, PELEX SS-H
   · Sodium alkanesulfonate
   · LATEMUL PS
Products available from TAKEMOTO OIL & FAT Co., Ltd.
   ·Sodium alkyl diphenyl ether disulfonate
      Pyonine A-43-D, TAKESURF A-43-NQ

### (j) Dicarboxylic Acid Salt

According to a preferred embodiment of the present invention, the "dicarboxylic acid salt" means a compound having two carboxyl groups (-CH₃COO-) in the same molecule. The "dicarboxylic acid salt" as used herein means a compound other than the amine compound, and does not include "(k) alkylamine diacetic acid salt" described below. With such a configuration, the kinetic friction coefficient in particles having a particle diameter of 300 µm or more and less than 600 µm can be significantly reduced. Examples of the dicarboxylic acid salt include an alkenyl succinic acid salt and an acyl asparagine salt. In one embodiment, the alkenyl succinic acid salt is preferably a compound represented by the following general formula (Chem. 12). In one embodiment, the acyl asparagine salt is preferably a compound represented by the following general formula (Chem. 13).

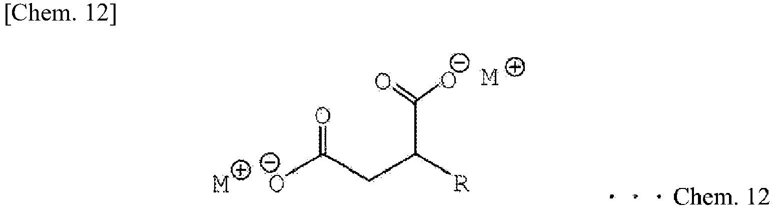

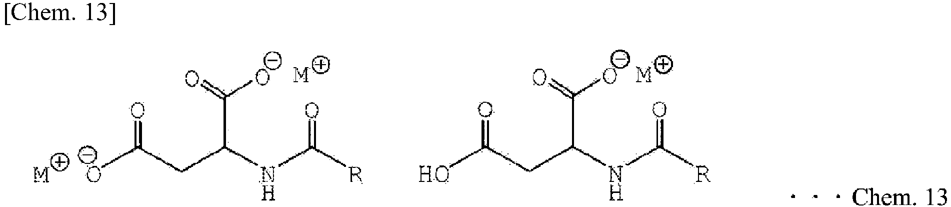

In the above formulas (Chem. 12) and (Chem. 13), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, M⁺ may be an alkali metal ion (Li⁺, Na⁺, K⁺⁾ or an ammonium ion. In addition, not only a form in which two carboxylic acid groups form a salt in one molecule (a form in which two carboxylic acid salt moieties are present), but also a form in which only one carboxylic acid group forms a salt (a form in which one carboxylic acid salt moiety is present) may be used.

The dicarboxylic acid salt is readily available from the market, and for example, the following products are preferably exemplified.
Products available from Kao Corporation:
   · Dipotassium alkenyl succinate
      LATEMUL ASK
Products available from Asahi Kasei Fine Chemicals Co., Ltd.:
   · Sodium Cocoyl Glutamate
      Aminosurfact (trade name, the same applies hereinafter) ACDS-L
   · Na Lauroyl Aspartate
      AminoFoamer (trade name, the same applies hereinafter) FLDS-L

### (k) Alkylamine Diacetic Acid Salt

According to a preferred embodiment of the invention, "alkylamine diacetic acid salt" means an amine compound having an alkyl group and two carboxyl groups (-CH₃COO-). With such a configuration, the kinetic friction coefficient in particles having a particle diameter of 300 µm or more and less than 600 µm can be significantly reduced. In one embodiment, the alkylamine diacetic acid salt is preferably a compound represented by the following general formula (Chem. 14).

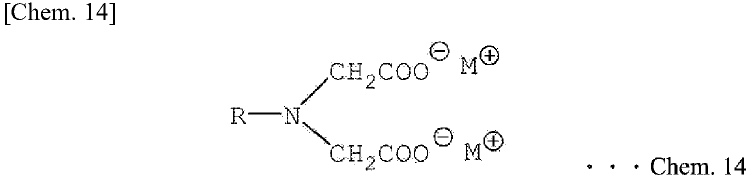

In the above formula (Chem. 14), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, M⁺ may be an alkali metal ion (Li⁺, Na⁺, K⁺⁾ or an ammonium ion.

Alkylamine diacetic acid salts are readily available from the market, and for example, the following products are preferably exemplified.
Products available from NOF Corporation:
· Sodium laurylaminodiacetate
   NISSAN ANON LA

### (l) Phosphoric Acid Ester Salt of Higher Alcohol Alkylene Oxide Adduct

According to a preferred embodiment of the present invention, the "phosphoric acid ester salt of a higher alcohol alkylene oxide adduct" means a compound in which one terminal of a (poly)alkylene glycol is modified with a substituent having a C1-C30 hydrocarbon group, and the other terminal is a phosphoric acid ester salt. With such a configuration, the kinetic friction coefficient in particles having a particle diameter of 300 µm or more and less than 600 µm can be significantly reduced. In one embodiment, the phosphoric acid ester salt of a higher alcohol alkylene oxide adduct is preferably a compound represented by the following general formula (Chem. 15).

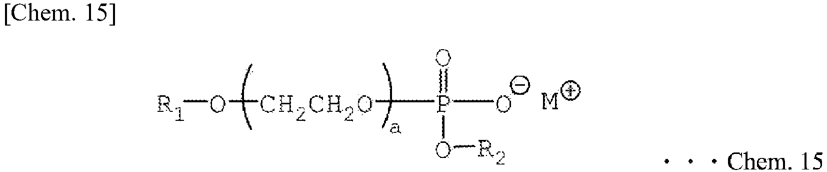

In the above formula (Chem. 15), R₁ and R₂ are each independently a C1-C30 hydrocarbon group. The C1-C30 hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, a represents the number of repeating units (CH₂CH₂O), and is preferably from 1 to 1000, more preferably from 2 to 500, still more preferably from 2 to 300. When the number of repeating units is 1000 or less, the viscosity does not become too high, and it becomes easy to uniformly add the compound to the water-absorbent resin.

In the above formula, M⁺ may be an alkali metal ion (Li⁺, Na⁺, K⁺⁾ or an ammonium ion.

The phosphoric acid ester salt of a higher alcohol alkylene oxide adduct can be readily available from the market, and for example, the following products are preferably exemplified.
Products available from DKS Co., Ltd.:
   Prisurf (trade name, the same applies hereinafter) A212C, Prisurf A207H, Prisurf A208S
Products available from TAKEMOTO OIL & FAT Co., Ltd.:
   TAKESURF A-72TK65, TAKESURF A-7004

### (m) Carboxylic Acid Salt of Higher Alcohol Alkylene Oxide Adduct

According to a preferred embodiment of the present invention, "carboxylic acid salt of a higher alcohol alkylene oxide adduct" means a compound in which one terminal of the (poly)alkylene glycol is modified with a substituent having a C1-C30 hydrocarbon group, and the other terminal is a carboxylic acid salt. With such a configuration, the kinetic friction coefficient in particles having a particle diameter of 300 µm or more and less than 600 µm can be significantly reduced. In one embodiment, the carboxylic acid salt of a higher alcohol alkylene oxide adduct is preferably a compound represented by the following general formula (Chem. 16).

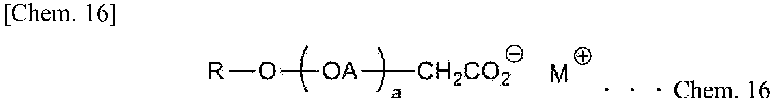

In the above formula (Chem. 16), R is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained. The hydrocarbon group is preferably a linear or branched saturated hydrocarbon group.

In the above formula, (AO) is a repeating unit that can also be represented by CₙH₂ₙO (n is a natural number). The number of carbon atoms constituting AO (n described above) is preferably from C1 to C6, more preferably from C1 to C3, still more preferably from C2 to C3, particularly preferably C2. That is, the repeating unit (AO) in the above general formula (Chem. 16) is particularly preferably [CH₂CH₂O], and the repeating unit may have a structure derived from ethylene oxide addition or condensation of ethylene glycol.

In the above formula, a represents the number of the repeating units (AO), and when a is 2 or more, the number of carbon atoms constituting each repeating unit may be the same or different. That is, the alkylene oxide moiety contained in the above general formula (Chem. 16) may be a polymer having the same repeating unit (AO), or may be a block polymer or a random polymer having different repeating units (AO).

In the above formula, a is preferably from 1 to 1000, more preferably from 2 to 500, still more preferably from 2 to 300. When the repeating unit is 1000 or less, the viscosity does not become too high, and it becomes easy to uniformly add the polymer to the water-absorbent resin.

In the above formula, M⁺ may be an alkali metal ion (Li⁺, Na⁺, K⁺⁾ or an ammonium ion.

In one embodiment, the carboxylic acid salt of a higher alcohol alkylene oxide adduct as the flowability improver is preferably a compound represented by the above general formula (Chem. 16) in which R is a C4-C24 linear saturated hydrocarbon group (alkyl group), the number of carbon atoms constituting AO is from 2 to 3 (from C2 to C3), a is from 2 to 500, and M⁺ is an alkali metal ion. In one embodiment, the carboxylic acid salt of a higher alcohol alkylene oxide adduct as the flowability improver is preferably a compound represented by the above general formula (Chem. 16) in which R is a C6-C22 linear saturated hydrocarbon group (alkyl group), AO is -CH₂CH₂O-, a is from 2 to 300, and M⁺ is an alkali metal ion.

The carboxylic acid salt of a higher alcohol alkylene oxide adduct is readily available from the market, and for example, the following products are preferably exemplified.
Products available from Kao Corporation
·Polyoxyethylene lauryl ether sodium acetate
   KAO AKYPO RLM-100NV, KAO AKYPO RLM-100, KAO AKYPO RLM-45NV, KAO AKYPO RLM-45

### (n) Ammonium Salt

According to a preferred embodiment of the invention, "ammonium salt" means a compound in which at least one hydrogen of the ammonium salt is modified with a substituent having a C1-C30 hydrocarbon group. With such a configuration, the kinetic friction coefficient in particles having a particle diameter of 300 µm or more and less than 600 µm can be significantly reduced. In one embodiment, the ammonium salt is preferably a compound represented by the following general formula (Chem. 17).

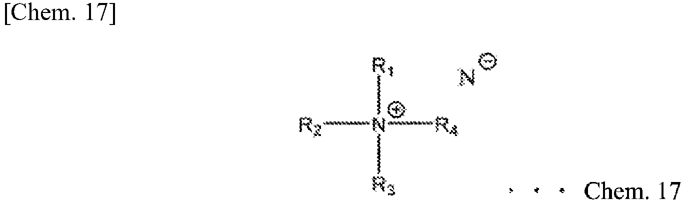

In the above formula (Chem. 17), R₁ is a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C2 to C28, still more preferably from C3 to C26, particularly preferably from C4 to C24, most preferably from C6 to C22. When the number of carbon atoms in the hydrocarbon group is C30 or less, the hydrophobicity is not too strong, and the surface tension of the water-absorbing agent composition can be favorably maintained.

In the above formula, R₂, R₃, and R₄ are each independently hydrogen or a C1-C30 hydrocarbon group. The hydrocarbon group may be a linear, branched, or cyclic saturated hydrocarbon group and/or unsaturated hydrocarbon group, an aromatic hydrocarbon group (such as an alkylphenyl group or an alkylbenzyl group), or a polycyclic aromatic hydrocarbon group (such as a naphthyl group). Further, the hydrocarbon group may have a reactive functional group such as a hydroxy group, an amino group, or a glycidyl group, and may have an ether bond, an ester bond, a urethane bond, or an amide bond. The number of carbon atoms in the hydrocarbon group is preferably from C1 to C30, more preferably from C1 to C25, still more preferably from C1 to C20. When the number of carbon atoms in the hydrocarbon group is C30 or less, the hydrophobicity is not too strong, and the surface tension of the water-absorbing agent composition can be favorably maintained. R₁, R₂, R₃, and R₄ may be different from or the same as each other.

In the above formula, N⁻ is a counter anion of an ammonium cation, and examples thereof include a halide ion, a carboxylate ion (for example, an acetate ion), a sulfonate ion, a hydroxy ion, BF⁴⁻, PF⁶⁻, ClO⁴⁻, AsF⁶⁻, and SbF⁶⁻.

The ammonium salt can be readily available from the market, and for example, the following products are preferably exemplified.
Products available from Kao Corporation
   · Coconut amine acetate
      Acetamine (trade name, the same applies hereinafter) 24
   · Stearylamine acetate
      Acetamine 86
   · Lauryltrimethylammonium chloride
      Quartamin (trade name, the same applies hereinafter) 24P
   · Stearyltrimethylammonium chloride
      Quartamin 86W
   · Cetyltrimethylammonium chloride
      Quartamin 60W
   · Distearyldimethylammonium chloride
      Quartamin D86P
   · Alkylbenzyldimethylammonium chloride
      SANISOL (trade name, the same applies hereinafter) C, SANISOL B-50
Products available from NOF Corporation
   · Tetradecylamine acetate
      NISSAN CATION (trade name, the same applies hereinafter) MA
   · Dodecyltrimethylammonium chloride
      NISSAN CATION BB
   · Yasialkyltrimethylammonium chloride
      NISSAN CATION FB
   · Hexadecyltrimethylammonium chloride
      NISSAN CATION PB-300
   · Tallowalkyltrimethylammonium chloride
      NISSAN CATION ABT2-500
   · Octadecyltrimethylammonium chloride
      NISSAN CATION AB, NISSAN CATION AB-600
   · Behenyltrimethylammonium chloride
      NISSAN CATION VB-M FLAKES, NISSAN CATION VB-F
   · Didecyldimethylammonium chloride
      NISSAN CATION 2-DB-500E
   · Dioleyldimethylammonium chloride
      NISSAN CATION 2-OLR
   · Yasialkyldimethylbenzylammonium chloride
      NISSAN CATION F2-50R
   · Tetradecyldimethylbenzylammonium chloride
      NISSAN CATION M2-100R

The HLB of the nonionic substance is preferably 11 or more, more preferably 12 or more. When the HLB of the nonionic substance is within the above-described range, the nonionic substance can be added as an aqueous solution, and can be uniformly added to the water-absorbent resin having a high specific surface area.

Here, the HLB is a value calculated by the Griffin method. The HLB of a nonionic substance whose HLB is unknown can be determined by the following method. A certain type of oil is emulsified with a nonionic substance whose HLB is to be determined (a surfactant having a known HLB is added as necessary), the same oil is separately emulsified with surfactants having a known HLB value (surfactants having different HLB of values are used), and the HLB of the surfactant that produces an emulsified state the same as that produced by the nonionic substance is defined as the HLB of the nonionic substance.

In the production method according to the present invention, the flowability improver to be added to the water-absorbent resin is preferably selected from a nonionic substance, a
zwitterionic substance, an anionic substance, and a cationic substance, and more preferably selected from a nonionic substance. That is, according to a preferred embodiment of the present invention, the water-soluble flowability improver contains at least one selected from nonionic substances. The water-soluble flowability improver more preferably contains at least one selected from nonionic substances having a polyalkylene glycol chain in the molecule. According to another preferred embodiment, the flowability improver contains at least one selected from (a) polyols and (b) modified products of hydroxy groups of polyols.

According to another preferred embodiment, the flowability improver contains at least one selected from a nonionic substance, a
zwitterionic substance, an anionic substance, and a cationic substance, the nonionic substance is a modified product of a hydroxy group of (a) polyols or (b) modified products of hydroxy groups of polyols, the
zwitterionic substance is (f) an alkylamine oxide, the anionic substance is (m) a carboxylic acid salt of a higher alcohol alkylene oxide adduct, and the cationic substance is (n) an ammonium salt.

The amount of the flowability improver added is more than 0 ppm and less than 200 ppm relative to the mass of the water-absorbent resin. By setting the amount of the flowability improver added within the above-described range, a decrease in surface tension of the water-absorbing agent composition can be suppressed, and further, a decrease in fixed height absorption value (FHA) of the water-absorbing agent composition at a height of 20 cm can be suppressed. By suppressing the decrease in FHA in this manner, the water absorption capacity when pressure is applied to the absorbent body is favorably maintained. A preferred range and the like of FHA will be described later.

In addition, some flowability improvers improve the flowability of the water-absorbent resin at the time of transportation with a feeder or the like, that is, improve the flowability of the water-absorbent resin in a dynamic environment, and at the same time, inhibit movement of the water-absorbent resin from a stationary state. Even when such a flowability improver is used, the amount of the flowability improver added within the above-described ranges makes it possible to suitably improve the flowability in a dynamic environment while suppressing the effect of inhibiting movement from a stationary state, that is, while suppressing a decrease in the flow rate of the water-absorbing agent composition.

The lower limit value of the amount of the flowability improver added is preferably 1 ppm or more, more preferably 2 ppm or more, still more preferably 3 ppm or more, relative to the mass of the water-absorbent resin. The upper limit value of the amount of the flowability improver added is preferably 180 ppm or less, more preferably 160 ppm or less, still more preferably 150 ppm or less, particularly preferably 80 ppm or less, relative to the mass of the water-absorbent resin. A preferred range of the amount of the flowability improver added can be a range defined by any combinations selected from the upper and lower limit values. Thus, in the production method according to the present invention, the amount of the flowability improver added may be, for example, 1 ppm or more and 180 ppm or less, 2 ppm or more and 160 ppm or less, 3 ppm or more and 160 ppm or less, 3 ppm or more and 150 ppm or less, or 3 ppm or more and 80 ppm or less.

In the production method according to the present invention, an additive can be added in a step from the step of preparing an aqueous monomer solution to the step before the surface crosslinking step included in the step of producing the water-absorbent resin. The additive here may be a component used as a flowability improver (the component is the same as the flowability improver, and is also referred to as "water-soluble component" in the present description). In such a case, the water-soluble component added before the surface crosslinking step is not included in the amount of the flowability improver added. That is, the amount of the flowability improver added is intended to be the total amount of the flowability improver added in the surface crosslinking step or in a step after the surface crosslinking step. This is because the flowability improver added in the surface crosslinking step and in a step after the surface crosslinking step and the water-soluble component added before the surface crosslinking step have different contributions to the flowability of the water-absorbing agent composition (behavior on the surface of the water-absorbent resin).

As the flowability improver, a flowability improver having the mass-average molecular weight described above may be added in the amount described above, and a plurality of flowability improvers may be used within the ranges described above. When one or more flowability improvers are used, flowability improvers having a plurality of mass-average molecular weights may be used in combination. The amount added in the case of using a plurality of flowability improvers is intended to be the total amount thereof.

### [2-9-3] Addition Step and Mixing Step

In the method for producing a water-absorbing agent composition according to the present invention, the flowability improver having the mass-average molecular weight may be added to the water-absorbent resin in the amount added described above in the surface crosslinking step or in a step after the surface crosslinking step. The addition of the flowability improver may be performed only in the surface crosslinking step, may be performed only in a step after the surface crosslinking step, or may be performed both in the surface crosslinking step and in a step after the surface crosslinking step. In one embodiment, the step of adding the flowability improver is preferably performed at least in a step subsequent to the surface crosslinking step (the addition of the flowability improver is performed after the surface crosslinking step), and more preferably performed in the cooling step (the addition of the flowability improver is performed in the cooling step after the surface crosslinking step). This is because when the flowability improver is added in a step after the surface crosslinking step, there is no possibility of modification of the flowability improver due to heating of the flowability improver at a high temperature.

The flowability improver is added in the form of an aqueous solution when added to and mixed with the water-absorbent resin. Thus, in one embodiment, it is preferred to prepare an aqueous solution of the flowability improver in advance and then add the aqueous solution to the water-absorbent resin. In this case, the concentration of the aqueous solution is 0.01 mass% or more and 20 mass% or less, preferably 0.02 mass% or more and 15 mass% or less, more preferably 0.05 mass% or more and 10 mass% or less, particularly preferably 0.1 mass% or more and 3 mass% or less. By setting the aqueous solution concentration within the above-described ranges, the handleability of the aqueous solution containing the flowability improver is improved, and the flowability improver can be uniformly mixed with the water-absorbent resin.

The lower limit value of the pH of the aqueous solution of the flowability improver is preferably 4.5 or more, more preferably 4.6 or more, still more preferably 5.0 or more, particularly preferably 5.5 or more. When the flowability improver contained in the aqueous solution of the flowability improver is an ester compound containing a polyalkylene glycol chain, hydrolysis may proceed due to long-term storage, exposure to high temperature, or the like. As the hydrolysis proceeds, a carboxylic acid is produced as a by-product, and thus, the pH of the aqueous solution of the flowability improver decreases. In addition, the flowability improver in which hydrolysis has progressed has increased hydrophobicity, and thus, it causes a problem that a residue of dissolution occurs or a spray nozzle is clogged by precipitation. The lower limit value of the pH of the aqueous solution of the flowability improver is preferably controlled within the above-described ranges because the occurrence of the above problem can be prevented. The upper limit value of the pH of the aqueous solution of the flowability improver is preferably 11.0 or less, more preferably 10.5 or less, still more preferably 10.0 or less, particularly preferably 9.5 or less, from the viewpoint of suppressing deterioration of an apparatus to be in contact with the aqueous solution and safety of an operator. A preferred range of the pH can be a range defined by any combinations selected from the upper and lower limit values. Thus, the pH of the aqueous solution of the flowability improver may be, for example, 4.5 or more and 11.0 or less, 4.6 or more and 10.5 or less, 5.0 or more and 10.0 or less, or 5.5 or more and 9.5 or less.

The lower limit value of the amount of the aqueous solution of the flowability improver added is preferably 0.001 parts by mass or more, more preferably 0.002 parts by mass or more, still more preferably 0.003 parts by mass or more, relative to 100 parts by mass of the water-absorbent resin. On the other hand, the upper limit value thereof is preferably 10 parts by mass or less, more preferably 9 parts by mass or less, still more preferably 8 parts by mass or less. A preferred range of the amount of the flowability improver added can be a range defined by any combinations selected from the upper and lower limit values. When the amount of the flowability improver added is 10 parts by mass or less relative to 100 parts by mass of the water-absorbent resin, a large amount of drying energy is not required for adjusting the water content of the water-absorbing agent composition after addition, which is advantageous in terms of production cost. In addition, it is possible to suppress firm aggregation of the water-absorbent resin because of a large amount of the flowability improver added, and thus, it is not necessary to add a step of pulverizing the aggregate. Thus, it is possible to suppress destruction of the surface crosslinking layer (destruction of the surface crosslinking layer already formed) by adding the pulverizing step, and as a result, it is possible to suppress a decrease in absorption capacity under pressure.

The aqueous solution of the flowability improver is added to and mixed with the water-absorbent resin in a droplet state. At this time, when the average droplet diameter of the droplets exceeds 1 mm, the number of droplets per added amount of the aqueous solution of the flowability improver decreases, and thus, the encounter probability with the water-absorbent resin decreases, the particles are not uniformly mixed, and the flowability of the particles cannot be uniformly improved. As a result, the effect of reducing the kinetic friction coefficient in the particles having a particle diameter of 300 µm or more and less than 600 µm is reduced. In addition, aggregated coarse particles are likely to be generated when the aqueous solution of the flowability improver in which the average droplet diameter of the droplets exceeds 1 mm and a water-absorbent resin having a large specific surface area, in particular, are stirred and mixed. Thus, the upper limit value of the average droplet diameter (diameter) is 1 mm or less, preferably 0.9 mm (900 µm) or less, more preferably 0.8 mm (800 µm) or less, still more preferably 0.5 mm (500 µm) or less. On the other hand, as the droplet diameter of the aqueous solution of the flowability improver to be added decreases, the aqueous solution of the flowability improver and the water-absorbent resin are more likely to be uniformly mixed, but the cost for making the droplets finer becomes too high relative to the obtained effect. Thus, the lower limit value of the average droplet diameter (diameter) is 10 µm or more, preferably 20 µm or more, more preferably 30 µm or more, still more preferably 40 µm or more. A preferred range of the average droplet diameter can be a range defined by any combinations selected from the upper and lower limit values. Thus, the average droplet diameter may be, for example, 10 µm or more and 0.9 mm or less, 20 µm or more and 0.8 mm or less, 30 µm or more and 0.5 mm or less, or 40 µm or more and 0.5 mm or less. Examples of the means for adding the aqueous solution of the flowability improver in a droplet state include spraying means such as a spray nozzle having a desired nozzle diameter, and using a straight pipe having a desired inner diameter. The average droplet diameter can be measured by photographing the state at the time of addition using a high-speed camera or the like, and is a value determined by the inner diameter of the droplet addition port in the spray unit or the spray pressure.

When the aqueous solution of the flowability improver is added to the water-absorbent resin, the lower limit value of the temperature of the aqueous solution of the flowability improver, specifically, the temperature of the droplets is preferably 20°C or higher, and the upper limit value thereof is preferably 80°C or lower, more preferably 70°C or lower, still more preferably 60°C or lower. A preferred range of the temperature of the aqueous solution of the flowability improver (temperature of the droplets) can be a range defined by any combinations selected from the upper and lower limit values. Thus, the temperature of the aqueous solution of the flowability improver is preferably 20°C or higher and 80°C or lower, more preferably 20°C or higher and 70°C or lower, still more preferably 20°C or higher and 60°C or lower. The aqueous solution of the flowability improver adjusted to the above-described temperature is particularly effective in improving the mixing property with the water-absorbent resin having a high specific surface area. When the temperature of the aqueous solution of the flowability improver is too high, moisture of the aqueous solution of the flowability improver evaporates and the flowability improver is likely to precipitate, and thus, uniform mixing becomes difficult. On the other hand, when the temperature is too low, the solubility of the flowability improver in water decreases, and the concentration of the aqueous solution of the flowability improver that can be produced decreases. Thus, when a desired flowability improver is added, the amount of water added increases, and as a result, there is a possibility that many aggregated coarse particles (product lumps) in the product are generated, which is not preferred. The temperature range of the aqueous solution is a temperature measured before being affected by the temperature of the water-absorbent resin or the apparatus. The apparatus as used herein refers to an apparatus in which the water-absorbent resin is retained.

The temperature of the aqueous solution of the flowability improver and the average droplet diameter of the aqueous solution may be controlled alone, but as a result of examining the relationship between these, the inventors of the present invention have found that when these are controlled in combination within the above-described preferred ranges, it is particularly effective for improving the mixing property with a water-absorbent resin having a high specific surface area. That is, it has been found that by setting both the temperature and the average droplet diameter of the aqueous solution within the above-described preferred ranges, a remarkable effect of suppressing segregation when the water-absorbing agent composition obtained by mixing the flowability improver with the water-absorbent resin is transported with a feeder is obtained. Specifically, the water absorption speed increases as the temperature of the aqueous solution of the flowability improver added to the water-absorbent resin having a large specific surface area increases, and thus, it is effective to reduce the average droplet diameter as a means for mixing the water-absorbent resin more uniformly. In addition, when the temperature of the aqueous solution of the flowability improver is too high, or the droplet diameter is too small, the aqueous solution of the flowability improver may be volatilized and easily precipitated. Thus, it is preferred to control each of them within the above-described ranges.

The apparatus (mixing apparatus) used for mixing the aqueous solution of the flowability improver preferably has a large mixing force. Specific examples of the mixing apparatus include a cylindrical mixer, a double-wall conical mixer, a V-shaped mixer, a ribbon-shaped mixer, a screw type mixer, a rotary disk mixer, a double-arm type kneader, an internal mixer, a pulverizing kneader, a rotary mixer, a screw type extruder, a fluidized bed type mixer, and a flow type mixer. In addition, an apparatus capable of performing mixing by stirring is more preferred, and a high-speed stirring type mixing apparatus and a vertical rotary disk type mixing apparatus are exemplified. A high-speed stirring type continuous mixing apparatus is more preferred, and a horizontal high-speed stirring type continuous mixing apparatus or a vertical high-speed stirring type continuous mixing apparatus is still more preferred. Specific examples of the mixer include a Schwei mixer (available from Hosokawa Micron Corporation), a turbulizer (available from Hosokawa Micron Corporation), a Loedige mixer (available from Loedige Corporation), and a flow jet mixer (available from FUNKEN POWTECHS, INC.).

In addition, the water-absorbent resin having a high specific surface area has a faster water absorption speed than known products, and thus, it is difficult to uniformly mix a trace amount of flowability improver with an amount added of less than 200 ppm between particles. Thus, the inventors of the present invention have conducted a plurality of experiments by changing the peripheral speed, the mixing time, and the average droplet diameter in any manner, and as a result, have found that even when a trace amount of flowability improver, the added amount of which is less than 200 ppm, is added, mixing can be uniformly performed between particles of the water-absorbent resin having a high specific surface area by controlling the mixing power index defined by the following (Equation 1), instead of controlling each of the peripheral speed, the mixing time, and the average droplet diameter alone. That is, when the aqueous solution of the flowability improver is added to and mixed with the water-absorbent resin, the flowability of the water-absorbing agent composition can be uniformly improved, and the occurrence of particle size segregation after transportation can be suppressed by setting the mixing power index appropriately determined in the following (Equation 1) to a specific value or more.

### Mixing Power Index

In the production method according to the present invention, the mixing power index when the aqueous solution of the flowability improver is added to and mixed with the water-absorbent resin is determined from the following (Equation 1). The mixing power index is a value calculated every time the flowability improver is added and mixed. For example, when the addition and mixing of the flowability improver is performed both in the surface crosslinking step and in a step subsequent to the surface crosslinking step, it is intended to mean that the mixing power index is the mixing power index in each step. In the production method according to the present invention, either the mixing power index in the surface crosslinking step or the mixing power index in a step subsequent to the surface crosslinking step is preferably 70000 or more, and at least the mixing power index in the step subsequent to the surface crosslinking step is preferably 70000 or more.
[Math.5]$\begin{matrix}Mixing power index \left(unit: none\right) \\ = peripheral speed \left(m/s\right) \times mixing time \left(s\right) / \left\{average droplet diameter\left(mm\right)/1000\right\}\end{matrix}$

In (Equation 1), the peripheral speed (unit: m/s) refers to the peripheral speed of the stirring blade, and specifically, the peripheral speed (m/s) = π × stirring blade diameter (m) × rotational speed (rpm)/60 is satisfied.

The lower limit value of the mixing power index is 70000 or more, preferably 80000 or more, more preferably 90000 or more, still more preferably 100000 or more, particularly preferably 500000 or more, most preferably 1000000 or more, from the viewpoint of uniformly mixing the particles of the water-absorbent resin in which the flowability improver has a high specific surface area. On the other hand, the water-absorbent resin having a high specific surface area has larger number of uneven shapes on the surface than known products. Thus, the water-absorbent resin has low damage resistance, and thus, fine particles are likely to be generated due to chipping of the surface when the water-absorbent resin is mixed in the apparatus. Thus, the upper limit value of the mixing power index is preferably 6000000 or less, more preferably 5000000 or less, still more preferably 4000000 or less, particularly preferably 3000000 or less, from the viewpoint of suppressing generation of fine particles. A preferred range of the mixing power index can be a range defined by any combinations selected from the upper and lower limit values. Thus, the mixing power index may be, for example, 70000 or more and 6000000 or less, 80000 or more and 5000000 or less, 90000 or more and 4000000 or less, 100000 or more and 3000000 or less, 500000 or more and 3000000 or less, or 1000000 or more and 3000000 or less.

The lower limit value of the stirring blade diameter of the mixing apparatus is preferably 0.01 m or more, more preferably 0.02 m or more, still more preferably 0.03 m or more, from the viewpoint of efficient stirring and mixing. The upper limit value of the stirring blade diameter of the mixing apparatus is preferably 10 m or less, more preferably 5 m or less, still more preferably 3 m or less, from the viewpoint of the size and cost of the mixing apparatus. A preferred range of the stirring blade diameter of the mixing apparatus can be a range defined by any combinations selected from the upper and lower limit values. Thus, the stirring blade diameter of the mixing apparatus is preferably 0.01 m or more and 10 m or less, more preferably 0.02 m or more and 5 m or less, still more preferably 0.03 m or more and 3 m or less.

The lower limit value of the rotational speed of the stirring type mixer is preferably 5 rpm or more, more preferably 10 rpm or more, still more preferably 15 rpm or more, particularly preferably 100 rpm or more, most preferably 200 rpm or more. The upper limit value of the rotational speed is preferably 10000 rpm or less, more preferably 2000 rpm or less, still more preferably 1000 rpm or less, particularly preferably 500 rpm or less. A preferred range of the rotational speed can be a range defined by any combinations selected from the upper and lower limit values. Thus, the rotational speed may be, for example, 5 rpm or more and 10000 rpm or less, 10 rpm or more and 2000 rpm or less, 15 rpm or more and 1000 rpm or less, 100 rpm or more and 500 rpm or less, or 200 rpm or more and 500 rpm or less.

The peripheral speed (peripheral speed of the stirring blade of the mixing apparatus) may be a constant value or may change in the middle. It is intended that the case where "the peripheral speed changes" means that the peripheral speed changes in one mixing apparatus or that two or more mixing apparatuses having different peripheral speeds are used. The lower limit value of the peripheral speed of the stirring blade of the mixing apparatus is preferably 0.002 m/s or more, more preferably 0.01 m/s or more, still more preferably 0.02 m/s or more, particularly preferably 0.5 m/s or more, most preferably 1.0 m/s or more, from the preferred ranges of the stirring blade diameter and the rotational speed. On the other hand, the upper limit value thereof is preferably 6000 m/s or less, more preferably 600 m/s or less, still more preferably 200 m/s or less, particularly preferably 100 m/s or less, most preferably 50 m/s or less. A preferred range of the peripheral speed of the stirring blade of the mixing apparatus can be a range defined by any combinations selected from the upper and lower limit values. Thus, the peripheral speed of the stirring blade may be, for example, 0.002 m/s or more and 6000 m/s or less, 0.01 m/s or more and 600 m/s or less, 0.02 m/s or more and 200 m/s or less, 0.5 m/s or more and 100 m/s or less, or 1.0 m/s or more and 50 m/s or less.

The flowability improver having a mass-average molecular weight within the above-described ranges is less likely to be incorporated into particles of the water-absorbent resin with respect to a low-molecular-weight water-soluble substance. That is, when the flowability improver is added to the water-absorbent resin and then sufficiently mixed, the flowability improver attached to the particle surface of one water-absorbent resin can be brought into contact with the particle surface of another water-absorbent resin. As a result, the mixing uniformity of the flowability improver into the entire water-absorbent resin improves. From this, the "mixing time" in the present description means the total time during which the water-absorbent resin is stirred and mixed within 1 hour from the time point at which the flowability improver comes into contact with the water-absorbent resin. The lower limit value of the mixing time is preferably 10 seconds or more, more preferably 15 seconds or more, still more preferably 20 seconds or more. The upper limit value of the mixing time is preferably 60 minutes or less, more preferably 30 minutes or less, still more preferably 20 minutes or less. A preferred range of the mixing time can be a range defined by any combinations selected from the upper and lower limit values. Thus, the mixing time may be, for example, 10 seconds or more and 60 minutes or less, 15 seconds or more and 30 minutes or less, or 20 seconds or more and 20 minutes or less.

For example, when an aqueous solution of the flowability improver is added at an average droplet diameter E1 (mm) and stirred and mixed at a peripheral speed C1 (m/s) and a mixing time D1 (s), where D1 is within 1 hour after the flowability improver and the water-absorbent resin come into contact with each other, the mixing power index is calculated from the equation: "mixing power index (unit: none) = C1 (m/s) × D1 (s)/{E1 (mm)/1000}".

For example, when an aqueous solution of the flowability improver is added at an average droplet diameter E2 (mm) and stirred and mixed at a peripheral speed C2 (m/s) and a mixing time D2 (s), where D2 is from 1 hour to 10 minutes after the flowability improver and the water-absorbent resin come into contact with each other, the mixing power index is calculated from "mixing power index (unit: none) = C2 (m/s) × 3600 (s)/{E2 (mm)/1000}", considering 1 hour (3600 seconds) as the mixing time.

For example, when an aqueous solution of the flowability improver is added at an average droplet diameter E3 (mm), stirred and mixed at a peripheral speed C3 (m/s) and a mixing time D3 (s), then stirred and mixed at a peripheral speed C4 (m/s) and a mixing time D4 (s), where D3 and D4 are within 1 hour after the flowability improver and the water-absorbent resin come into contact with each other, the mixing power index is calculated from "mixing power index (unit: none) = {C3 (m/s) × D3 (s) + C4 (m/s) × D4 (s)}/{E3 (mm)/1000}".

For example, when an aqueous solution of the flowability improver is added at an average droplet diameter E4 (mm), stirred and mixed at a peripheral speed C5 (m/s) and a mixing time D5 (s), then stirred and mixed at a peripheral speed C6 (m/s) and a mixing time D6 (s), and further stirred and mixed at a peripheral speed C7 (m/s) and a mixing time D7 (s), where D5 and D6 are within 1 hour after the flowability improver and the water-absorbent resin come into contact with each other, and D7 is one hour or more after the flowability improver and the water-absorbent resin come into contact with each other, the mixing power index is calculated from "mixing power index (unit: none) = {C5 (m/s) × D5 (s) + C6 (m/s) × D6 (s)}/{(E4 (mm)/1000}".

The stirring and mixing may be continuous or divided into a plurality of times as long as it is within 1 hour after the flowability improver and the water-absorbent resin come into contact with each other, but it is preferred that the stirring is performed at least immediately after the flowability improver and the water-absorbent resin come into contact with each other.

When the aqueous solution of the flowability improver is added, the aqueous solution of the flowability improver may further contain at least one selected from the group consisting of other additives such as a chelating agent, a plant component, an antibacterial agent, and an inorganic salt described later. In such a case, the content of the additive is appropriately selected as necessary. The content is desirably 0.001 mass% or more and 50 mass% or less of the aqueous solution of the flowability improver. As the chelating agent, a chelating agent having high ion capping ability and chelating ability toward Fe and Cu is preferred. Specific examples thereof include a chelating agent having a stability constant with respect to Fe ions of 10 or more, preferably a chelating agent having a stability constant with respect to Fe ions of 20 or more, more preferably an amino polycarboxylic acid and a salt thereof, particularly preferably an amino carboxylic acid having 3 or more carboxyl groups and a salt thereof. Specific examples of these polyvalent carboxylic acids include diethylenetriaminepentaacetic acid, triethylenetetraaminhexaacetic acid, cyclohexane-1,2-diaminetetraacetic acid, N-hydroxyethylethylenediaminetriacetic acid, ethylene glycol diethyletherdiaminetetraacetic acid, ethylenediaminetetrapropionic acid, N-alkyl-N'-carboxymethylaspartic acid, N-alkenyl-N'-carboxymethylaspartic acid, and alkali metal salts, alkaline earth metal salts, ammonium salts, and amine salts thereof. The salt may be a fully neutralized product, a partially neutralized product, or a mixture. Among these, diethylenetriaminepentaacetic acid, triethylenetetraaminhexaacetic acid, N-hydroxyethylethylenediaminetriacetic acid, and salts thereof are most preferred. The lower limit value of the amount of the chelating agent used is preferably 0.00001 parts by mass or more, more preferably 0.0001 parts by mass or more, relative to 100 parts by mass of the water-absorbent resin. On the other hand, the upper limit value thereof is preferably 10 parts by mass or less, more preferably 1 part by mass or less. A preferred range of the amount of the chelating agent used can be a range defined by any combination selected from the upper and lower limit values described above.

The lower limit value of the amount of the plant component used is preferably 0 part by mass or more, more preferably 0.001 parts by mass or more, still more preferably 0.002 parts by mass or more, relative to 100 parts by mass of the water-absorbent resin to exhibit deodorizing properties. On the other hand, the upper limit value of the amount may be preferably 10 parts by mass or less, more preferably 5 parts by mass or less, still more preferably 3 parts by mass or less. A preferred range of the amount of the plant component used can be a range defined by any combination selected from the upper and lower limit values described above. The plant component is preferably at least one compound selected from polyphenols, flavones and the like, and caffeine, more preferably at least one compound selected from tannin, tannic acid, quintuple, gallnut, and gallic acid. Examples of the above-described antibacterial agent include known antibacterial agents having antibacterial properties, for example, an antibacterial agent described in JP 11-267500 A.

### [2-9-4] Curing Step

The mixture of the water-absorbent resin and the aqueous solution of the flowability improver obtained through the series of operations is preferably subjected to a curing treatment. That is, the production method according to the present invention preferably further includes a step of mixing the flowability improver with the water-absorbent resin, and then curing the mixture of the water-absorbent resin and the flowability improver. The term "curing" refers to an operation of removing the wettability of the surface of the water-absorbent resin and powderizing the water-absorbent resin. The "curing treatment" is a treatment for curing an object by controlling the temperature of the object to a predetermined curing temperature and holding the temperature state for a predetermined curing time.

In one embodiment of the present invention, it is preferred that a heating medium such as hot air is used for the curing treatment. When the curing treatment is performed in a step after the surface crosslinking step, the lower limit value of the heating temperature, for example, the heating medium temperature or the material temperature is preferably 40°C or higher, more preferably 50°C or higher. The upper limit value is preferably 150°C or lower, more preferably 140°C or lower, particularly preferably 100°C or lower. The curing time within this temperature range is preferably 1 minute or more and 2 hours or less, more preferably 5 minutes or more and 1.5 hours or less, particularly preferably 10 minutes or more and 1 hour or less. By setting the curing temperature and the curing time within the above-described ranges, it is possible to prevent the resulting water-absorbing agent composition from having a wet surface state and having strong tackiness, and handling as powder is facilitated. When the curing temperature is too high or the curing time is too long, it becomes uneconomical in terms of energy. Thus, the curing temperature and the curing time are preferably within the above-described ranges. Each preferred range of the heating temperature and the curing time (heating time) can be set to a range defined by any combinations selected from the upper and lower limit values.

The addition and mixing of the aqueous solution of the flowability improver and the subsequent curing treatment may be performed by the same apparatus or different apparatuses. The series of treatments may be performed in the heat treatment step in the surface crosslinking step, in the cooling step, or after the cooling step. The above-described apparatus (mixing apparatus) is exemplified as the apparatus to be used, and for example, a heating medium such as a gas or conductive heating may be adjusted so that the temperature in the apparatus becomes the above temperature. In the case of curing, stirring may be performed as long as the temperature and the water content can be controlled within a predetermined range, or standing, that is, no stirring may be performed. When the curing treatment is performed in a standing state, the lower limit value of the thickness of the water-absorbent resin to be stacked is preferably 1 cm or more, more preferably 5 cm or more, still more preferably 10 cm or more. On the other hand, the water-absorbent resin may be stacked in the form of a layer having the upper limit value of the thickness of preferably 100 cm or less, more preferably 80 cm or less, still more preferably 70 cm or less, and then curing treatment may be performed. A preferred range of the thickness can be a range defined by any combinations selected from the upper and lower limit values. The water-absorbent resin after curing can be pulverized or classified as necessary to obtain a water-absorbing agent composition having a desired particle size.

### [2-10] Other Steps

In the present invention, in addition to the above-described steps, a granulation step, a particle size adjustment step, a fine powder removal step, a fine powder recovery step, a step of reusing fine powder, an iron removal step, and the like, can be performed as necessary. At least one step selected from among a transportation step, a storage step, a packaging step, a keeping step, and the like may be further included.

### [2-11] Kinetic Friction Coefficient Reduction Percentage

According to the production method of the present invention, the kinetic friction coefficient of the water-absorbent resin (that is, water-absorbing agent composition) after addition of the flowability improver can be significantly reduced as compared with the kinetic friction coefficient of the water-absorbent resin before addition of the flowability improver. Specifically, there can be provided a method for producing a water-absorbing agent composition in which the kinetic friction coefficient reduction percentage calculated by (Equation 2) described below is 10% or more.
[Math.6]$Kinetic friction coefficient reduction percentage \left(%\right) = \left(A-B\right) / A \times 100$

In Equation 2,
A: kinetic friction coefficient of particles having a particle diameter of 300 µm or more and less than 600 µm in the water-absorbent resin before addition of the flowability improver;
B: kinetic friction coefficient of particles having a particle diameter of 300 µm or more and less than 600 µm in the water-absorbing agent composition after addition of the flowability improver The kinetic friction coefficients of A and B are measured by the method described in Examples.

In the present description, the term "particles having a particle diameter of 300 µm or more and less than 600 µm" refers to particles that pass through a sieve with a mesh size of 600 µm but remain on a sieve with a mesh size of 300 µm after being classified by the classification method in the method for evaluating the kinetic friction coefficient of Examples.

In the production method according to the present invention, the kinetic friction coefficient reduction percentage is preferably 10% or more.

The lower limit value of the kinetic friction coefficient reduction percentage is preferably 12% or more, more preferably 15% or more, particularly preferably 20% or more. On the other hand, a higher upper limit value is more preferred, and the upper limit value is not particularly limited, but is, for example, 90% or less, preferably 70% or less, more preferably 50% or less, still more preferably 35% or less. A preferred range of the kinetic friction coefficient reduction percentage can be a range defined by any combinations selected from the upper and lower limit values. That is, the kinetic friction coefficient reduction percentage is, for example, 10% or more and 90% or less, preferably 12% or more and 90% or less, more preferably 15% or more and 90% or less, particularly preferably 20% or more and 90% or less.

In a preferred embodiment, the water-absorbent resin (before addition of the flowability improver) and the water-absorbing agent composition to be obtained each contain particles having a particle diameter of 300 µm or more and less than 600 µm in an amount of 50 mass% or more, and the kinetic friction coefficient reduction percentage calculated by the above (Equation 2) can be 10% or more. In this case, the preferred range of the kinetic friction coefficient reduction percentage is the same as described above.

### [3] Physical Property of Water-Absorbing Agent Composition

The water-absorbing agent composition obtained through the above steps is a final product as long as it is ready for shipment. Through the production method according to the present invention described above, a water-absorbing agent composition satisfying the following (1) to (5) is obtained. Thus, another aspect of the present invention provides a water-absorbing agent composition containing a water-absorbent resin as a main component, the water-absorbing agent composition containing a flowability improver and satisfying all of the following (1) to (5):
(1) the water-absorbing agent composition has a specific surface area of 25 m²/kg or more;
(2) the water-absorbing agent composition has a surface tension of 56 mN/m or more;
(3) the water-absorbing agent composition has a flow rate of 10.0 g/s or more;
(4) the mass proportion of particles having a particle diameter of 300 µm or more and less than 600 µm in the water-absorbing agent composition is 50 mass% or more;
(5) particles having a particle diameter of 300 µm or more and less than 600 µm in the water-absorbing agent composition has a kinetic friction coefficient of 0.80 or less.

### [3-1] Specific Surface Area

By setting the lower limit value of the specific surface area of the water-absorbing agent composition according to the present invention to 25 m²/kg or more, a further improved water absorption speed measured by the Vortex method can be obtained. A higher specific surface area of the water-absorbing agent composition is more preferred. The specific surface area is preferably 26 m²/kg or more, more preferably 27 m²/kg or more, still more preferably 28 m²/kg or more, still more preferably 29 m²/kg or more, still more preferably 30 m²/kg or more, particularly preferably 35 m²/kg or more, most preferably 36 m²/kg or more. On the other hand, the upper limit value of the specific surface area of the water-absorbing agent composition is preferably 60 m²/kg or less, more preferably 55 m²/kg or less. A preferred range of the specific surface area of the water-absorbing agent composition can be a range defined by any combinations selected from the upper and lower limit values. Thus, the specific surface area of the water-absorbing agent composition may be, for example, 25 m²/kg or more and 60 m²/kg or less, 26 m²/kg or more and 60 m²/kg or less, 27 m²/kg or more and 60 m²/kg or less, 28 m²/kg or more and 60 m²/kg or less, 29 m²/kg or more and 60 m²/kg or less, 30 m²/kg or more and 55 m²/kg or less, 35 m²/kg or more and 55 m²/kg or less, or 36 m²/kg or more and 55 m²/kg or less. From the viewpoint of increasing the water absorption speed, it is desirable that the specific surface area of the water-absorbing agent composition is as high as possible. However, when the specific surface area is too large, excessive foaming polymerization in the polymerization step or gel-grinding that is too fine in the gel-grinding step is required, and as a result, AAP (absorption capacity under pressure) may decrease. On the other hand, when the specific surface area of the water-absorbing agent composition is less than 25 m²/kg, it is difficult to obtain a water-absorbing agent composition having a desired water absorption speed (Vortex), which is not preferred.

### [3-2] Surface Tension

The water-absorbing agent composition according to the present invention has a lower limit value of surface tension of 56 mN/m or more, preferably 58 mN/m or more, more preferably 60 mN/m or more, still more preferably 65 mN/m or more. The upper limit value is not particularly limited, and is preferably 75 mN/m or less from the viewpoint of balance with other physical properties. When the surface tension is less than 56 mN/m, the return amount of the liquid when pressure is applied to the absorbent body increases, and thus it is not suitable as a water-absorbing agent composition to be used for an absorbent article such as a disposable diaper. A preferred range of the surface tension of the water-absorbing agent composition can be a range defined by any combinations selected from the upper and lower limit values. Thus, the surface tension of the water-absorbing agent composition is, for example, 56 mN/m or more and 75 mN/m or less, preferably 58 mN/m or more and 75 mN/m or less, more preferably 60 mN/m or more and 75 mN/m or less, still more preferably 65 mN/m or more and 75 mN/m or less. The surface tension can be controlled by an additive or the like added after surface crosslinking. Detailed measurement conditions of the surface tension are described in Examples.

### [3-3] Flow Rate

The water-absorbing agent composition according to the present invention has a lower limit value of a flow rate of 10.0 g/s or more. The lower limit value of the flow rate is preferably 10.2 g/s or more, more preferably 10.5 g/s or more. When the flow rate is less than 10.0 g/s, the time required for supplying the water-absorbing agent composition to a hopper or supplying the water-absorbing agent composition from the hopper to a feeder increases, which is not preferred from the viewpoint of production efficiency. The upper limit value of the flow rate is preferably 20.0 g/s or less, more preferably 15.0 g/s or less. Performing the treatment for making the flow rate higher than the above-described range may deteriorate economic efficiency. A preferred range of the flow rate can be a range defined by any combinations selected from the upper and lower limit values. Thus, the flow rate is, for example, 10.0 g/s or more and 20.0 g/s or less, preferably 10.2 g/s or more and 20.0 g/s or less, more preferably 10.5 g/s or more and 15.0 g/s or less. Detailed measurement conditions of the flow rate are described in Examples.

### [3-4] Mass Proportion of Particle Having Particle Diameter of 300 µm or More and Less Than 600 µm

In the water-absorbing agent composition according to the present invention, the lower limit value of the mass proportion of particles having a particle diameter of 300 µm or more and less than 600 µm is 50 mass% or more, preferably 53 mass% or more, more preferably 55 mass% or more, still more preferably 57 mass% or more. When the mass proportion is 50 mass% or more, the transport stability of the water-absorbing agent composition with a feeder further improves. In continuous commercial production, it may be very difficult to set each of the mass proportion of particles of less than 300 µm and the mass proportion of particles of 600 µm or more to 0 mass% from the viewpoint of production efficiency. Thus, the upper limit value of the mass proportion of particles of 300 µm or more and less than 600 µm is preferably 95 mass% or less, more preferably 90 mass% or less, still more preferably 85 mass% or less, particularly preferably 80 mass% or less, most preferably 75 mass% or less. A preferred range of the mass proportion of the particles of 300 µm or more and less than 600 µm can be a range defined by any combinations selected from the upper and lower limit values. Thus, the mass proportion of particles of 300 µm or more and less than 600 µm is, for example, 50 mass% or more and 95 mass% or less, preferably 53 mass% or more and 90 mass% or less, more preferably 55 mass% or more and 85 mass% or less, still more preferably 57 mass% or more and 80 mass% or less, particularly preferably 57 mass% or more and 75 mass% or less. According to the production method of the present invention, the aqueous solution of the flowability improver can be uniformly added to the water-absorbent resin having a large specific surface area. That is, the flowability improver is not added unevenly to particles having a fine particle diameter and a large specific surface area (particles having a particle diameter of less than 300 µm), and the like, and the kinetic friction coefficient of the particles occupying the majority of the water-absorbing agent composition, that is, the kinetic friction coefficient of the particles having a particle diameter of 300 µm or more and less than 600 µm can be greatly reduced. That is, greatly reducing the kinetic friction coefficient in the particles having a particle diameter of 300 µm or more and less than 600 µm means uniformly improving the flowability of all the particles.

### [3-5] Kinetic Friction Coefficient

In the water-absorbing agent composition according to the present invention, the upper limit value of the kinetic friction coefficient (in the present description, it may be simply referred to as "kinetic friction coefficient") in particles having a particle diameter of 300 µm or more and less than 600 µm is 0.80 or less, preferably 0.79 or less, more preferably 0.78 or less, still more preferably 0.77 or less, particularly preferably 0.76 or less, most preferably 0.73 or less. The fact that the kinetic friction coefficient exceeds 0.80 means that particles having a particle diameter of 300 µm or more and less than 600 µm are unevenly added to the flowability improver. That is, when the kinetic friction coefficient exceeds 0.80, the difference in flowability between the particles having a particle diameter of 300 µm or more and less than 600 µm and the particles not having a particle diameter of 300 µm or more and less than 600 µm increases, and as a result, the particle size segregation of the water-absorbing agent composition occurs during transportation with a feeder, which is not preferred. On the other hand, the lower limit value thereof is, for example, 0.10 or more, preferably 0.30 or more, more preferably 0.50 or more, still more preferably 0.60 or more. A preferred range of the kinetic friction coefficient of the particles of 300 µm or more and less than 600 µm can be a range defined by any combinations selected from the upper and lower limit values. Thus, the kinetic friction coefficient of the particles of 300 µm or more and less than 600 µm is, for example, 0.10 or more and 0.80 or less, preferably 0.30 or more and 0.79 or less, more preferably 0.50 or more and 0.78 or less, still more preferably 0.60 or more and 0.77 or less, particularly preferably 0.60 or more and 0.76 or less, most preferably 0.60 or more and 0.73 or less. Detailed measurement conditions of the kinetic friction coefficient of the particles having a particle diameter of 300 µm or more and less than 600 µm are described in Examples.

### [3-6] Preferred Embodiment

The water-absorbing agent composition according to the present invention preferably includes at least one of the specific surface area, the surface tension, the flow rate, the mass proportion of particles having a particle diameter of 300 µm or more and less than 600 µm, and the kinetic friction coefficient in a suitable range on the premise that all of (1) to (5) described above are satisfied. More preferably, the water-absorbing agent composition satisfies at least suitable ranges of the surface tension and the flow rate, respectively, on the premise that all of (1) to (5) are satisfied. Still more preferably, the water-absorbing agent composition satisfies at least suitable ranges of the surface tension, the flow rate, and the kinetic friction coefficient, respectively, on the premise that all of (1) to (5) are satisfied. Particularly preferably, the water-absorbing agent composition satisfies at least suitable ranges of the specific surface area, the surface tension, the flow rate, and the kinetic friction coefficient, respectively, on the premise that all of (1) to (5) are satisfied. Most preferably, the water-absorbing agent composition satisfies all of the specific surface area, the surface tension, the flow rate, the mass proportion of the particles, and the kinetic friction coefficient, on the premise that all of (1) to (5) are satisfied.

More specifically, on the premise that all of (1) to (5) are satisfied, the water-absorbing agent composition according to the present invention has a surface tension of preferably 56 mN/m or more and 75 mN/m or less, more preferably 58 mN/m or more and 75 mN/m or less, still more preferably 60 mN/m or more and 75 mN/m or less, particularly preferably 65 mN/m or more and 75 mN/m or less, and a flow rate of preferably 10.0 g/s or more and 20.0 g/s or less, more preferably 10.2 g/s or more and 20.0 g/s or less, particularly preferably 10.5 g/s or more and 15.0 g/s or less. Further, in addition to the above, the water-absorbing agent composition has a kinetic friction coefficient of preferably 0.10 or more and 0.80 or less, more preferably 0.30 or more and 0.79 or less, still more preferably 0.50 or more and 0.78 or less, still more preferably 0.60 or more and 0.77 or less, particularly preferably 0.60 or more and 0.76 or less, most preferably 0.60 or more and 0.73 or less. Further, in addition to the above, the water-absorbing agent composition has a specific surface area of preferably 25 m²/kg or more and 60 m²/kg or less, more preferably 26 m²/kg or more and 60 m²/kg or less, still more preferably 27 m²/kg or more and 60 m²/kg or less, still more preferably 28 m²/kg or more and 60 m²/kg or less, still more preferably 29 m²/kg or more and 60 m²/kg or less, still more preferably 30 m²/kg or more and 55 m²/kg or less, particularly preferably 35 m²/kg or more and 55 m²/kg or less, most preferably 36 m²/kg or more and 55 m²/kg or less. Further, in addition to the above, in the water-absorbing agent composition, the mass proportion of particles having a particle diameter of 300 µm or more and less than 600 µm is preferably 50 mass% or more and 95 mass% or less, preferably 53 mass% or more and 90 mass% or less, more preferably 55 mass% or more and 85 mass% or less, still more preferably 57 mass% or more and 80 mass% or less, particularly preferably 57 mass% or more and 75 mass% or less.

### [3-7] Other Physical Properties of Water-Absorbing Agent Composition

The water-absorbing agent composition according to the present invention preferably further has at least one of the following preferred ranges of physical properties (a) to (g).

(a) D50 (mass-average particle diameter), (b) mass proportion of particles having particle diameter of less than 150 µm, (c) CRC (absorption capacity without pressure), (d) AAP (absorption capacity under pressure), (e) SFC (saline flow conductivity), (f) Vortex (water absorption speed), (g) FHA (fixed height absorption value at height of 20 cm)

Any two or more of the preferred ranges of the physical properties (a) to (g) may be provided in combination. Most preferably, all of the preferred ranges of (a) to (g) are provided.

### (a) D50 (Mass-Average Particle Diameter)

The lower limit value of D50 (mass-average particle diameter) of the water-absorbing agent composition according to the present invention is preferably 250 µm or more, more preferably 300 µm or more, still more preferably 330 µm or more. On the other hand, the upper limit value thereof is preferably less than 550 µm, more preferably less than 500 µm, still more preferably less than 450 µm. A preferred range of D50 of the water-absorbing agent composition can be a range defined by any combinations selected from the upper and lower limit values. Thus, the D50 (mass-average particle diameter) is preferably 250 µm or more and less than 550 µm, more preferably 300 µm or more and less than 500 µm, still more preferably 330 µm or more and less than 450 µm. By setting the D50 (mass-average particle diameter) within the above-described ranges, it is possible to further control AAP (absorption capacity under pressure) and Vortex (water absorption speed), which are preferred absorption characteristics, in a well-balanced manner. When the D50 (mass-average particle diameter) is 250 µm or more, AAP (absorption capacity under pressure), which is a preferred absorption characteristic, can be maintained at a good value. On the other hand, when the D50 (mass-average particle diameter) is less than 550 µm, Vortex (water absorption speed), which is a preferred absorption characteristic, can be maintained at a good value. In addition, the roughness of the particles of the water-absorbing agent composition is less likely to stand out, and the texture and wearing feeling can be favorably maintained when the water-absorbing agent composition is used for an absorbent article such as a disposable diaper and a sanitary napkin. Detailed measurement conditions of the D50 (mass-average particle diameter) are described in Examples.

### (b) Proportion of Particles Having Particle Diameter of Less Than 150 µm

The upper limit value of the proportion of particles having a particle diameter of less than 150 µm in the water-absorbing agent composition according to the present invention is preferably 3 mass% or less, more preferably 2 mass% or less, still more preferably 1 mass% or less, particularly preferably 0 mass%, relative to 100 mass% of the water-absorbing agent composition. In continuous commercial production, it may be very difficult to set the proportion of particles of less than 150 µm to 0 mass% from the viewpoint of production efficiency. Thus, the lower limit value thereof is preferably 0.1 mass% or more, more preferably 0.2 mass% or more, still more preferably 0.3 mass% or more. A preferred range of the proportion of the particles of less than 150 µm can be a range defined by any combinations selected from the upper and lower limit values. Thus, the proportion of the particles of less than 150 µm is preferably 0.1 mass% or more and 3 mass% or less, more preferably 0.2 mass% or more and 2 mass% or less, still more preferably 0.3 mass% or more and 1 mass% or less. By setting the proportion of the particles of less than 150 µm within the above-described ranges, AAP (absorption capacity under pressure) and Vortex (water absorption speed) can be more easily controlled in a well-balanced manner. When the proportion of the particles having a particle diameter of less than 150 µm is 3 mass% or less, not only AAP (absorption capacity under pressure), which is a preferred absorption characteristic, is maintained at a good value, but also deterioration of a working environment due to scattering of dust at a place where the water-absorbing agent composition is handled and difficulty in handling due to deposition of fine particles in an apparatus can be suppressed.

In addition, the water-absorbing agent composition preferably satisfies the preferred range of D50 (mass-average particle diameter) within the above-described ranges and the preferred range of the proportion of the particles of less than 150 µm within the above-described ranges. By satisfying both of them, the above-described effect is synergistically obtained. Specifically, the proportion of the particles having a D50 (mass-average particle diameter) of 250 µm or more and less than 550 µm in the water-absorbing agent composition and having a particle diameter of less than 150 µm in the water-absorbing agent composition is preferably 3 mass% or less. Preferred ranges of D50 (mass-average particle diameter) and the proportion of the particles having a particle diameter of less than 150 µm in the water-absorbing agent composition are as described in (a) and (b), respectively. The D50 (mass-average particle diameter) and particles having a particle diameter of less than 150 µm in the water-absorbing agent composition are measured by the method described in Examples.

### (c) CRC (Absorption Capacity Without Pressure)

The lower limit value of CRC (absorption capacity without pressure) of the water-absorbing agent composition according to the present invention is preferably 25 g/g or more. On the other hand, the upper limit value thereof is preferably 40 g/g or less, more preferably 38 g/g or less, still more preferably 35 g/g or less, particularly preferably 32 g/g or less, most preferably 30 g/g or less. A preferred range of the CRC can be a range defined by any combinations selected from the upper and lower limit values. When the CRC (absorption capacity without pressure) is too low, the absorption capacity of the water-absorbing agent composition decreases, and there is a possibility that the water-absorbing agent composition is not suitable for use as an absorbent body of an absorbent article such as a disposable diaper or a sanitary napkin. On the other hand, when the CRC (absorption capacity without pressure) is too high, the gel strength may be weakened.

### (d) AAP (Absorption Capacity Under Pressure)

The lower limit value of AAP (absorption capacity under pressure) of the water-absorbing agent composition according to the present invention is preferably 20 g/g or more, more preferably 23 g/g or more, still more preferably 25 g/g or more, particularly preferably 25.2 g/g or more. On the other hand, the upper limit value thereof is preferably 30 g/g or less, more preferably 28 g/g or less. A preferred range of the AAP can be a range defined by any combinations selected from the upper and lower limit values. Thus, the AAP is preferably 20 g/g or more and 30 g/g or less, more preferably 23 g/g or more and 30 g/g or less, still more preferably 25 g/g or more and 30 g/g or less, particularly preferably 25.2 g/g or more and 28 g/g or less. By setting the AAP (absorption capacity under pressure) within the above-described ranges, the return amount of the liquid when pressure is applied to the absorbent body can be further reduced, and thus, a water-absorbent resin or a water-absorbing agent composition suitable for use in an absorbent body of an absorbent article such as a disposable diaper or a sanitary napkin is obtained.

### (e) SFC (Saline Flow Conductivity)

The lower limit value of SFC (saline flow conductivity) of the water-absorbing agent composition according to the present invention is preferably 1 × 10⁻⁷ cm³·sec/g or more, more preferably 2 × 10⁻⁷ cm³·sec/g or more, still more preferably 3 × 10⁻⁷ cm³·sec/g or more, still more preferably 5 × 10⁻⁷ cm³·sec/g or more, still more preferably 10 × 10⁻⁷ cm³·sec/g or more, still more preferably 15 × 10⁻⁷ cm³·sec/g or more, particularly preferably 20 × 10⁻⁷ cm³·sec/g or more, most preferably 25 × 10⁻⁷ cm³·sec/g or more. The upper limit value of SFC is preferably as high as possible, and is not particularly limited, but it may be, for example, 50 × 10⁻⁷ cm³·sec/g or less. A preferred range of SFC can be a range defined by any combinations selected from the upper and lower limit values. Thus, SFC is preferably 1 × 10⁻⁷ cm³·sec/g or more and 50 × 10⁻⁷ cm³·sec/g or less, more preferably 2 × 10⁻⁷ cm³·sec/g or more and 50 × 10⁻⁷ cm³·sec/g or less, still more preferably 3 × 10⁻⁷ cm³·sec/g or more and 50 × 10⁻⁷ cm³·sec/g or less, still more preferably 5 × 10⁻⁷ cm³·sec/g or more and 50 × 10⁻⁷ cm³·sec/g or less, still more preferably 10 × 10⁻⁷ cm³·sec/g or more and 50 × 10⁻⁷ cm³·sec/g or less, still more preferably 15 × 10⁻⁷ cm³·sec/g or more and 50 × 10⁻⁷ cm³·sec/g or less, particularly preferably 20 × 10⁻⁷ cm³·sec/g or more and 50 × 10⁻⁷ cm³·sec/g or less, most preferably 25 × 10⁻⁷ cm³·sec/g or more and 50 × 10⁻⁷ cm³·sec/g or less. Detailed measurement conditions of SFC are described in Examples.

### (f) Vortex (Water Absorption Speed)

The upper limit value of Vortex (water absorption speed) of the water-absorbing agent composition according to the present invention is preferably 50 seconds or less, more preferably 48 seconds or less, still more preferably 46 seconds or less, particularly preferably 44 seconds or less, most preferably 42 seconds or less. On the other hand, the lower limit value thereof is preferably more than 10 seconds, more preferably 15 seconds or more. A preferred range of the Vortex can be a range defined by any combinations selected from the upper and lower limit values. Thus, the Vortex (water absorption speed) is preferably more than 10 seconds and 50 seconds or less, more preferably more than 10 seconds and 48 seconds or less, still more preferably more than 10 seconds and 46 seconds or less, particularly preferably more than 10 seconds and 44 seconds or less, most preferably 15 seconds or more and 42 seconds or less. When the Vortex (water absorption speed) is 50 seconds or less, the water absorption speed for body fluids such as urine and blood of the resulting water-absorbing composition is high, and the water-absorbing agent composition is suitable as an absorbent body of an absorbent article such as a disposable diaper. Vortex (water absorption speed) can be controlled by foaming polymerization, particle size distribution, and the like. Detailed measurement conditions of Vortex (water absorption speed) are described in Examples.

### (g) FHA (Fixed Height Absorption Value at Height of 20 cm)

The lower limit value of FHA (fixed height absorption value at a height of 20 cm) of the water-absorbing agent composition according to the present invention is preferably 24.0 g/g or more, more preferably 24.5 g/g or more, still more preferably 25.0 g/g or more, particularly preferably 25.5 g/g or more. The upper limit value is not particularly limited, and is preferably 30.0 g/g or less from the viewpoint of balance with other physical properties. A preferred range of the FHA can be a range defined by any combinations selected from the upper and lower limit values. When the FHA is 24.0 g/g or more, a water absorption capacity when pressure is applied to the absorbent body is improved, and thus the water-absorbing agent material is suitable as an absorbent body of an absorbent article such as a disposable diaper. The FHA can be controlled with an internal crosslinking agent, a particle size, a surface crosslinking agent, and/or an additive added after the surface crosslinking step. Detailed measurement conditions of the FHA (fixed height absorption value at a height of 20 cm) are described in Examples.

### [3-8] Relationship Between Water-Absorbent Resin and Water-Absorbing Agent Composition

The lower limit value of the amount (mass proportion) of the water-absorbent resin contained in the water-absorbing agent composition according to the present invention is preferably 80% or more, more preferably 85% or more, still more preferably 90% or more, particularly preferably 95 mass% or more, more preferably 98 mass% or more, still more preferably 99 mass% or more, relative to the total amount of the water-absorbing agent composition. When the above-described various additives (flowability improver, other additives, and water added together when they are added) are contained, the amount of the water-absorbent resin contained in the water-absorbing agent composition is not 100 mass%. That is, the upper limit value of the amount (mass proportion) of the water-absorbent resin contained in the water-absorbing agent composition according to the present invention may be less than 100 mass%.

The shape of the water-absorbing agent composition according to the present invention may be any of a spherical shape, a granulated product, an aggregate, an irregularly crushed shape, and the like, but in consideration of the water absorption speed, it is preferred that the water-absorbing agent composition has an irregularly crushed shape.

### [4] Application of Water-Absorbing Agent Composition

The water-absorbing agent composition according to the present invention is preferably used mainly as an absorbent body of an absorbent article such as a disposable diaper or a sanitary napkin, or as an absorbent layer (hereinafter, collectively referred to as an "absorbent body"), and more preferably used as an absorbent body of an absorbent article having a large amount used per absorbent article. That is, another aspect of the present invention provides an absorbent body including the water-absorbing agent composition.

The absorbent body means an article obtained by molding the water-absorbing agent composition into a sheet shape, a fibrous shape, a cylindrical shape, or the like, and is preferably molded into a sheet shape to form an absorbent layer. In addition to the water-absorbing agent composition according to the present invention, an absorbent material such as pulp fibers (pulp), or an adhesive, a nonwoven fabric, or the like can be molded and used in combination. Thus, in one embodiment, the absorbent body according to the present invention further contains pulp fibers (pulp) in addition to the absorbing agent. In this case, the lower limit of the amount of the water-absorbing agent composition in the absorbent body (hereinafter, referred to as "core concentration") is preferably 50 mass% or more, more preferably 60 mass% or more, still more preferably 70 mass% or more, particularly preferably 80 mass% or more. On the other hand, the upper limit value thereof is preferably 100 mass% or less. A preferred range of the core concentration can be a range defined by any combinations selected from the upper and lower limit values. By setting the core concentration within the above-described ranges, when the absorbent body is used for an absorbent article, an appropriate space can be formed between gel particles even though the water-absorbing agent composition is gelled by absorbing urine.

In the absorbent body, the basis weight of the water-absorbing agent composition is preferably 25 g/m² or more and 450 g/m² or less, more preferably 50 g/m² or more and 400 g/m² or less, still more preferably 75 g/m² or more and 350 g/m² or less. When the basis weight of the water-absorbing agent composition is within the above-described ranges, urine can be efficiently absorbed when the absorbent body is used for an absorbent article.

In the absorbent body, the basis weight of the absorbent material such as pulp (preferably, pulp) is preferably 0 g/m² or more and 300 g/m² or less, more preferably 0 g/m² or more and 250 g/m² or less, still more preferably 0 g/m² or more and 200 g/m² or less, particularly preferably 0 g/m² or more and 150 g/m² or less. When the basis weight of the pulp is within the above-described ranges, the absorbent article can be reduced in thickness when the absorbent body is used for the absorbent article. As described above, in the absorbent body, the basis weight of the absorbent material such as pulp (preferably, pulp) may be 0 g/m². That is, in one embodiment, the absorbent body may include no pulp.

Specific examples of the absorbent body include a long water-absorbent sheet in which the water-absorbing agent composition (water-absorbent resin) is immobilized between two sheets, the long water-absorbent sheet being cut into a rectangle having a width of about 10 cm × a length of about 10 cm. Such an absorbent body is produced by cutting the long water-absorbent sheet in a step of producing a disposable diaper, and in recent years, has come to be used for producing a disposable diaper (so-called SAP sheet diaper). The disposable diaper manufacturer can simplify the step of producing a disposable diaper by purchasing or producing the long water-absorbent sheet, and can further reduce the thickness of the disposable diaper by not using pulp. The water-absorbent sheet has a configuration in which the water-absorbing agent composition (water-absorbent resin particles) is sandwiched and immobilized between upper and lower sheets (in particular, nonwoven fabric sheets). In the production of a disposable diaper, usually, after the long water-absorbent sheet is produced, the sheet is cut into a rectangle having a width of about 10 cm and a length of several 10 cm, and incorporated into a disposable diaper (see WO 2010/143635).

### [5] Absorbent Article

The water-absorbing agent composition according to the present invention is suitably used in an absorbent article. That is, another aspect of the present invention is an absorbent article including the water-absorbing agent composition. Another aspect of the present invention provides an absorbent article including the absorbent body. Such an absorbent article usually includes a surface sheet having liquid permeability and a back sheet having liquid impermeability, in addition to the absorbent body. Examples of the absorbent article include a disposable diaper and a sanitary napkin.

When the absorbent article is, for example, a disposable diaper, the disposable diaper is produced by sandwiching an absorbent body containing the water-absorbing agent composition of the present invention between a liquid-permeable topsheet positioned on the side that comes into contact with human skin when worn and a liquid-impermeable backsheet positioned outside when worn. The disposable diaper is further provided with a member known to those skilled in the art, such as an adhesive tape for fixing the disposable diaper while being worn.

In one embodiment, it is preferred that the absorbent article contains no pulp. That is, the absorbent article is preferably a diaper containing no pulp. The absorbent article may be a diaper containing no pulp in which the basis weight of the water-absorbing agent composition in the absorbent body contained therein is within the above-described range.

In another embodiment, the absorbent article may further contain pulp in addition to the water-absorbing agent composition. That is, the absorbent article may be a diaper containing pulp. The absorbent article may be a diaper containing pulp in which the basis weight of pulp in the absorbent body contained therein is within the above-described range.

### Examples

Hereinafter, the present invention will be described more specifically by way of Examples, but the present invention is not limited by the following Examples as a matter of course, and can be implemented with appropriate modifications within a range that can be adapted to the gist described above and below, and all of them are included in the technical scope of the present invention. In addition, in the present invention, the measurement method of each physical property is based on the measurement method described in Examples unless otherwise specified.

The method for measuring each physical property in the table is as follows. Each measurement was performed under the conditions of room temperature (23 ± 2°C) and a relative humidity of 35 ± 5%RH. When the object to be measured is a substance other than the water-absorbing agent composition (for example, a particulate hydrogel, a water-absorbent resin before/after surface crosslinking, and the like) in the following description related to the method for measuring each physical property, the "water-absorbing agent composition" in the following description is applied by replacing it with "particulate hydrogel", "water-absorbent resin before surface crosslinking", and "water-absorbent resin after surface crosslinking".

### Method for Measuring Physical Property

### Kinetic Friction Coefficient

In the water-absorbing agent composition according to the present invention, the kinetic friction coefficient of particles having a particle diameter of 300 µm or more and less than 600 µm was measured by the method described below.

### Classification of Water-Absorbing Agent Composition

The water-absorbing agent composition in an amount of 100.0 g was classified using two JIS standard sieves (THE IIDA TESTING SIEVE: diameter 16 cm) having openings of 600 µm and 300 µm. The classification was performed by sieving for 5 minutes with a vibration classifier (IIDA SIEVE SHAKER, type: ES-65 type (rotational speed: 60 Hz 230 rpm, number of impulses: 60 Hz 130 rpm), SER. No. 0501).

Through the classification operation, a water-absorbing agent composition having a particle diameter of 600 µm or more, a water-absorbing agent composition having a particle diameter of 300 µm or more and less than 600 µm, and a water-absorbing agent composition having a particle diameter of less than 300 µm were obtained.

### (Measurement of Shear Stress with Rheometer)

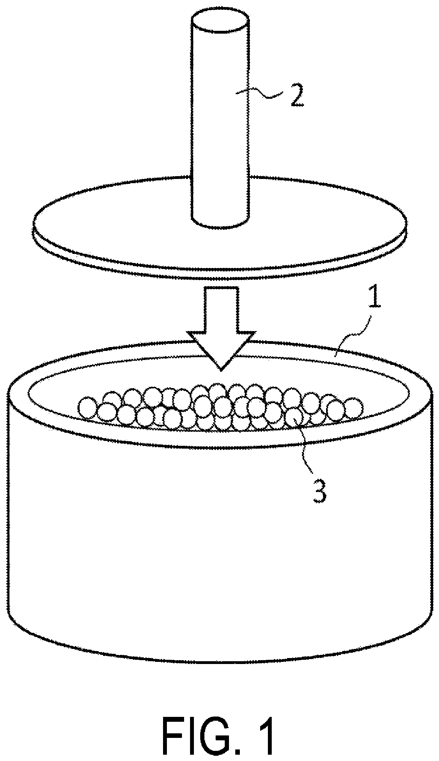

The shear stress of the water-absorbing agent composition having a particle diameter of 300 µm or more and less than 600 µm obtained through the classification operation was measured using a rheometer (MCR301 available from Anton Paar GmbH) (see FIG. 1). Hereinafter, the measurement method will be described in detail with reference to FIG. 1.
1. Dish 1 (inner diameter; 52 mm, depth; 30 mm, material; aluminum, remarks; the inner bottom surface is sandblasted) and parallel plate 2 (diameter; 50 mm, material; aluminum, remarks; the plate surface is processed into a lattice pattern) were placed on a rheometer. The dish 1 and the parallel plate 2 to be used were sufficiently washed and dried. The rheometer and the dish 1 were installed so as to be strictly horizontal.
2. The measurement temperature was set to 25°C, and the lift position (distance between the dish 1 and the parallel plate 2) was set to 100 mm.
3. The zero gap between the dish 1 and the parallel plate 2 was adjusted.
4. The parallel plate 2 was lifted up to the lift position set through the above-described operation 2, and the distance between the dish 1 and the parallel plate 2 was increased to 100 mm. Thereafter, 10.0 g of the water-absorbing agent composition having a particle diameter of 300 µm or more and less than 600 µm obtained through the classification operation was uniformly sprayed into the dish 1. The dish 1 and the parallel plate 2 were not removed from the rheometer after the zero gap adjustment operation in the above-described 3. That is, when the water-absorbing agent composition was sprayed into the dish 1, the operation was performed with the dish 1 and the parallel plate 2 being installed in the rheometer. When the dish 1 and the parallel plate 2 were once removed from the rheometer for washing or the like, the zero gap adjustment was performed again when the dish 1 and the parallel plate 2 were installed in the rheometer, and the water-absorbing agent composition was applied without removing the dish 1 or the parallel plate 2 from the rheometer.
5. The parallel plate 2 was lowered to a measurement start position (a position where the water-absorbing agent composition sprayed in the dish 1 and the parallel plate 2 were in contact with each other).
6. The shear stress of the water-absorbing agent composition was measured under the measurement conditions described in Table 1 shown below. Conditions (1) to (5) described in Table 1 shown below were continuously measured, and the time required from the start of condition (1) to the end of condition (5) was 1050 seconds. After the measurement, the parallel plate was pulled up to a lift position of 100 mm, and the powder surface (surface in contact with the parallel plate) of the water-absorbing agent composition was checked. At this time, for example, when a part of the powder surface had a recess or the like and a part of the parallel plate was not in contact with the powder surface, the measurement was performed again. That is, only a case where the powder surface of the water-absorbing agent composition after the measurement had no recess or the like and the entire bottom surface of the parallel plate was in contact with the powder surface was adopted as a case where the measurement was correctly performed.
7. Among the shear stresses obtained in the measurement of 6, the average value of the values at the 41st to 60th measurement points among the shear stresses measured under the conditions (3), (4), and (5) described in Table 1 shown below was defined as the shear stress (unit: Pa) of the water-absorbing agent composition under the conditions (3), (4), and (5), that is, under normal loads of 1 N, 3 N, and 5 N.

The shear stress under normal loads of 1 N (condition (3)), 3 N (condition (4)), and 5 N (condition (5)) of the water-absorbing agent composition having a particle diameter of 300 µm or more and less than 600 µm obtained through the classification operation in the present invention was measured twice using the same sample (water-absorbing agent composition), and the average value thereof was taken as the shear stress. However, when there was a difference of more than 150 Pa in the shear stress at 5 N (condition (5)) in two measurements, additional measurements were performed, and an average value was calculated from the two measurements in which the difference was 150 Pa or less.

**Table 1**

| | Normalizing conditions | | Measurement conditions | | |
|---|---|---|---|---|---|
| | Condition (1) | Condition (2) | Condition (3) | Condition (4) | Condition (5) |
| Measurement mode | Rotation | Vibration | Vibration | Vibration | Vibration |
| Rotation condition or vibration condition | Rotation 10/min | Strain: 2% Angular frequency | Strain: 3% Angular frequency | Strain: 3% Angular frequency | Strain: 3% Angular frequency |
| | | ω: 10 rad/s | ω: 50 rad/s | ω: 50 rad/s | ω: 50 rad/s |
| Normal load (N) | 0.1 | 0.3 | 1 | 3 | 5 |
| Measurement temperature (°C) | 25 | 25 | 25 | 25 | 25 |
| Measurement interval (sec) | 5 | 5 | 5 | 5 | 5 |
| Measurement time (sec) | 90 | 60 | 300 | 300 | 300 |
| Number of measurement points (points) | 18 | 12 | 60 | 60 | 60 |

### Calculation of Vertical Stress

The vertical stress (unit: Pa) of the water-absorbing agent composition under the conditions (3), (4), and (5) described in Table 1 was calculated according to the following (Equation a).$Vertical stress \left(Pa\right) = \left(normal load\left(N\right)\right) / \left(area of parallel plate\left({m}^{2}\right)\right)$

The area of the parallel plate in (Equation a) is 0.0252 × π (m²).

That is, the vertical stresses of the water-absorbing agent composition having a particle diameter of 300 µm or more and less than 600 µm obtained through the classification operation under the normal loads of 1 N (condition (3)), 3 N (condition (4)), and 5 N (condition (5)) were 509 Pa, 1528 Pa, and 2546 Pa, respectively.

### Calculation of Kinetic Friction Coefficient

The kinetic friction coefficient of the water-absorbing agent composition having a particle diameter of 300 µm or more and less than 600 µm obtained through the classification operation was calculated by the following method.
1. The shear stresses and the vertical stresses of the water-absorbing agent composition at normal loads of 1 N, 3 N, and 5 N were plotted with the vertical axis as the shear stress and the horizontal axis as the vertical stress.
2. The plot of 3 points was linearly approximated by a straight line passing through the origin without intercept, and the slope of the straight line was taken as the kinetic friction coefficient of the water-absorbing agent composition.

In the linear approximation, when the R2-squared value of the approximate expression was less than 0.9000, the shear stress measurement with the rheometer was performed again, and only the slope of the linear approximation in which the R2-squared value was 0.9000 or more was adopted as the kinetic friction coefficient

### Kinetic Friction Coefficient Reduction Percentage

In the water-absorbing agent composition according to the present invention, the kinetic friction coefficient reduction percentage (unit: %) in particles having a particle diameter of 300 µm or more and less than 600 µm was calculated according to the following (Equation 2). $Kinetic friction coefficient reduction percentage \left(%\right) = \left(A-B\right) / A \times 100$

In Equation 2,
A: kinetic friction coefficient of particles having a particle diameter of 300 µm or more and less than 600 µm in the water-absorbent resin before addition of the flowability improver;
B: kinetic friction coefficient of particles having a particle diameter of 300 µm or more and less than 600 µm in the water-absorbing agent composition after addition of the flowability improver

The kinetic friction coefficients of A and B were each measured by the above-described method, and then the kinetic friction coefficient reduction percentage was calculated using (Equation 2).

### Feeding Test

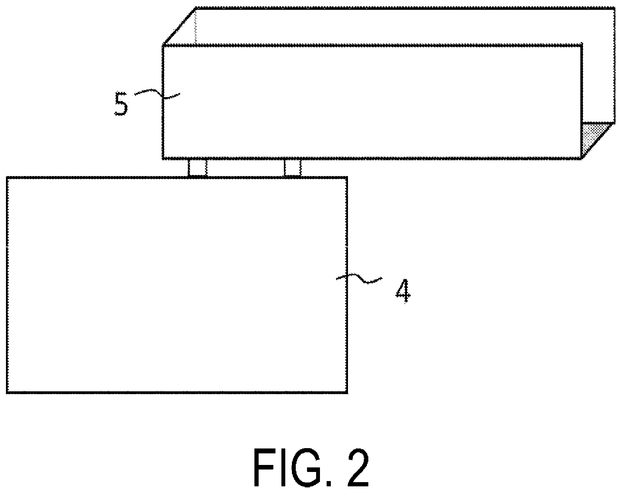

A feeding test of the water-absorbing agent composition according to the present invention was performed using an electromagnetic feeder drive unit 4 (series; MFS small series, type; MUS-6, manufacturer; MURAKAMISEIKI Co., Ltd.) (see FIG. 2) and a controller (series; MC type, type; MC-2-2, manufacturer; MURAKAMISEIKI Co., Ltd.). The test method will be described in detail below with reference to FIG. 2.
1. A trough 5 (total length; 20 cm, width; 7 cm, weir; 5 cm, material; SUS made) was installed in the electromagnetic feeder drive unit 4.
2. The water-absorbing agent composition in an amount of 50.0 g was disposed on the trough 5 so as to be located at a position of 12 cm to 20 cm from the outlet of the trough 5.
3. The controller scale was set to 2, and the feeding was initiated.
4. The water-absorbing agent composition dropped from the outlet of the trough 5 was sampled from 70 seconds to 120 seconds after the start of the feeding.

### Particle Size Change Percentage

The particle size change percentage (unit: %) of the water-absorbing agent composition according to the present invention was calculated by the following method.

### (Classification of Water-Absorbing Agent Composition)

Using two JIS standard sieves (THE IIDA TESTING SIEVE: diameter 8 cm) having openings of 600 µm and 300 µm, the water-absorbing agent composition before performing the feeding test and 10.0 g of the water-absorbing agent composition sampled in the feeding test were classified. The classification was performed by sieving for 5 minutes with a vibration classifier (IIDA SIEVE SHAKER, type: ES-65 type (rotational speed: 60 Hz 230 rpm, number of impulses: 60 Hz 130 rpm), SER. No. 0501).

The water-absorbing agent composition before the feeding test and the water-absorbing agent composition sampled in the feeding test were classified into a water-absorbing agent composition having a particle diameter of 600 µm or more, a water-absorbing agent composition having a particle diameter of 300 µm or more and less than 600 µm, and a water-absorbing agent composition having a particle diameter of less than 300 µm through the classification operation.

### Calculation of Particle Size Change Percentage

The particle size change percentage was calculated based on the following (Equation b) to (Equation d) for "the water-absorbing agent composition having a particle diameter of 600 µm or more", "the water-absorbing agent composition having a particle diameter of 300 µm or more and less than 600 µm", and "the water-absorbing agent composition having a particle diameter of less than 300 µm". 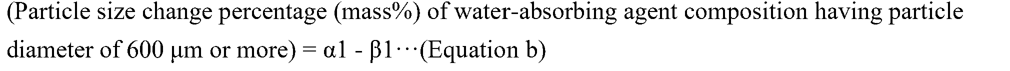 (Particle size change percentage (mass%) of water-absorbing agent composition having particle diameter of 600 µm or more) = α1 - β1···(Equation b) where:
α1: mass proportion of water-absorbing agent composition having particle diameter of 600 µm or more in water-absorbing agent composition before feeding test (unit: mass%)
β1: mass proportion of water-absorbing agent composition having particle diameter of 600 µm or more in water-absorbing agent composition sampled in feeding test (unit: mass%) 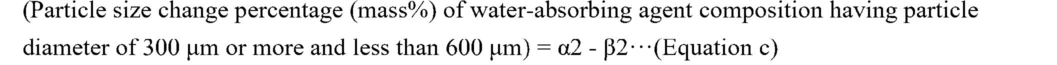
(Particle size change percentage (mass%) of water-absorbing agent composition having particle diameter of 300 µm or more and less than 600 µm) = α2 - β2···(Equation c) where:
α2: mass proportion of water-absorbing agent composition having particle diameter of 300 µm or more and less than 600 µm in water-absorbing agent composition before feeding test (unit: mass%)
β2: mass proportion of water-absorbing agent composition having particle diameter of 300 µm or more and less than 600 µm in water-absorbing agent composition sampled in feeding test (unit: mass%) 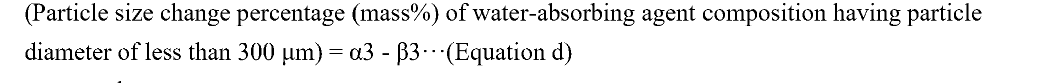
(Particle size change percentage (mass%) of water-absorbing agent composition having particle diameter of less than 300 µm) = α3 - β3···(Equation d) where:
α3: mass proportion of water-absorbing agent composition having particle diameter of less than 300 µm in water-absorbing agent composition before feeding test (unit: mass%)
β3: mass proportion of water-absorbing agent composition having particle diameter of less than 300 µm in water-absorbing agent composition sampled in feeding test (unit: mass%)

### Specific Surface Area

The specific surface area of the water-absorbing agent composition according to the present invention is a value obtained by analyzing three-dimensional image data of the water-absorbing agent composition acquired using a microfocus X-ray CT system (inspeXio SMX-100CT available from Shimadzu Corporation) with high-speed three-dimensional analysis software (TRI/3D-VOL-FCS64 available from RATOC SYSTEM ENGINEERING CO., LTD.). Specifically, first, 1.0 g of the water-absorbing agent composition was put into a plastic lidded cylindrical container having an inner diameter of about 1 cm and a height of about 5 cm, and was shaken well so that there was no deviation in particle size. Subsequently, a double-sided tape was attached to the bottom surface of the cylindrical container, the container was fixed on a sample stage of the microfocus X-ray CT system, and then three-dimensional image data was acquired under the conditions of Table 2 shown below.

### Table 2

**Table 2**

| | | | |
|---|---|---|---|
| X-ray tube voltage (kV) | : 50 | Number of views | : 1200 |
| X-ray tube current (µA) | : 40 | Average number | : 5 |
| Inch size (inch) | : 4.0 | Number of multiple rotation | : None |
| X-ray filter | : None | Smoothing | : YZ |
| SDD (mm) | : 500 | Slice thickness (mm) | : 0.008 |
| SRD (mm) | : 40 | Distance between slices (mm) | : 0.010 |
| Z (mm) | : 108 | Scaling factor | : 50 |
| X (mm) | : 0 | BHC data | : None |
| Y (mm) | : 0 | Fine mode | : Present |
| CT mode 1 | : CBCT | FOVXY (mm) | : 5.0 |
| CT mode 2 | : Normal scanning | FOVZ (mm) | : 4.0 |
| Scan angle | : Full scan | Voxel size (mm/voxcel) | : 0.010 |

Subsequently, analysis was performed according to the following procedure using the high-speed three-dimensional analysis software.
1. From the menu field, particle measurement > 3D particles > particle separation > giant particle separation was selected.
2. In the Binarize tab on the EVC panel, L-W was selected, the W value was maintained at the initial value, the L value was changed from the initial value to a value larger by one, and a circular region to be measured was extracted. This process was then applied to all slice images. The image data extracted through this operation is denoted by (A).
3. In the Binarize tab on the EVC panel, L-W was selected, the W value was maintained at the initial value, the L value was changed from the initial value to 37580, and all the particles in the region to be measured were extracted. This process was then applied to all slice images. The particle image data extracted through this operation is denoted by (B).
4. Based on the particle image data (B), first, Ers Sml was selected in the Binary tab on the EVC panel to remove particles considered as noise with a particle size of 10 voxcel or less. Subsequently, in the Binary tab on the EVC panel, Invert was selected to invert the region from which the particles were extracted and the region from which the particles were not extracted. Subsequently, Ers Sml was selected to remove particles considered as noise with a particle size of 10 voxcel or less. Subsequently, in the 3D tab on the EVC panel, Labeling was selected, the volume and Max were further selected, and only the region with the largest volume was extracted. Finally, by selecting Invert again in the Binary tab on the EVC panel, noise was removed, and all the particles were extracted in a state of being filled with Void in the region to be measured. The particle image data extracted through these operations is denoted by (C). The term "Void" as used herein refers to a cavity that is present inside the water-absorbent resin and is not in contact with the outside world.
5. Void was extracted by subtracting the particle image data (B) from the particle image data (C) with the L Op tab (inter-channel logic operation processing), selecting Ers Sml in the Binary tab on the EVC panel, and removing particles considered to be noise with a particle size of 10 voxcel or less.
6. Based on the particle image data (C), small particle extraction was selected on a large particle separation panel (large particle extraction was not selected), the constriction ratio, Repair Filter Size, and Repair Mrg Sml Diameter were all set to 0, and particle separation and color-coding were performed.
7. In the 3D tab on the EVC panel, Labeling was selected, the coordinate value (cycle) was further selected, the microparticle size was set to 10, and the particle separation operation was performed.
8. Particle measurement > Void in 3D particles > Measurement after separation was selected from the menu field. Subsequently, on the post-separation measurement panel, voxcel was selected as the unit, edge particles were removed, surface area calculation and Void calculation were selected as measurement items, image data (A) extracted through the operation 2 described above was selected as measurement ROI designation, and calculation processing was performed.

Through the above-described operation, the total surface area (unit: mm²) and the apparent total volume (unit: mm³) of all the particles in the region to be measured, and the void total volume (unit: mm³) were calculated. The apparent total volume refers to the total volume of all the particles calculated assuming that there is no void inside the particles. In addition, the specific surface area (unit: m²/kg) of the water-absorbing agent composition was calculated from the following (Equation e) using the value obtained through the image analysis and assuming that the true density of the water-absorbing agent composition was 1.7 g/cm³. 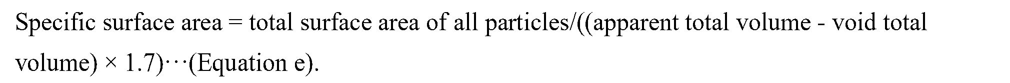 Specific surface area = total surface area of all particles/((apparent total volume - void total volume) × 1.7)···(Equation e).

### ST (Surface Tension)

ST (surface tension) of the water-absorbing agent composition according to the present invention was measured by the following method.

First, 40 mL of a 0.9 mass% aqueous sodium chloride solution adjusted from 23°C to 24°C was put in a sufficiently washed 50 mL beaker, and the surface tension of the 0.9 mass% aqueous sodium chloride solution was measured using a surface tensiometer (K11 automatic surface tensiometer available from KRUSS GmbH). In this measurement, the value of the surface tension needs to be within the range of 72 mN/m to 74 mN/m.

Next, after surface tension measurement adjusted from 23°C to 24°C was performed, a sufficiently washed cylindrical stirrer having a length of 25 mm and a section diameter of 7 mm and 0.5 g of the water-absorbing agent composition were put into a beaker containing 40 mL of a 0.9 mass% aqueous sodium chloride solution, and stirred at 350 rpm for 3 minutes. After 3 minutes, the stirring was stopped, and the mixture was allowed to stand for 2 minutes. After the water-absorbing agent composition that had absorbed water was settled, the surface tension of the supernatant was measured again through the same operation. In the present measurement, a plate method using a platinum plate was adopted, and the plate was used after being sufficiently washed with deionized water and heated and washed with a gas burner before each measurement.

### Flow Rate

The flow rate (unit: g/s) of the water-absorbing agent composition according to the present invention was measured in accordance with WSP250.3 (10). Specifically, 100.0 g of the water-absorbing agent composition was put into a funnel provided with a damper at the lower part, the damper was opened, and the time from the start of flowing down to the end of flowing down was measured to calculate the amount of flowing down of the water-absorbing agent composition per unit time, and this was taken as the flow rate (unit: g/s).

### [D50 (Mass-Average Particle Diameter) and σζ (Logarithmic Standard Deviation Of Particle Size Distribution)]

D50 (mass-average particle diameter) and σζ (logarithmic standard deviation of particle size distribution) of the water-absorbing agent composition according to the present invention were measured in accordance with the measurement method described in paragraphs [0245] to [0246] of US 7638570.

### Vortex (Water Absorption Speed)

Vortex (water absorption speed) of the water-absorbing agent composition according to the present invention was measured by the following procedure in accordance with JIS K 7224 (1996). First, 0.02 parts by mass of Food Blue No. 1 (CAS No. 3844-45-9) as a food additive was added to 1000 parts by mass of physiological saline for coloring, and the liquid temperature was adjusted to 30°C. This was used as a test liquid. Next, 50 mL of the test liquid was weighed in a beaker having a volume of 100 mL, a cylindrical stirrer having a length of 40 mm and a diameter of 8 mm was put in the beaker, and stirring was started at 600 rpm. Subsequently, 2.0 g of the water-absorbent resin was placed in the test liquid during stirring, and the time until the stirrer (stirrer chip) was covered with the test liquid was measured and taken as the water absorption speed by the Vortex method.

### SFC (Saline Flow Conductivity)

SFC (saline flow conductivity) (unit: ×10⁻⁷ cm³·sec/g) of the water-absorbing agent composition according to the present invention was measured in accordance with the measurement method described in US 5669894.

Specifically, 1.500 g of the water-absorbing agent composition was uniformly placed in a container, then the water-absorbing agent composition was immersed in artificial urine, and the water-absorbing agent composition was swollen while being pressurized at a pressure of 2.07 kPa. The artificial urine was prepared by mixing 0.25 g of calcium chloride dihydrate, 2.0 g of potassium chloride, 0.50 g of magnesium chloride hexahydrate, 2.0 g of sodium sulfate, 0.85 g of ammonium dihydrogen phosphate, 0.15 g of diammonium hydrogen phosphate, and 994.25 g of pure water.

60 minutes after the pressurization, the height (cm) of the gel layer of the swollen water-absorbing agent composition was recorded. Next, a 0.69 mass% aqueous sodium chloride solution was allowed to pass through the gel layer while the gel layer was pressurized at a pressure of 2.07 kPa. The room temperature at this time was adjusted from 20°C to 25°C. Then, the amount of saline passing through the gel layer was recorded at intervals of 20 seconds using a balance and a computer, and the flow rate Fs (T) of the saline passing through the gel layer was measured. The flow rate Fs (T) is measured by dividing the mass of saline passed (g), which increases every 20 seconds, by the transit time (s). The time at which the hydrostatic pressure of the saline became constant and a stable flow rate was obtained was defined as Ts, and the flow rate Fs (T = 0) was calculated using data measured for 10 minutes starting from Ts. That is, Fs (T) was plotted against time, and Fs (T = 0) was calculated based on the result obtained by the least squares method. Fs (T = 0) is the initial flow rate (g/s) of saline through the gel layer. Then, the saline flow conductivity (SFC) was calculated by the following (Equation f).$SFC = \left\{Fs\left(T=0\right)\times L0\right\} / \left(ρ\times A\times ΔP\right)$ where
L0: height of gel layer (unit: cm)
ρ: density of saline (unit: g/cm³)
A: sectional area of gel layer (unit: cm²)
ΔP: hydrostatic pressure applied to gel layer (unit: dyne/cm²)

### CRC (Absorption Capacity Without Pressure)

CRC (absorption capacity without pressure) of the water-absorbing agent composition according to the present invention was measured in accordance with NWSP 241.0. R2 (19). Specifically, 0.2 g of the water-absorbing agent composition was put in a bag made of a nonwoven fabric, and then immersed in a large excess of a 0.9 mass% aqueous sodium chloride solution for 30 minutes to freely swell the water-absorbing agent composition. Thereafter, the water-absorbing agent composition was dehydrated using a centrifuge (250 G), and then CRC (absorption capacity without pressure) (unit: g/g) was measured.

### AAP (Absorption Capacity Under Pressure)

AAP (absorption capacity under pressure) of the water-absorbing agent composition according to the present invention was measured in accordance with NWSP 242.0. R2 (19). Specifically, 0.9 g of the water-absorbing agent composition was allowed to swell under a load of 4.83 kPa (49 g/cm², 0.7psi) for 1 hour in a large excess of a 0.9 mass% aqueous sodium chloride solution, and then AAP (absorption capacity under pressure) (unit: g/g) was measured.

### FHA (Fixed Height Absorption Value at Height of 20 cm)

FHA (fixed height absorption value at a height of 20 cm) of the water-absorbing agent composition according to the present invention was measured in accordance with the measurement method described in paragraphs [0104] to [0116] of US 2005/0003191 A.

### pH of Aqueous Solution of Flowability Improver

The pH of the aqueous solution of the flowability improver according to the present invention was measured using a portable pH meter D-71 available from HORIBA, Ltd. while the aqueous solution was stirred at 30 rpm.

### Production of Water-Absorbent Resin

### Production Example 1

### (Step of Preparing Aqueous Monomer Solution)

Into a polypropylene container having a capacity of 2 L, 422.0 parts by mass of acrylic acid, 173.9 parts by mass of a 48.5 mass% aqueous sodium hydroxide solution, 2.5 parts by mass of polyethylene glycol diacrylate (average molecular weight: 523), 1.3 parts by mass of a 2.0 mass% aqueous diethylenetriamine pentaacetic acid/trisodium solution, 0.2 parts by mass of polyethylene glycol 600 (mass-average molecular weight: 600, available from FUJIFILM Wako Pure Chemical Corporation), and 403.1 parts by mass of deionized water were charged and mixed to prepare an aqueous monomer solution (1'). The liquid temperature of the aqueous monomer solution (1') exceeded 40°C because of the heat of neutralization and the heat of dissolution generated in the mixing process.

### Polymerization Step

Then, the aqueous monomer solution (1') was cooled with stirring, and when the liquid temperature reached 40°C, 178.7 parts by mass of a 48.5 mass% aqueous sodium hydroxide solution adjusted to 40°C was charged into the aqueous monomer solution (1') over about 20 seconds in an atmosphere open state, and mixed (neutralization in the second stage was started). A aqueous monomer solution (1) was thus prepared. At this time, the liquid temperature of the aqueous monomer solution (1) increased to about 78°C because of the heat of neutralization and the heat of dissolution generated in the mixing process. In addition, immediately after the aqueous monomer solution (1') was started to be mixed with the aqueous sodium hydroxide solution, a precipitate was observed, but the aqueous monomer solution gradually dissolved, and the prepared aqueous monomer solution (1) became a transparent homogeneous solution.

Then, under stirring, nitrogen gas was introduced into the aqueous monomer solution (1) for 5 seconds under the conditions of a pressure of 0.1 MPa and a flow rate of 0.1 L/min using a gas filtration tube (available from TOP CORPORATION, particle number #4). Subsequently, 18.4 parts by mass of a 4.5 mass% aqueous sodium persulfate solution was added to the aqueous monomer solution (1) into which nitrogen gas was introduced. Thereafter, the aqueous monomer solution (1) was immediately poured into a tray-shaped container (bottom surface 340 mm × 340 mm, height 25 mm, inner surface: Teflon (trade name) coating) made of stainless steel in an atmosphere open state. The time from the start of the second stage neutralization to the pouring of the aqueous monomer solution (1) into the tray-shaped container was 65 seconds. The tray-shaped container was heated in advance to a surface temperature of 50°C using a hot plate (NEO HOTPLATE HI-1000, available from Iuchi Seieido Co., Ltd.) before the aqueous monomer solution (1) was poured into the tray-shaped container.

The aqueous monomer solution (1) was poured into the tray-shaped container, and then a polymerization reaction was started within 1 minute. In the polymerization reaction, the polymerization of the aqueous monomer solution (1) proceeded while expanding and foaming in all directions while generating water vapor. Thereafter, the obtained polymer shrank to a size slightly larger than the bottom surface of the tray-shaped container. 2 minutes after the start of the polymerization reaction, a hydrogel (1), which was the obtained polymer, was taken out from the tray-shaped container. The series of operations was performed in an atmosphere open state.

### Gel-Grinding Step

Then, the hydrogel (1) obtained in the polymerization reaction was cut so that the mass per one piece was about 60 g, and then subjected to gel grinding using a meat chopper (HL-G22SN, plate hole diameter 6.0 mm/available from REMACOM CO., LTD.) to obtain a particulate hydrogel (1). The input amount of the hydrogel (1) was about 360 g/min, and in parallel with the input of the hydrogel (1), gel grinding was performed while deionized water adjusted to 90°C was added to the meat chopper at 50 g/min. The particulate hydrogel (1) had a D50 (mass-average particle diameter) of 390 µm.

### Drying Step

Then, the particulate hydrogel (1) was spread and placed on a wire mesh having a mesh size of 300 µm, and placed in a hot-air dryer. Thereafter, the particulate hydrogel (1) was dried by passing hot air at 190°C for 30 minutes to obtain a dried polymer (1). There was no undried product in the dried polymer (1).

### Pulverizing Step/Classification Step

Then, the dried polymer (1) was charged into a roll mill (WML type roll pulverizing apparatus, available from Inokuchi Giken Co., Ltd.), ground, and then classified using two types of JIS standard sieves having mesh sizes of 710 µm and 150 µm. Through this operation, a water-absorbent resin (1) before surface crosslinking in an irregularly crushed shape, which passed through the sieve with a mesh size of 710 µm and remained on the sieve with a mesh size of 150 µm, was obtained.

### Surface Crosslinking Step

Then, to 100 parts by mass of the water-absorbent resin (1) before surface crosslinking, an aqueous surface crosslinking agent solution composed of 0.2 parts by mass of 1,6-hexanediol, 0.4 parts by mass of triethylene glycol, and 3.0 parts by mass of deionized water was added by spraying and mixed uniformly. Thereafter, the obtained mixture was heat-treated at 210°C for 40 minutes to perform surface crosslinking. Then, the mixture after the heat treatment was classified using two types of JIS standard sieves having mesh sizes of 710 µm and 150 µm, respectively. Through this operation, a water-absorbent resin (1) after surface crosslinking, which passed through the sieve with a mesh size of 710 µm and remained on the sieve with a mesh size of 150 µm, was obtained.

### Production Example 2

A water-absorbent resin (2) was produced under the following production conditions with reference to Reference Example 1 described in JP 4926474 B.

### Step of Preparing Aqueous Monomer Solution

To 5500 parts by mass of an aqueous sodium acrylate solution having a neutralization percentage of 75 mol% (monomer concentration: 38 mass%), 5.9 parts by mass of polyethylene glycol diacrylate (average molecular weight: 523) was added and mixed to adjust an aqueous monomer solution (2).

### Polymerization and Gel-Grinding Step

Next, the aqueous monomer solution (2) was degassed for 30 minutes under a nitrogen gas atmosphere.

Subsequently, the aqueous monomer solution (2) was supplied to a reactor formed by attaching a lid to a jacketed double-arm kneader made of stainless steel having an inner volume of 10 L and two sigma-shaped blades, and the system was purged with nitrogen gas while keeping the aqueous monomer solution (2) at 30°C. Subsequently, while stirring the aqueous monomer solution (2), 2.46 parts by mass of sodium persulfate and 0.10 parts by mass of L-ascorbic acid were added. Polymerization started after about 1 minute. Then, polymerization was performed at 30°C to 90°C, and 60 minutes after the polymerization was started, a particulate hydrogel (2) was taken out.

The obtained particulate hydrogel (2) was subdivided into pieces having a particle diameter of about 5 mm.

### Drying Step

Then, the particulate hydrogel (2) was spread and placed on a wire mesh with a mesh size of 300 µm, and placed in a hot-air dryer. Thereafter, the particulate hydrogel (2) was dried by passing hot air at 150°C for 90 minutes, whereby a dried polymer (2) was obtained. There was no undried product in the dried polymer (2).

### Pulverizing Step/Classification Step

Next, the dried polymer (2) was charged into a roll mill (WML type roll pulverizing apparatus, available from Inokuchi Giken Co., Ltd.), pulverized, and then classified using two types of JIS standard sieves with mesh sizes of 850 µm and 150 µm. Through this operation, a water-absorbent resin (2) before surface crosslinking in an irregularly crushed shape, which passed through the sieve with a mesh size of 850 µm and remained on the sieve with a mesh size of 150 µm, was obtained.

### Surface Crosslinking Step

Next, to 100 parts by mass of the water-absorbent resin (2) before surface crosslinking, an aqueous surface crosslinking agent solution composed of 0.03 parts by mass of ethylene glycol glycidyl ether, 0.5 parts by mass of propylene glycol, 0.3 parts by mass of 1,4-butanediol, and 3.0 parts by mass of water was sprayed and mixed uniformly. Thereafter, the obtained mixture was heat-treated at 200°C for 45 minutes to perform surface crosslinking. Then, the mixture after the heat treatment was classified using two types of JIS standard sieves with mesh sizes of 850 µm and 150 µm, respectively. Through this operation, a water-absorbent resin (2) after surface crosslinking, which passed through the sieve with a mesh size of 850 µm and remained on the sieve with a mesh size of 150 µm, was obtained.

### Production Example 3

### Step of Preparing Aqueous Monomer Solution

Into a polypropylene container having a capacity of 2 L, 422.0 parts by mass of acrylic acid, 173.9 parts by mass of a 48.5 mass% aqueous sodium hydroxide solution, 2.3 parts by mass of polyethylene glycol diacrylate (average molecular weight: 523), 1.3 parts by mass of a 2.0 mass% aqueous diethylenetriamine pentaacetic acid/trisodium solution, 0.2 parts by mass of polyethylene glycol 600 (mass-average molecular weight: 600, available from FUJIFILM Wako Pure Chemical Corporation), and 403.2 parts by mass of deionized water were charged and mixed to prepare an aqueous monomer solution (3'). The liquid temperature of the aqueous monomer solution (3') exceeded 40°C because of the heat of neutralization and the heat of dissolution generated in the mixing process.

### Polymerization Step

Then, the aqueous monomer solution (3') was cooled with stirring, and when the liquid temperature reached 40°C, 178.7 parts by mass of a 48.5 mass% aqueous sodium hydroxide solution adjusted to 40°C was charged into the aqueous monomer solution (3') over about 20 seconds in an atmosphere open state, and mixed (neutralization in the second stage was started). An aqueous monomer solution (3) was thus prepared. At this time, the liquid temperature of the aqueous monomer solution (1) increased to about 78°C because of the heat of neutralization and the heat of dissolution generated in the mixing process. In addition, immediately after the aqueous monomer solution (3') was started to be mixed with the sodium hydroxide aqueous solution, a precipitate was observed, but the aqueous monomer solution gradually dissolved, and the prepared aqueous monomer solution (3) became a transparent homogeneous solution.

Then, under stirring, nitrogen gas was introduced into the aqueous monomer solution (3) for 5 seconds under the conditions of a pressure of 0.1 MPa and a flow rate of 0.1 L/min using a gas filtration tube (available from TOP CORPORATION, particle number #4). Subsequently, 18.4 parts by mass of a 4.5 mass% aqueous sodium persulfate solution was added to the aqueous monomer solution (3) into which nitrogen gas was introduced. Thereafter, the aqueous monomer solution (3) was immediately poured into a tray-shaped container (bottom surface 340 mm × 340 mm, height 25 mm, inner surface: Teflon (trade name) coating) made of stainless steel in an atmosphere open state. The time from the start of the second stage neutralization to the pouring of the aqueous monomer solution (3) into the tray-shaped container was 67 seconds. The tray-shaped container was heated in advance to a surface temperature of 50°C using a hot plate (NEO HOTPLATE HI-1000, available from Iuchi Seieido Co., Ltd.) before the aqueous monomer solution (3) was poured into the tray-shaped container.

The aqueous monomer solution (3) was poured into the tray-shaped container, and then a polymerization reaction was started within 1 minute. In the polymerization reaction, the polymerization of the aqueous monomer solution (3) proceeded while expanding and foaming in all directions while generating water vapor. Thereafter, the obtained polymer shrank to a size slightly larger than the bottom surface of the tray-shaped container. 2 minutes after the start of the polymerization reaction, a hydrogel (3), which was the obtained polymer, was taken out from the tray-shaped container. The series of operations was performed in an atmosphere open state.

### Gel-Grinding Step

Then, the hydrogel (3) obtained in the polymerization reaction was cut so that the mass per piece was about 60 g, and then subjected to gel grinding using a meat chopper (HL-G22SN, plate hole diameter 8.0 mm/available from REMACOM CO., LTD.), whereby a particulate hydrogel (3) was obtained. The input amount of the hydrogel (3) was about 360 g/min, and in parallel with the input of the hydrogel (3), gel grinding was performed while deionized water adjusted to 90°C was added to the meat chopper at 50 g/min. The particulate hydrogel (3) had a D50 (mass-average particle diameter) of 700 µm.

### Drying Step

Then, the particulate hydrogel (3) was spread and placed on a wire mesh with a mesh size of 300 µm, and placed in a hot-air dryer. Thereafter, the particulate hydrogel (3) was dried by passing hot air at 190°C for 30 minutes, whereby a dried polymer (3) was obtained. There was no undried product in the dried polymer (3).

### Pulverizing Step/Classification Step

Next, the dried polymer (3) was charged into a roll mill (WML type roll pulverizing apparatus, available from Inokuchi Giken Co., Ltd.), pulverized, and then classified using two types of JIS standard sieves with mesh sizes of 850 µm and 150 µm. Through this operation, a water-absorbent resin (3) before surface crosslinking in an irregularly crushed shape, which passed through the sieve with a mesh size of 850 µm and remained on the sieve with a mesh size of 150 µm, was obtained.

### Surface Crosslinking Step

Next, an aqueous surface crosslinking agent solution (which contains a flowability improver. Concentration of the flowability improver in the aqueous solution: 0.03 mass%, pH of the aqueous solution: 4.7) composed of 3.0 parts by mass of deionized water and 0.01 parts by mass of an aqueous solution (amount of the flowability improver added: 10 ppm) containing 0.2 parts by mass of 1,6 hexanediol, 0.4 parts by mass of triethylene glycol, and 10 mass% of polyoxyethylene (20) sorbitan monostearate (flowability improver, product name: RHEODOL TW-S120 V, manufacturer: Kao Corporation) were uniformly mixed with 100 parts by mass of the water-absorbent resin (3) before surface crosslinking using a straight tube having an inside diameter of 0.2 mm (average droplet diameter: 0.4 mm (400 µm)). The aqueous surface crosslinking agent solution was added using a three-one motor equipped with an anchor type stirring blade (diameter: 57 mm, height: 70 mm) made from a metal rod having a diameter of 3 mm while stirring the water-absorbent resin (3) at a rotational speed of 450 rpm (peripheral speed: 1.34 m/s). After stirring (mixing power index: 33500) for 10 seconds from the start of addition, the obtained mixture was subjected to surface crosslinking through heat treatment at 210°C for 40 minutes. Then, the mixture after the heat treatment was classified using two types of JIS standard sieves with mesh sizes of 850 µm and 150 µm, respectively. Through this operation, a water-absorbent resin (3) after surface crosslinking, which passed through the sieve with a mesh size of 850 µm and remained on the sieve with a mesh size of 150 µm, was obtained.

### Production Example 4

A water-absorbent resin (4) after surface crosslinking was obtained by performing the same operations as those of Production Example 3 except that in the surface crosslinking step in Production Example 3, an aqueous surface crosslinking agent solution (which contains a flowability improver. Concentration of the flowability improver in the aqueous solution: 0.08 mass%, pH of the aqueous solution: 4.7), in which the amount of the aqueous solution containing 10 mass% of polyoxyethylene (20) sorbitan monostearate (flowability improver, product name: RHEODOL TW-S120 V, manufacturer: Kao Corporation) was changed to 0.03 parts by mass (amount of the flowability improver added: 30 ppm), was used, and during the addition of the aqueous surface crosslinking agent solution, the stirring time was changed to 30 seconds (mixing power index: 100500) from the start of the addition.

### Example 1

The water-absorbent resin (1) after surface crosslinking obtained in Production Example 1 in an amount of 750 g was heated to 60°C and then charged in a Loedige mixer heated to 60°C (type: M5R, manufacturer: Loedige Corporation; stirring blade diameter: 0.19 m). Subsequently, while stirring the water-absorbent resin (1) after surface crosslinking at a rotational speed of 340 rpm (peripheral speed: 3.38 m/s), an aqueous solution (1) (amount of the flowability improver added: 10 ppm, concentration of the flowability improver in the aqueous solution: 0.20 mass%, pH of the aqueous solution: 6.9) having a liquid temperature of 25°C composed of 0.5 parts by mass of deionized water and 0.01 parts by mass of a 10 mass% aqueous polyoxyethylene (20) sorbitan monostearate solution (product name: RHEODOL TW-S120 V, manufacturer: Kao Corporation) was added by spraying relative to 100 parts by mass of the water-absorbent resin (1) after surface crosslinking using an air-conical nozzle (specification: 1/4M-K008, manufacturer: H. IKEUCHI & CO., LTD., average droplet diameter: 100 µm). After stirring (mixing power index: 2028000) for 60 seconds from the start of addition, the mixture was discharged from the Loedige mixer. The obtained mixture was stacked in the form of a layer of 5 cm, and allowed to stand for 30 minutes in a ventilation type hot-air dryer in which the atmospheric temperature was set to 60°C to be cured. The mixture subjected to the curing treatment was passed through a wire mesh with a mesh size of 850 µm, whereby a water-absorbing agent composition (1) was obtained.

### Comparative Example 1

A comparative water-absorbing agent composition (1) was obtained by performing the same operations as those of Example 1 except that the air-conical nozzle used for adding the aqueous solution (1) was changed to a syringe (volume: 12 ml, manufacturer: Terumo Corporation, average droplet diameter: 4 mm) in Example 1. The mixing power index in Comparative Example 1 was 50700.

### Example 2

A water-absorbing agent composition (2) was obtained by performing the same operations as those of Example 1 except that the aqueous solution (1) was changed to an aqueous solution (2) (amount of the flowability improver added: 50 ppm, the concentration of the flowability improver in the aqueous solution: 0.91 mass%, pH of the aqueous solution: 6.9) having a liquid temperature of 25°C composed of 0.5 parts by mass of deionized water and 0.05 parts by mass of a 10 mass% polyoxyethylene (20) sorbitan monostearate aqueous solution (product name: RHEODOL TWS120V, manufacturer: Kao Corporation) in Example 1.

### Comparative Example 2

A comparative water-absorbing agent composition (2) was obtained by performing the same operations as those of Comparative Example 1 except that the water-absorbent resin after surface crosslinking used in Comparative Example 1 was changed to the water-absorbent resin (2) after surface crosslinking obtained in Production Example 2.

### Example 3

In a plastic container having a capacity of 200 mL (inner diameter: 70 mm, depth: 140 mm), 50 g of the water-absorbent resin (3) after surface crosslinking obtained in Production Example 3 was put after the temperature was adjusted to 60°C. Subsequently, while stirring the water-absorbent resin (3) after surface crosslinking in the plastic container at a rotational speed of 450 rpm (peripheral speed: 1.34 m/s) using a three-one motor equipped with an anchor type stirring blade (diameter: 57 mm, height: 70 mm) made from a metal rod having a diameter of 3 mm, 0.50 parts by mass (amount of the flowability improver added: 10 ppm) of an aqueous solution (3) (concentration of the flowability improver in aqueous solution: 0.20 mass%, pH of the aqueous solution: 6.9) having a liquid temperature of 25°C containing 0.20 mass% of polyoxyethylene (20) sorbitan monostearate (product name: RHEODOL TW-S120 V, manufacturer: Kao Corporation) was added relative to 100 parts by mass of the water-absorbent resin (3) after surface crosslinking using a straight pipe having an inner diameter of 0.2 mm (average droplet diameter: 0.4 mm (400 µm)). After stirring (mixing power index: 100500) for 30 seconds from the start of addition, the mixture was discharged from the plastic container. The obtained mixture was stacked in the form of a layer of 5 cm, and allowed to stand for 30 minutes in a ventilation type hot-air dryer in which the atmospheric temperature was set to 60°C to be cured. The mixture subjected to the curing treatment was passed through a wire mesh with a mesh size of 850 µm, whereby a water-absorbing agent composition (3) was obtained.

### Comparative Example 3

A comparative water-absorbing agent composition (3) was obtained by performing the same operations as those of Example 3 except that the stirring time when the aqueous solution (3) was added to the water-absorbent resin (3) after surface crosslinking was changed to 3 seconds (mixing power index: 10050) in Example 3.

### Example 4

A water-absorbing agent composition (4) was obtained by performing the same operations as those of Example 3 except that the aqueous solution (3) was changed to an aqueous solution (4) (concentration of the flowability improver in the aqueous solution: 0.20 mass%, pH of the aqueous solution: 6.2) having a liquid temperature of 25°C containing 0.20 mass% of polyoxyethylene polyoxypropylene alkyl ether (product name: EMULGEN MS-110, manufacturer: Kao Corporation), and the amount of the aqueous solution added was changed to 1.00 parts by mass (amount of the flowability improver added: 20 ppm) in Example 3.

### Example 5

A water-absorbing agent composition (5) was obtained by performing the same operations as those of Example 4 except that the aqueous solution (4) was changed to an aqueous solution (5) (concentration of the flowability improver in the aqueous solution: 0.20 mass%, pH of the aqueous solution: 5.3) having a liquid temperature of 25°C containing 0.20 mass% of polyoxyethylene (6) lauryl ether (product name: EMULGEN 108, manufacturer: Kao Corporation) in Example 4.

### Example 6

A water-absorbing agent composition (6) was obtained by performing the same operations as those of Example 4 except that the aqueous solution (4) was changed to an aqueous solution (6) (concentration of the flowability improver in the aqueous solution: 0.20 mass%, pH of the aqueous solution: 5.6) having a liquid temperature of 25°C containing 0.20 mass% of polyoxyethylene (10) lauryl ether sodium acetate (product name: KAO AKIPO RLM-100NV, manufacturer: Kao Corporation) in Example 4.

### Example 7

A water-absorbing agent composition (7) was obtained by performing the same operations as those of Example 4 except that the aqueous solution (4) was changed to an aqueous solution (7) (concentration of the flowability improver in the aqueous solution: 0.20 mass%, pH of the aqueous solution: 6.2) having a liquid temperature of 25°C containing 0.20 mass% of polyoxyethylene (4.5) lauryl ether sodium acetate (product name: KAO AKIPO RLM-45NV, manufacturer: Kao Corporation) in Example 4.

### Example 8

A water-absorbing agent composition (8) was obtained by performing the same operations as those of Example 4 except that the aqueous solution (4) was changed to an aqueous solution (8) (concentration of the flowability improver in the aqueous solution: 0.20 mass%, pH of the aqueous solution: 6.0) having a liquid temperature of 25°C containing 0.20 mass% of coconut amine acetate (product name: Acetamine 24, manufacturer: Kao Corporation) in Example 4.

### Example 9

A water-absorbing agent composition (9) was obtained by performing the same operations as those of Example 4 except that the aqueous solution (4) was changed to an aqueous solution (9) (concentration of the flowability improver in the aqueous solution: 0.20 mass%, pH of the aqueous solution: 5.2) having a liquid temperature of 25°C containing 0.20 mass% of stearylamine acetate (product name: Acetamine 86, manufacturer: Kao Corporation) in Example 4.

### Example 10

A water-absorbing agent composition (10) was obtained by performing the same operations as those of Example 4 except that the aqueous solution (4) was changed to an aqueous solution (10) (concentration of the flowability improver in the aqueous solution: 0.20 mass%, pH of the aqueous solution: 7.4) having a liquid temperature of 25°C containing 0.20 mass% of lauryl dimethyl amine oxide (molecular weight: 229, product name: AMPHITOL 20N, manufacturer: Kao Corporation) in Example 4.

### Comparative Example 4

A comparative water-absorbing agent composition (4) was obtained by performing the same operations as those of Example 3 except that the aqueous solution (3) was changed to an aqueous solution (4') (concentration of the flowability improver in the aqueous solution: 0.20 mass%, pH of the aqueous solution: 6.7) having a liquid temperature of 25°C containing 0.20 mass% of glycerin (molecular weight: 92, manufacturer: FUJIFILM Wako Pure Chemical Corporation) in Example 3.

### Example 11

A water-absorbing agent composition (11) was obtained by performing the same operations as those of Example 3 except that the aqueous solution (3) was changed to an aqueous solution (11) (concentration of the flowability improver in the aqueous solution: 0.20 mass%, pH of the aqueous solution: 6.2) having a liquid temperature of 25°C containing 0.20 mass% of polyethylene glycol 20000 (mass-average molecular weight: 20000, manufacturer: FUJIFILM Wako Pure Chemical Corporation) in Example 3.

### Comparative Example 5

A comparative water-absorbing agent composition (5) was obtained by performing the same operations as those of Example 3 except that the aqueous solution (3) was changed to an aqueous solution (5') (concentration of the flowability improver in the aqueous solution: 0.20 mass%, pH of the aqueous solution: 5.7) having a liquid temperature of 25°C containing 0.20 mass% of polyethylene glycol 70000 (mass-average molecular weight: 70000, manufacturer: Wako Pure Chemical Industries, Ltd.) in Example 3.

### Comparative Example 6

A comparative water-absorbing agent composition (6) was obtained by performing the same operations as those of Example 3 except that the aqueous solution (3) was changed to an aqueous solution (6') (concentration of the flowability improver in the aqueous solution: 0.20 mass%, pH of the aqueous solution: 7.7) having a liquid temperature of 25°C containing 0.20 mass% of polyethylene glycol 500000 (mass-average molecular weight: 500000, manufacturer: FUJIFILM Wako Pure Chemical Corporation) in Example 3.

### Example 12

A water-absorbing agent composition (12) was obtained by performing the same operations as those of Example 3 except that the aqueous solution (3) was changed to an aqueous solution (12) (concentration of the flowability improver in the aqueous solution: 1.00 mass%, pH of the aqueous solution: 5.1) having a liquid temperature of 25°C containing 1.00 mass% of stearylamine acetate (product name: Acetamine 86, manufacturer: Kao Corporation), and the amount of the aqueous solution added was changed to 1.50 parts by mass (amount of flowability improver added: 150 ppm) in Example 3.

### Comparative Example 7

A comparative water-absorbing agent composition (7) was obtained according to the following procedure. The water-absorbent resin used was the water-absorbent resin (3) after surface crosslinking obtained in Production Example 3, and the stirring time was 30 seconds when an aqueous solution was added.

That is, in a plastic container having a capacity of 200 mL (inner diameter: 70 mm, depth: 140 mm), 40 g of the water-absorbent resin (3) after surface crosslinking obtained in Production Example 3 was put after the temperature was adjusted to 24°C. Subsequently, while stirring the water-absorbent resin (3) after surface crosslinking in the plastic container using a three-one motor equipped with an anchor type stirring blade (diameter: 57 mm, height: 70 mm) made from a metal rod having a diameter of 3 mm, an aqueous solution (7') (amount of the flowability improver added: 800 ppm, concentration of the flowability improver in the aqueous solution: 32 mass%, pH of the aqueous solution: 5.9) having a liquid temperature of 25°C composed of 0.17 parts by mass of deionized water and 0.08 parts by mass of polyethylene glycol 600 (mass-average molecular weight: 600, manufacturer: FUJIFILM Wako Pure Chemical Corporation) was added to 100 parts by mass of the water-absorbent resin (3) after surface crosslinking at a rotational speed of 450 rpm (peripheral speed: 1.34 m/s) using a straight pipe having an inner diameter of 0.5 mm (average droplet diameter: 1.0 mm). After stirring (mixing power index: 40200) for 30 seconds from the start of addition, the mixture was discharged from the plastic container. The obtained mixture was stacked in the form of a layer having a thickness of 5 cm, and allowed to stand for 30 minutes in a ventilation type hot-air dryer in which the atmospheric temperature was set to 60°C to be cured. The mixture subjected to the curing treatment was passed through a wire mesh with a mesh size of 850 µm, whereby a comparative water-absorbing agent composition (7) was obtained.

### Comparative Example 8

A comparative water-absorbing agent composition (8) was obtained according to the following procedure. The water-absorbent resin used was the water-absorbent resin (3) after surface crosslinking obtained in Production Example 3, and the stirring time was 30 seconds when an aqueous solution was added.

That is, the comparative water-absorbing agent composition (8) was obtained by performing the same operations as those of Comparative Example 7 except that the aqueous solution (7') in Comparative Example 7 was changed to a mixed solution (8') (amount of the flowability improver added: 250 ppm, concentration of the flowability improver in the mixed solution: 4.95 mass%) having a liquid temperature of 25°C composed of 0.48 parts by mass of isopropyl alcohol and 0.025 parts by mass of polyoxyethylene (20) sorbitan monostearate (product name: RHEODOL TW-S120 V, manufacturer: Kao Corporation).

### Comparative Example 9

A comparative water-absorbing agent composition (9) was obtained by performing the same operations as those of Example 3 except that the amount of the aqueous solution (3) added was changed to 12.5 parts by mass (amount of the flowability improver added: 250 ppm) in Example 3.

### Example 13

The water-absorbent resin (4) after surface crosslinking obtained in Production Example 4 was used as a water-absorbing agent composition (13).

The conditions at the time of adding and mixing the aqueous solution of the flowability improver in Examples and Comparative Examples are shown in Table 3. Various physical properties of the water-absorbent resin after surface crosslinking obtained in the Production Examples and the water-absorbing agent compositions obtained in Examples and Comparative Examples are shown in Table 4. Further, the particle size distributions of the water-absorbent resin after surface crosslinking obtained in each Production Example and the water-absorbing agent compositions obtained in Examples and Comparative Examples are shown in Table 5. The measurement of the particle size distribution of the water-absorbing agent composition in Table 5 was performed by sieving the water-absorbing agent composition for 5 minutes with a vibration classifier (IIDA SIEVE SHAKER, TYPE: ES-65 type (rotational speed: 60 Hz 230 rpm, number of impulses: 60 Hz 130 rpm), SER.No. 0501) using each sieve with a mesh size described in the table. Further, the particle size change percentages obtained by the feeding test for some of Examples and Comparative Examples are shown in Table 6. In Table 3 shown below, the item of "Classification" of the aqueous solution of the flowability improver indicates which one of (a) to (n) is the preferred form of the flowability improver described above. In Table 4 shown below, the unit of (1) SFC is [×10⁻⁷ cm³·sec/g], the item of (2) "150 ↓" represents the mass proportion (mass%) of particles having a particle diameter of less than 150 µm, the item of (3) "Kinetic friction coefficient" represents the kinetic friction coefficient of particles having a particle diameter of 300 µm or more and less than 600 µm in the water-absorbing agent composition, and the item of (4) "Reduction percentage" represents the reduction percentage (%) of the kinetic friction coefficient before and after addition of the aqueous solution of the flowability improver. In addition, in Tables 3 and 4, for each value such as the type of flowability improver and the mixing power index described in the upper row and the lower row, the upper row shows the value in the surface crosslinking step, and the lower row shows the value in the step of adding a flowability improver performed after the surface crosslinking step. The same applies to the type of the flowability improver. In Table 5 shown below, for example, the item of "on 850 µm" represents the mass proportion of the water-absorbing agent composition that has not passed through a sieve with a mesh size of 850 µm, and the item of "on 710 µm" represents the mass proportion of the water-absorbing agent composition that has passed through a sieve with a mesh size of 850 µm and has not passed through a sieve with a mesh size of 710 µm. The item of "thru 45 µm" represents the mass proportion of the water-absorbing agent composition that has passed through a sieve with a mesh size of 45 µm.

**Table 3-1**

| Table 3 | | | | | | | | | | |
|---|---|---|---|---|---|---|---|---|---|---|
| | Name | Aqueous solution of flowability improver | | | | | Amount of flowability improver added [ppm] | Peripheral speed [m/s] | Mixing time [s] | Mixing power index |
| | | Type of flowability improver | Classification | Average droplet diameter | Concentration [mass%] | pH [-] | | | | |
| Example 1 | Water-absorbing agent composition (1) | Polyoxyethylene (20) sorbitan monostearate | b (b-3) | 100 µm (0.1mm) | 0.20 | 6.9 | 10 | 3.38 | 60 | 2028000 |
| Comparative Example 1 | Comparative water-absorbing agent composition (1) | Polyoxyethylene (20) sorbitan monostearate | b (b-3) | 4 mm | 0.20 | 6.9 | 10 | 3.38 | 60 | 50700 |
| Example 2 | Water-absorbing agent composition (2) | Polyoxyethylene (20) sorbitan monostearate | b (b-3) | 100 µm (0.1mm) | 0.91 | 6.9 | 50 | 3.38 | 60 | 2028000 |
| Comparative Example 2 | Comparative water-absorbing agent composition (2) | Polyoxyethylene (20) sorbitan monostearate | b (b-3) | 4 mm | 0.20 | 6.9 | 10 | 3.38 | 60 | 50700 |
| Production Example 3 | Water-absorbent resin after surface crosslinking (3) | Polyoxyethylene (20) sorbitan monostearate | b (b-3) | 0.4 mm | 0.03 | 4.7 | 10 | 1.34 | 10 | 33500 |
| Example 3 | Water-absorbing agent composition (3) | Polyoxyethylene (20) sorbitan monostearate/polyoxyethylene (20) sorbitan monostearate | b (b-3) | 0.4 mm, 0.4 mm | 0.03, 0.20 | 4.7, 6.9 | 20 | 1.34, 1.34 | 10, 30 | 33500, 100500 |
| Comparative Example 3 | Comparative water-absorbing agent composition (3) | Polyoxyethylene (20) sorbitan monostearate/polyoxyethylene (20) sorbitan monostearate | b (b-3) | 0.4 mm, 0.4 mm | 0.03, 0.20 | 4.7, 6.9 | 20 | 1.34, 1.34 | 10, 3 | 33500, 10050 |
| Example 4 | Water-absorbing agent composition (4) | Polyoxyethylene (20) sorbitan monostearate/polyoxyethylene polyoxypropylene alkyl ether | b (b-3), (b-2) | 0.4 mm, 0.4 mm | 0.03, 0.20 | 4.7, 6.2 | 30 | 1.34, 1.34 | 10, 30 | 33500, 100500 |
| Example 5 | Water-absorbing agent composition (5) | Polyoxyethylene (20) sorbitan monostearate/polyoxyethylene (6) lauryl ether | b (b-3), (b-2) | 0.4 mm, 0.4 mm | 0.03, 0.20 | 4.7, 5.3 | 30 | 1.34, 1.34 | 10, 30 | 33500, 100500 |

**Table 3-2**

| Table 3 (continued) | | | | | | | | | | |
|---|---|---|---|---|---|---|---|---|---|---|
| | Name | Aqueous solution of flowability improver | | | | | Amount of flowability improver added [ppm] | Peripheral speed [m/s] | Mixing time [s] | Mixing power index |
| | | Type of flowability improver | Classification | Average droplet diameter | Concentration [mass%] | pH [-] | | | | |
| Example 6 | Water-absorbing agent composition (6) | Polyoxyethylene (20) sorbitan monostearate/polyoxyethylene (10) lauryl ether sodium acetate | b(b-3), m | 0.4 mm, 0.4 mm | 0.03, 0.20 | 4.7, 5.6 | 30 | 1.34, 1.34 | 10, 30 | 33500, 100500 |
| Example 7 | Water-absorbing agent composition (7) | Polyoxyethylene (20) sorbitan monostearate/polyoxyethylene (4.5) lauryl ether sodium acetate | b(b-3), m | 0.4 mm, 0.4 mm | 0.03, 0.20 | 4.7, 6.2 | 30 | 1.34, 1.34 | 10, 30 | 33500, 100500 |
| Example 8 | Water-absorbing agent composition (8) | Polyoxyethylene (20) sorbitan monostearate/coconut amine acetate | b(b-3), n | 0.4 mm, 0.4 mm | 0.03, 0.20 | 4.7, 6.0 | 30 | 1.34, 1.34 | 10, 30 | 33500, 100500 |
| Example 9 | Water-absorbing agent composition (9) | Polyoxyethylene (20) sorbitan monostearate/stearylamine acetate | b(b-3), n | 0.4 mm, 0.4 mm | 0.03, 0.20 | 4.7, 5.2 | 30 | 1.34, 1.34 | 10, 30 | 33500, 100500 |
| Example 10 | Water-absorbing agent composition (10) | Polyoxyethylene (20) sorbitan monostearate/lauryl dimethyl amine oxide | b(b-3), f | 0.4 mm, 0.4 mm | 0.03, 0.20 | 4.7, 7.4 | 30 | 1.34, 1.34 | 10, 30 | 33500, 100500 |
| Comparative Example 4 | Comparative water-absorbing agent composition (4) | Polyoxyethylene (20) sorbitan monostearate/glycerin | b(b-3), - | 0.4 mm, 0.4 mm | 0.03, 0.20 | 4.7, 6.7 | 20 | 1.34, 1.34 | 10, 30 | 33500, 100500 |
| Example 11 | Water-absorbing agent composition (11) | Polyoxyethylene (20) sorbitan monostearate/polyethylene glycol 20000 | b(b-3), a | 0.4 mm, 0.4 mm | 0.03, 0.20 | 4.7, 6.2 | 20 | 1.34, 1.34 | 10, 30 | 33500, 100500 |

**Table 3-3**

| Table 3 (continued) | | | | | | | | | | |
|---|---|---|---|---|---|---|---|---|---|---|
| | Name | Aqueous solution of flowability improver | | | | | Amount of flowability improver added [ppm] | Peripheral speed [m/s] | Mixing time [s] | Mixing power index |
| | | Type of flowability improver | Classification | Average droplet diameter | Concentration [mass%] | pH [-] | | | | |
| Comparative Example 5 | Comparative water-absorbing agent composition (5) | Polyoxyethylene (20) sorbitan monostearate/polyethylene glycol 70000 | b(b-3), | 0.4 mm, 0.4 mm | 0.03, 0.20 | 4.7, 5.7 | 20 | 1.34, 1.34 | 10, 30 | 33500, 100500 |
| Comparative Example 6 | Comparative water-absorbing agent composition (6) | Polyoxyethylene (20) sorbitan monostearate/polyethylene glycol 500000 | b(b-3), | 0.4 mm, 0.4 mm | 0.03, 0.20 | 4.7, 7.7 | 20 | 1.34, 1.34 | 10, 30 | 33500, 100500 |
| Example 12 | Water-absorbing agent composition (12) | Polyoxyethylene (20) sorbitan monostearate/stearylamine acetate | b(b-3), n | 0.4 mm, 0.4 mm | 0.03, 1.00 | 4.7, 5.1 | 160 | 1.34, 1.34 | 10, 30 | 33500, 100500 |
| Comparative Example 7 | Comparative water-absorbing agent composition (7) | Polyoxyethylene (20) sorbitan monostearate/polyethylene glycol 600 | b(b-3), | 0.4 mm, 1.0 mm | 0.03, 32.00 | 4.7, 5.9 | 810 | 1.34, 1.34 | 10, 30 | 33500, 40200 |
| Comparative Example 8 | Comparative water-absorbing agent composition (8) | Polyoxyethylene (20) sorbitan monostearate/polyoxyethylene (20) sorbitan monostearate | b(b-3) | 0.4 mm, 1.0 mm | 0.03, 4.95 | 4.7, | 260 | 1.34, 1.34 | 10, 30 | 33500, 40200 |
| Comparative Example 9 | Comparative water-absorbing agent composition (9) | Polyoxyethylene (20) sorbitan monostearate/polyoxyethylene (20) sorbitan monostearate | b(b-3) | 0.4 mm, 0.4 mm | 0.03, 0.20 | 4.7, 6.9 | 260 | 1.34, 1.34 | 10, 30 | 33500, 100500 |
| Example 13 | Water-absorbing agent composition (13) | Polyoxyethylene (20) sorbitan monostearate | b(b-3) | 0.4 mm | 0.08 | 4.7 | 30 | 1.34 | 30 | 100500 |

**Table 4-1**

| Table 4 | | | | | | | | | | | | | | | |
|---|---|---|---|---|---|---|---|---|---|---|---|---|---|---|---|
| | Name | Type of flowability improver | Amount of flowability improver added | Mixing *power* index | Physical properties of water-absorbing agent composition | | | | | | | | | | |
| | | | | | CRC | AAP | SFC | Vortex | FHA | Surface tension | Flow velocity | Specific surface area | 150↓²⁾ | Kinetic friction coefficient ³⁾ | Reduction percentage ⁴⁾ |
| | | | [ppm] | | [g/g] | [g/g] | [(1)] | [s] | [g/g] | [mN/m] | [g/s] | [m²/kg] | [mass%] | [-] | [%] |
| Production Examples 1 | Water-absorbent resin after surface crosslinking (1) | - | - | - | 29.0 | 26.1 | 29 | 31 | 26.1 | 71.2 | 10.7 | 41 | 0.7 | 0.97 | - |
| Examples 1 | Water-absorbing agent composition (1) | Polyoxyethylene (20) sorbitan monostearate | 10 | 2028000 | 29.1 | 26.0 | 29 | 32 | 26.2 | 70.3 | 10.8 | 41 | 0.5 | 0.75 | 23 |
| Comparative Example 1 | Comparative Water-absorbing agent composition (1) | Polyoxyethylene (20) sorbitan monostearate | 10 | 50700 | 28.9 | 26.1 | 29 | 31 | 26.4 | 70.0 | 10.8 | 41 | 0.5 | 0.89 | 8 |
| Examples 2 | Water-absorbing agent composition (2) | Polyoxyethylene (20) sorbitan monostearate | 50 | 2028000 | 29.4 | 26.0 | 31 | 31 | 25.8 | 61.0 | 11.2 | 41 | 0.6 | 0.69 | 29 |
| Production Examples 2 | Water-absorbent resin after surface crosslinking (2) | - | - | - | 33.6 | 23.4 | 2 | 64 | 24.3 | 72.0 | 10.5 | 22 | 1.7 | 0.88 | - |
| Comparative Example 2 | Comparative water-absorbing agent composition (2) | Polyoxyethylene (20) sorbitan monostearate | 10 | 50700 | 33.8 | 23.2 | 2 | 65 | 24.3 | 71.1 | 10.8 | 22 | 1.1 | 0.62 | 30 |
| Production Examples 3 | Water-absorbent resin after surface crosslinking (3) | Polyoxyethylene (20) sorbitan monostearate | 10 | 33500 | 29.7 | 26.1 | 26 | 40 | 26.2 | 71.4 | 10.5 | 27 | 0.4 | 0.88 | - |
| Examples 3 | Water-absorbing agent composition (3) | Polyoxyethylene (20) sorbitan monostearate/ polyoxyethylene (20) sorbitan monostearate | 20 | 33500, 100500 | 29.3 | 25.6 | 26 | 41 | 25.3 | 70.5 | 10.6 | 27 | 0.5 | 0.78 | 12 |
| Comparative Example 3 | Comparative water-absorbing agent composition (3) | Polyoxyethylene (20) sorbitan monostearate/ polyoxyethylene (20) sorbitan monostearate | 20 | 33500, 10050 | 28.9 | 25.6 | 26 | 41 | 25.6 | 70.6 | 10.5 | 27 | 0.2 | 0.82 | 7 |
| Examples 4 | Water-absorbing agent composition (4) | Polyoxyethylene (20) sorbitan monostearate/ polyoxyethylene polyoxypropylene alkyl ether | 30 | 33500, 100500 | 28.9 | 25.9 | 27 | 39 | 25.5 | 63.2 | 10.2 | 27 | 0.5 | 0.76 | 14 |

**Table 4-2**

| Table 4 (continued) | | | | | | | | | | | | | | | |
|---|---|---|---|---|---|---|---|---|---|---|---|---|---|---|---|
| | Name | Type of flowability improver | Amount of flowability improver added | Mixing power index | Physical properties of water-absorbing agent composition | | | | | | | | | | |
| | | | | | CRC | AAP | SFC | Vortex | FHA | Surface tension | Flow velocity | Specific surface area | 150↓ ²⁾ | Kinetic friction coefficient ³⁾ | Reduction percentage ⁴⁾ |
| | | | [ppm] | | [g/g] | [g/g] | [(1)] | [s] | [g/g] | [mN/m] | [g/s] | [m²/kg] | [mass%] | [-] | [%] |
| Examples 5 | Water-absorbing agent composition (5) | Polyoxyethylene (20) sorbitan monostearate/ polyoxyethylene (6) lauryl ether | 30 | 33500, 100500 | 28.9 | 26.0 | 25 | 39 | 25.8 | 65.1 | 10.5 | 27 | 0.6 | 0.69 | 22 |
| Examples 6 | Water-absorbing agent composition (6) | Polyoxyethylene (20) sorbitan monostearate/ polyoxyethylene (10) lauryl ether sodium acetate | 30 | 33500, 100500 | 29.3 | 25.9 | 26 | 41 | 25.7 | 66.5 | 10.4 | 27 | 0.5 | 0.79 | 10 |
| Examples 7 | Water-absorbing agent composition (7) | Polyoxyethylene (20) sorbitan monostearate/ polyoxyethylene (4.5) lauryl ether sodium acetate | 30 | 33500, 100500 | 29.1 | 25.9 | 22 | 39 | 25.8 | 67.0 | 10.3 | 27 | 0.5 | 0.76 | 14 |
| Examples 8 | Water-absorbing agent composition (8) | Polyoxyethylene (20) sorbitan monostearate/coconut amine acetate | 30 | 33500, 100500 | 28.6 | 25.8 | 26 | 39 | 25.7 | 69.7 | 10.2 | 27 | 0.5 | 0.78 | 12 |
| Examples 9 | Water-absorbing agent composition (9) | Polyoxyethylene (20) sorbitan monostearate/ stearylamine acetate | 30 | 33500, 100500 | 29.0 | 26.0 | 24 | 39 | 25.8 | 71.6 | 10.2 | 27 | 0.5 | 0.74 | 17 |
| Examples 10 | Water-absorbing agent composition (10) | Polyoxyethylene (20) sorbitan monostearate/ lauryl dimethyl amine oxide | 30 | 33500, 100500 | 28.7 | 25.0 | 25 | 41 | 25.9 | 68.8 | 10.0 | 27 | 0.6 | 0.75 | 15 |
| Comparative Example 4 | Comparative water-absorbing agent composition (4) | Polyoxyethylene (20) sorbitan monostearate/glycerin | 20 | 33500, 100500 | 28.4 | 25.4 | 25 | 42 | 25.5 | 71.0 | 10.4 | 27 | 0.6 | 0.86 | 2 |

**Table 4-3**

| Table 4 (continued) | | | | | | | | | | | | | | | |
|---|---|---|---|---|---|---|---|---|---|---|---|---|---|---|---|
| | Name | Type of flowability improver | Amount of flowability improver added | Mixing power index | Physical properties of water-absorbing agent composition | | | | | | | | | | |
| | | | | | CRC | AAP | SFC | Vortex | FHA | Surface tension | Flow velocity | Specific surface area | 150↓ ²⁾ | Kinetic friction coefficient ³⁾ | Reduction percentage ⁴⁾ |
| | | | [ppm] | | [g/g] | [g/g] | [(1)] | [s] | [g/g] | [mN/m] | [g/s] | [m²/kg] | [mass%] | [-] | [%] |
| Examples 11 | Water-absorbing agent composition (11) | Polyoxyethylene (20) sorbitan monostearate/ polyethylene glycol 20000 | 20 | 33500, 100500 | 29.0 | 25.6 | 26 | 40 | 25.8 | 69.4 | 10.5 | 27 | 0.5 | 0.72 | 18 |
| Comparative Example 5 | Comparative water-absorbing agent composition (5) | Polyoxyethylene (20) sorbitan monostearate/ polyethylene glycol 70000 | 20 | 33500, 100500 | 28.3 | 25.5 | 26 | 39 | 25.9 | 69.5 | 10.1 | 27 | 0.6 | 0.84 | 5 |
| Comparative Example 6 | Comparative water-absorbing agent composition (6) | Polyoxyethylene (20) sorbitan monostearate/ polyethylene glycol 500000 | 20 | 33500, 100500 | 29.0 | 25.5 | 24 | 40 | 26.0 | 70.1 | 10.2 | 27 | 0.6 | 0.89 | -1 |
| Examples 12 | Water-absorbing agent composition (12) | Polyoxyethylene (20) sorbitan monostearate/ stearylamine acetate | 160 | 33500, 100500 | 28.5 | 25.2 | 22 | 38 | 25.4 | 70.7 | 10.2 | 27 | 0.6 | 0.74 | 16 |
| Comparative Example 7 | Comparative water-absorbing agent composition (7) | Polyoxyethylene (20) sorbitan monostearate/ polyethylene glycol 600 | 810 | 33500, 40200 | 29.1 | 26.0 | 25 | 40 | 25.9 | 70.0 | 9.7 | 27 | 0.7 | 0.76 | 14 |
| Comparative Example 8 | Comparative water-absorbing agent composition (8) | Polyoxyethylene (20) sorbitan monostearate/ polyoxyethylene (20) sorbitan monostearate | 260 | 33500, 40200 | 29.3 | 25.9 | 25 | 39 | 25.7 | 54.8 | 10.3 | 27 | 0.6 | 0.75 | 15 |
| Comparative Example 9 | Comparative water-absorbing agent composition (9) | Polyoxyethylene (20) sorbitan monostearate/ polyoxyethylene (20) sorbitan monostearate | 260 | 33500, 100500 | 26.4 | 23.6 | 27 | 39 | 22.0 | 54.5 | 9.8 | 27 | 0.2 | 0.63 | 28 |
| Examples 13 | Water-absorbing agent composition (13) | Polyoxyethylene (20) sorbitan monostearate | 30 | 100500 | 29.8 | 25.9 | 25 | 41 | 26.0 | 70.6 | 10.7 | 27 | 0.4 | 0.78 | - |

### Table 5

**Table 5**

| | Name | on 850µm | on 710 µm | on 600 µm | on 300 µm | on 150 µm | on 45 µm | thru 45 µm | D50 | σζ |
|---|---|---|---|---|---|---|---|---|---|---|
| | | [mass%] | [mass%] | [mass%] | [mass%] | [mass%] | [mass%] | [mass%] | [µm] | [-] |
| Production Example 1 | Water-absorbent resin after surface crosslinking (1) | 0.0 | 0.1 | 10.1 | 58.0 | 31.2 | 0.7 | 0.0 | 386 | 0.40 |
| Example 1 | Water-absorbing agent composition (1) | 0.0 | 0.1 | 10.5 | 60.2 | 28.8 | 0.4 | 0.0 | 407 | 0.39 |
| Comparative Example 1 | Comparative water-absorbing agent composition (1) | 0.0 | 0.1 | 11.7 | 60.5 | 27.3 | 0.4 | 0.0 | 417 | 0.39 |
| Example 2 | Water-absorbing agent composition (2) | 0.1 | 0.1 | 8.1 | 59.9 | 31.3 | 0.6 | 0.0 | 387 | 0.39 |
| Production Example 2 | Water-absorbent resin after surface crosslinking (2) | 0.0 | 1.0 | 14.3 | 52.6 | 30.5 | 1.7 | 0.0 | 406 | 0.45 |
| Comparative Example 2 | Comparative water-absorbing agent composition (2) | 0.0 | 1.8 | 15.0 | 57.7 | 24.3 | 1.1 | 0.0 | 436 | 0.42 |
| Production Example 3 | Water-absorbent resin after surface crosslinking (3) | 0.0 | 0.2 | 8.2 | 65.3 | 26.0 | 0.4 | 0.0 | 413 | 0.37 |
| Example 3 | Water-absorbing agent composition (3) | 0.1 | 0.2 | 6.7 | 70.2 | 22.2 | 0.5 | 0.0 | 438 | 0.36 |
| Comparative Example 3 | Comparative water-absorbing agent composition (3) | 0.0 | 0.2 | 6.4 | 72.4 | 20.7 | 0.2 | 0.0 | 450 | 0.34 |
| Example 4 | Water-absorbing agent composition (4) | 0.0 | 0.1 | 5.1 | 67.5 | 26.7 | 0.5 | 0.0 | 397 | *0.36* |
| Example 5 | Water-absorbing agent composition (5) | 0.0 | 0.1 | 4.6 | 69.1 | 25.6 | 0.6 | 0.0 | 401 | *0.35* |
| Example 6 | Water-absorbing agent composition (6) | 0.0 | 0.1 | 4.2 | 66.3 | 28.9 | 0.5 | 0.0 | 390 | 0.36 |
| Example 7 | Water-absorbing agent composition (7) | 0.0 | 0.1 | 4.9 | *66.8* | 27.8 | 0.5 | 0.0 | *395* | *0.36* |
| Example 8 | Water-absorbing agent composition (8) | 0.0 | 0.5 | 6.8 | 68.1 | 24.1 | 0.5 | 0.0 | 414 | *0.36* |
| Example 9 | Water-absorbing agent composition (9) | 0.0 | 0.3 | 5.1 | 67.6 | 26.4 | 0.5 | 0.0 | 403 | 0.36 |
| Example 10 | Water-absorbing agent composition (10) | 0.1 | 0.2 | 6.1 | 68.4 | 24.7 | 0.6 | 0.0 | 423 | *0.36* |
| Comparative Example 4 | Comparative water-absorbing agent composition (4) | 0.1 | 0.2 | 9.3 | 67.8 | 21.9 | 0.5 | 0.1 | 438 | 0.37 |
| Example 11 | Water-absorbing agent composition (11) | 0.1 | 0.2 | 6.6 | 71.2 | 21.5 | 0.5 | 0.0 | 439 | 0.35 |
| Comparative Example 5 | Comparative water-absorbing agent composition (5) | 0.1 | 0.1 | 7.6 | 68.5 | 23.2 | 0.6 | 0.0 | 436 | 0.36 |
| Comparative Example 6 | Comparative water-absorbing agent composition (6) | 0.1 | 0.2 | 7.0 | 67.3 | 24.8 | 0.6 | 0.0 | 425 | 0.37 |
| Example 12 | Water-absorbing agent composition (12) | 0.1 | 0.1 | 5.0 | 68.9 | 25.3 | 0.6 | 0.0 | 417 | 0.36 |
| Comparative Example 7 | Comparative water-absorbing agent composition (7) | 0.1 | 0.1 | 5.2 | 67.6 | 26.4 | 0.7 | 0.0 | 416 | 0.37 |
| Comparative Example 8 | Comparative water-absorbing agent composition (8) | 0.1 | 0.1 | 4.1 | 71.4 | 23.8 | 0.6 | 0.0 | 419 | 0.35 |
| Comparative Example 9 | Comparative water-absorbing agent composition (9) | 0.1 | 1.6 | 14.0 | 64.3 | 19.9 | 0.2 | 0.0 | 457 | 0.37 |
| Example 13 | Water-absorbing agent composition (13) | 0.0 | 0.1 | 8.0 | 63.1 | 28.4 | 0.4 | 0.0 | 400 | 0.38 |

**Table 6**

| | Name | Particles having particle diameter of 600 µm or more | | | Particles having particle diameter of 300 µm or more and less than 600 µm | | | Particles having particle diameter of less than 300 µm | | |
|---|---|---|---|---|---|---|---|---|---|---|
| | | Before test ¹ | After test ² | Particle size change percentage | Before test ¹ | After test ² | Particle size change percentage | Before test 1 | After test 2 | Particle size change percentage |
| | | [mass%] | [mass%] | [%] | [mass%] | [mass%] | [%] | [mass%] | [mass%] | [%] |
| Example 1 | Water-absorbing agent composition (1) | 11 | 11 | 0 | 60 | 59 | 1 | 29 | 30 | -1 |
| Example 5 | Water-absorbing agent composition (5) | 5 | 4 | 1 | 69 | 69 | 0 | 26 | 27 | -1 |
| Comparative Example 1 | Comparative water-absorbing agent composition (1) | 12 | 7 | 5 | 60 | 61 | -1 | 28 | 32 | -4 |
| Comparative Example 2 | Comparative water-absorbing agent composition (2) | 17 | 17 | 0 | 58 | 57 | 1 | 25 | 26 | -1 |

| | | | | | | | | | | |
|---|---|---|---|---|---|---|---|---|---|---|
| 1) Before test: mass proportion before feeding test 2) After test: mass proportion after feeding test | | | | | | | | | | |

According to Table 4, in the water-absorbing agent compositions (Examples 1 to 13) obtained by the production method according to the present invention, the kinetic friction coefficient of the particles having a particle diameter of 300 µm or more and less than 600 µm was 0.80 or less. In Examples 1 to 12, the reduction percentage of the kinetic friction coefficient was as large as 10% or more.

Next, referring to Table 6, in Examples 1 and 5, the result was that the particle size change percentage was small (the absolute value of the particle size change percentage was small) in any particle diameter. From such results, it is found that in these Examples, the flowability of the entire particles is uniformly improved, and the particle size segregation during the feeding (during transportation) is suppressed. Then, considering the results in Table 6 together with the kinetic friction coefficient (kinetic friction coefficient of particles having a particle diameter of 300 µm or more and less than 600 µm) and the reduction percentage thereof in Table 4, it is considered that in Examples 1 and 5 in which the kinetic friction coefficient of particles having a particle diameter of 300 µm or more and less than 600 µm, which are particles having a large content proportion, was sufficiently reduced, the kinetic friction coefficient of the entire particles was reduced, and as a result, the particle size change percentage was reduced in any particle diameter as shown in Table 6. Thus, in Examples other than Example 1 or 5, the kinetic friction coefficient (the kinetic friction coefficient of particles having a particle diameter of 300 µm or more and less than 600 µm) shown in Table 4 is reduced, and thus, it is presumed that the kinetic friction coefficient of the entire particles is reduced. Thus, it is considered that in Examples other than Example 1 or 5, the flowability of the particles is uniformly improved, and the particle size segregation during transportation is suppressed.

On the other hand, Comparative Examples 1 and 3 are examples in which the specific surface area of the water-absorbent resin used is large, and in this case, when the mixing power index was smaller than a specific value, the kinetic friction coefficient increased, and the kinetic friction coefficient reduction percentage decreased (Table 4). Then, according to Table 6, in Comparative Example 1, the absolute values of the particle size change percentages of "particles having a particle diameter of 600 µm or more" and "particles having a particle diameter of less than 300 µm" before and after feeding were large, and particle size segregation occurred during the feeding.

Comparative Example 2 is an example in which the specific surface area of the water-absorbent resin used was small, and in this case, even when the mixing power index was smaller than a specific value, the kinetic friction coefficient was reduced, and the kinetic friction coefficient reduction percentage was improved (Table 4). Then, according to Table 6, the results showed that the absolute values of the particle size change percentages of "particles having a particle diameter of 600 µm or more", "particles having a particle diameter of 300 µm or more and less than 600 µm", and "particles having a particle diameter of less than 300 µm" before and after feeding were small. From this, it is found that when a water-absorbent resin having a small specific surface area is used, even when a flowability improver is added, the particle size segregation does not become a problem in the first place. From such results, it is presumed that when the specific surface area of the water-absorbent resin is small, the flowability improver is uniformly mixed even by a known mixing method, and particle size segregation hardly occurs in transportation of the resulting water-absorbent resin. However, in Comparative Example 2, the specific surface area of the resulting water-absorbing agent composition was small, and thus, the value of Vortex (water absorption speed) of the water-absorbing agent composition was large, and a water-absorbing agent composition having a good water absorption speed could not be obtained.

Comparative Examples 4 to 6 are examples in which the mass-average molecular weight of the flowability improver used is out of the specific range in the present invention, and in these Comparative Examples, the kinetic friction coefficient was not sufficiently reduced, and the kinetic friction coefficient reduction percentage was small.

Comparative Examples 7 to 9 are examples in which the amount of the flowability improver that was added and used was large (the concentration of the flowability improver in the water-absorbing agent composition was large), and in these Comparative Examples, the kinetic friction coefficient and the kinetic friction coefficient reduction percentage fell within good ranges, but the surface tension and/or the flow rate of the resulting water-absorbing agent composition decreased, and thus, good water absorption properties could not be maintained.

Production Example 1, Production Example 3, and Production Example 4 are examples in which 400 ppm of a polyalkylene glycol (polyethylene glycol 600) was added in the step of preparing an aqueous monomer solution. Production Example 3 is an example in which 10 ppm of polyoxyethylene (20) sorbitan monostearate (flowability improver) was added in the surface crosslinking step, but the mixing power index was small. In this case, the kinetic friction coefficient was large, and it cannot be said that the composition has sufficient flowability. On the other hand, Production Example 4 (Example 13) is an example in which polyoxyethylene (20) sorbitan monostearate was added in the surface crosslinking step, and the mixing power index is 70000 or more. In this case, a water-absorbing agent composition having a small kinetic friction coefficient and sufficient flowability was obtained.

From the above, it can be seen that when an aqueous solution of a flowability improver is added to a water-absorbent resin, it is important to satisfy the following (a) to (d) in addition to adding a flowability improver having a specific mass-average molecular weight in an amount of more than 0 ppm and less than 200 ppm relative to the mass of the water-absorbent resin to obtain a water-absorbing agent composition having a high water absorption speed, high flowability, and little particle size segregation during transportation.
(a) The water-absorbent resin has a specific surface area of 25 m²/kg or more;
(b) When a flowability improver is mixed with the water-absorbent resin, the flowability improver is in a form of an aqueous solution of 0.01 mass% or more and 20 mass% or less;
(c) When the aqueous solution is added to and mixed with the water-absorbent resin, the aqueous solution has an average droplet diameter of 10 µm or more and 1 mm or less;
(d) When the aqueous solution is added to and mixed with the water-absorbent resin, the mixing power index defined by the following (Equation 1) is 70000 or more
$\begin{matrix}Mixing power index \left(unit: none\right) \\ = peripheral speed \left(m/s\right) \times mixing time \left(s\right) / \left\{average droplet diameter\left(mm\right)/1000\right\}\end{matrix}$

As a result, it was found that according to the above-described production method, even when a water-absorbent resin having an increased specific surface area is used, the kinetic friction coefficient reduction percentage of particles having a particle diameter of 300 µm or more and less than 600 µm is 10% or more while maintaining good water absorption properties, and further, it is possible to suppress the absolute value of each of the particle size change percentages of "particles having a particle diameter of 600 µm or more", "particles having a particle diameter of 300 µm or more and less than 600 µm", and "particles having a particle diameter of less than 300 µm" before and after feeding to 2% or less. It is presumed that such an effect is due to uniform addition of the aqueous solution of the flowability improver.

It can be seen that the water-absorbing agent compositions obtained in Examples 1 to 13 are novel water-absorbing agent compositions in which the kinetic friction coefficient of particles having a particle diameter other than 300 µm or more and less than 600 µm is uniformly reduced in addition to the kinetic friction coefficient of particles having a large specific surface area and a particle diameter of 300 µm or more and less than 600 µm.

### Reference Example 1

Absorbent bodies were produced using the water-absorbing agent composition (1) and the comparative water-absorbing agent composition (1), respectively. Using each of these absorbent bodies, continuous production of disposable diapers was performed using an actual diaper production apparatus. Relative to the performance of the obtained disposable diaper, a test for comparing the quality stability was performed. The test for comparing the quality stability was performed with reference to the evaluation method for the "reversal amount" disclosed in paragraph [0140] of WO 2020/032282. Three points were randomly selected for each of the disposable diapers to be tested. The disposable diapers were of two types: a type containing pulp (basis weight of water-absorbing agent composition in absorbent body used: 250 g/m², basis weight of pulp in absorbent body used: 200 g/m²) and a type containing no pulp (SAP sheet type) (basis weight of the water-absorbing agent composition in the absorbent body used: 250 g/m²).

As shown in the evaluation results (third return amount) in Tables 7 and 8, in any type of disposable diaper, when the comparative water-absorbing agent composition (1) was used, the fluctuation range of the disposable diaper mass was large, and the fluctuation range of the reversal amount (return amount) was also large as compared with the water-absorbing agent composition (1). These results showed that the water-absorbing agent composition obtained by the production method according to the present invention was stably transported.

In addition, separately from the above evaluation, three points were additionally and randomly selected to each of a disposable diaper of a type containing pulp and a disposable diaper of a type containing no pulp (SAP sheet type), a water-absorbing agent composition was taken out from the disposable diaper, and a particle size distribution was measured. As a result, in any diaper using the comparative water-absorbing agent composition (1), a change of from 5 to 10% was confirmed in the mass proportion of particles having a particle diameter of 600 µm or more and particles having a particle diameter of less than 300 µm relative to the particle size distribution before addition to the diaper. That is, it was confirmed that when the comparative water-absorbing agent composition (1) was used, the particle size segregation occurred when the water-absorbing agent composition was fed in a diaper production apparatus and added to the diaper. Thus, also from these results, it was shown that according to the production method according to the present invention, the particle size segregation of the water-absorbing agent composition after transportation can be suppressed.

### Table 7

**Table 7**

| Pulp diaper | | | | |
|---|---|---|---|---|
| | Comparative water-absorbing agent composition (1) | | Water-absorbing agent composition (1) | |
| | Diaper mass (g) | Return amount (g) | Diaper mass (g) | Return amount (g) |
| n=1 | 34.5 | 1.0 | 34.0 | 0.9 |
| n=2 | 33.5 | 1.3 | 33.9 | 1.0 |
| n=3 | 34.0 | 0.5 | 34.1 | 0.7 |
| Fluctuation range | 1.0 | 0.8 | 0.2 | 0.3 |

### Table 8

**Table 8**

| SAP sheet diaper | | | | |
|---|---|---|---|---|
| | Comparative water-absorbing agent composition (1) | | Water-absorbing agent composition (1) | |
| | Diaper mass (g) | Return amount (g) | Diaper mass (g) | Return amount (g) |
| n=1 | 32.6 | 5.5 | 32.3 | 4.8 |
| n=2 | 33.0 | 4.0 | 32.0 | 5.4 |
| n=3 | 31.5 | 6.0 | 32.2 | 5.1 |
| Fluctuation range | 1.5 | 2.0 | 0.3 | 0.6 |

### Summary

As described above, comparison between Examples and Comparative Examples shows that when a flowability improver is added to a water-absorbent resin having a large specific surface area, it is important to add a flowability improver having a specific mass-average molecular weight in the form of an aqueous solution to satisfy the concentration of the aqueous solution and the average droplet diameter at the time of mixing and to set the mixing power index defined by the above (Equation 1) to a specific value or more to solve the problem of the present application.

According to the present invention, there is provided a novel water-absorbing agent composition having both a large specific surface area of 25 m²/kg or more and a kinetic friction coefficient of 0.80 or less in particles having a particle diameter of 300 µm or more and less than 600 µm, and having good water absorption properties. Patent Documents 1 to 12 do not disclose the water-absorbing agent composition or a method for producing the same.

### Industrial Applicability

The water-absorbing agent composition according to the present invention can be suitably used, for example, in disposable diapers. In addition, the water-absorbing agent composition according to the present invention can be suitably used for various applications such as absorbent articles other than disposable diapers (sanitary napkins, incontinence pads, etc.), soil humectants for agriculture and horticulture, and water-blocking agents for industry.

The present application is based on JP 2023-193269 filed on November 13, 2023, the disclosure content of which is incorporated herein by reference in its entirety.

### Reference Signs List

1 dish (container portion)
2 parallel plate
3 water-absorbing agent composition
4 electromagnetic feeder drive unit
5 trough

## Claims

1. A method for producing a water-absorbing agent composition including a water-absorbent resin as a main component, the method comprising a step of preparing an aqueous monomer solution, a polymerization step, a gel-grinding step, a drying step, a pulverizing step, a classification step, and a surface crosslinking step,
wherein during the surface crosslinking step or during a step after the surface crosslinking step, the method includes a step of mixing, with the water-absorbent resin, a water-soluble flowability improver having a mass-average molecular weight of 200 or more and 50000 or less in an amount of more than 0 ppm and less than 200 ppm relative to a mass of the water-absorbent resin,
the method satisfying all of (a) to (d) described below:
(a) the water-absorbent resin has a specific surface area of 25 m²/kg or more;
(b) when the water-soluble flowability improver is mixed with the water-absorbent resin, the water-soluble flowability improver is in a form of an aqueous solution of 0.01 mass% or more and 20 mass% or less;
(c) when the aqueous solution is added to and mixed with the water-absorbent resin, the aqueous solution has an average droplet diameter of 10 µm or more and 1 mm or less;
(d) when the aqueous solution is added to and mixed with the water-absorbent resin, a mixing power index defined by (Equation 1) described below is 70000 or more.
[Math.1] $\begin{matrix}Mixing power index \left(unit:none\right) \\ = peripheral speed \left(m/s\right) \times mixing time \left(s\right) / \left\{average droplet diameter \left(mm\right)/1000\right\}\end{matrix}$

2. The production method according to claim 1, wherein in the water-absorbent resin after addition of the water-soluble flowability improver, particles having a particle diameter of 300 µm or more and less than 600 µm have a kinetic friction coefficient of 0.80 or less.

3. The production method according to claim 1, wherein the water-soluble flowability improver is one or more selected from a nonionic substance, a
zwitterionic substance, an anionic substance, and a cationic substance.

4. The production method according to claim 3, wherein
the nonionic substance is selected from a polyol, a modified product of a hydroxy group of a polyol, a side-chain and/or terminal polyether-modified polysiloxane, and an alkylene oxide adduct of a higher aliphatic amine;
the
zwitterionic substance is selected from an alkyl betaine and an alkylamine oxide;
the anionic substance is selected from an alkyl sulfate ester salt, a sulfate ester salt of a higher alcohol alkylene oxide adduct, a sulfonic acid salt, a dicarboxylic acid salt, an alkylamine diacetic acid salt, a phosphoric acid ester salt of a higher alcohol alkylene oxide adduct, and a carboxylic acid salt of a higher alcohol alkylene oxide adduct; and
the cationic substance is selected from an ammonium salt.

5. The production method according to claim 1, wherein the water-soluble flowability improver includes at least one selected from a nonionic substance.

6. The production method according to claim 1, wherein the water-soluble flowability improver includes at least one selected from a nonionic substance having a polyalkylene glycol chain in a molecule.

7. The production method according to claim 1, wherein the water-soluble flowability improver includes at least one selected from a polyol and a modified product of a hydroxy group of a polyol.

8. The production method according to claim 1, wherein the aqueous solution has a pH of 4.5 or more.

9. The production method according to claim 1, wherein
the water-absorbent resin and the water-absorbing agent composition each include particles having a particle diameter of 300 µm or more and less than 600 µm in an amount of 50 mass% or more, and
a kinetic friction coefficient reduction percentage calculated from (Equation 2) described below is 10% or more:
[Math.2] $Kinetic friction coefficient reduction percentage \left(%\right) = \left(A-B\right) / A \times 100$
where
A is a kinetic friction coefficient of particles of the water-absorbent resin before addition of the water-soluble flowability improver, the particles having a particle diameter of 300 µm or more and less than 600 µm;
B is a kinetic friction coefficient of particles of the water-absorbing agent composition after addition of the water-soluble flowability improver, the particles having a particle diameter of 300 µm or more and less than 600 µm.

10. The production method according to claim 1, comprising further adding a polyalkylene glycol in at least one step selected from the step of preparing an aqueous monomer solution, the polymerization step, and the gel-grinding step, and/or between the steps.

11. The production method according to claim 10, wherein the polyalkylene glycol is a polyethylene glycol having a mass-average molecular weight of 3000 or less.

12. The production method according to claim 10, wherein an amount of the polyalkylene glycol added is from 0.01 mass% to 0.25 mass% relative to a total mass of monomers included in the aqueous monomer solution.

13. A water-absorbing agent composition including a water-absorbent resin as a main component,
the water-absorbing agent composition comprising a water-soluble flowability improver and satisfying all of (1) to (5) described below:
(1) the water-absorbing agent composition has a specific surface area of 25 m²/kg or more;
(2) the water-absorbing agent composition has a surface tension of 56 mN/m or more;
(3) the water-absorbing agent composition has a flow rate of 10.0 g/s or more;
(4) a mass proportion of particles having a particle diameter of 300 µm or more and less than 600 µm in the water-absorbing agent composition is 50 mass% or more;
(5) the particles having a particle diameter of 300 µm or more and less than 600 µm in the water-absorbing agent composition have a kinetic friction coefficient of 0.80 or less.

14. The water-absorbing agent composition according to claim 13, the water-absorbing agent composition having a Vortex (water absorption speed) of 50 seconds or less.

15. The water-absorbing agent composition according to claim 13, the water-absorbing agent composition having an SFC (saline flow conductivity) of 1 × 10⁻⁷ cm³·sec/g or more.

16. The water-absorbing agent composition according to claim 13, wherein
the water-absorbing agent composition has a D50 (mass-average particle diameter) of 250 µm or more and less than 550 µm, and
a mass proportion of particles having a particle diameter of less than 150 µm is 3 mass% or less.

17. The water-absorbing agent composition according to claim 13, the water-absorbing agent composition having an AAP (absorption capacity under pressure) of 20 g/g or more.

18. An absorbent body comprising the water-absorbing agent composition described in any one of claims 13 to 17.

19. The absorbent body according to claim 18, wherein a basis weight of pulp is 300 g/m² or less.

20. The absorbent body according to claim 18, wherein the water-absorbing agent composition has a basis weight of 450 g/m² or less.

21. An absorbent article comprising the absorbent body described in any one of claims 18 to 20.

22. The absorbent article according to claim 21, the absorbent article comprising no pulp.
